# EUROPEAN PATENT APPLICATION

(11) **EP 2 743 350 A1**
(43) Date of publication of application: **18.06.2014**
(21) Application number: 12196916.6
(22) Date of filing: 13.12.2012
(51) Int. Cl.: C12N 15/82, C12N 15/81, C12P 17/12, C12P 7/42

(54) **A method for producing ectoine or a derivative thereof and a yeast cell for use as a host cell in such a method**

(71) Applicant: Artes Biotechnology GmbH, 40764 Langenfeld (DE)
(72) Inventor: Eilert, Eva, 40223 Düsseldorf (DE); Suckow, Manfred, Dr., 40593 Düsseldorf (DE); Piontek, Michael, Dr., 42549 Velbert (DE)
(74) Representative: Zenz

(57) **Abstract**

A method for producing ectoine or a derivative thereof comprises the steps of (a) preparing host cells from a type of yeast, the host cells containing at least one recombinant DNA sequence encoding each of one or more heterologous enzymes for biosynthesis of ectoine, (b) causing the host cells, with the aid of the recombinant DNA sequences encoding the enzymes, to express the heterologous enzymes, and (c) causing the host cells, with the aid of the expressed heterologous enzymes, to synthesize ectoine and/or the derivative thereof from a precursor. A yeast cell of the orders Saccharomycetales or Schizosaccharomycetales for use as a host cell in such a method contains at least one recombinant DNA sequence encoding each of one or more heterologous enzymes for biosynthesis of ectoine.

## Description

The present invention relates to a method for producing ectoine or a derivative thereof, using host cells containing at least one recombinant DNA sequence encoding each of one or more heterologous enzymes for biosynthesis of ectoine.

Ectoine and 5-hydroxyectoine belong to the family of compatible solutes which are known to stabilize the cell's essential functions by maintaining the stability of proteins under stress conditions like high salinity, heat or aridity stress. Hence, compatible solutes are usually synthesized under elevated saline conditions and then accumulate in the cell (Lippert, K., Galinski, E.: Enzyme stabilization by ectoine-type compatible solutes: protection against heating, freezing and drying; Appl. Microbiol. Biotechnol. 37, 1992, p. 61-65).

Ectoine and 5-hydroxyectoine as well as the precursor Nyacetyl-diaminobutyrate are also known to have protective properties towards nucleic acids, membranes and whole cells. Ectoine was first discovered in *Ectothiorhodospira halochloris,* later more halophilic or halotolerant organisms with the ability to produce ectoine, or in addition 5-hydroxyectoine, were identified. Consequently it appears beneficial that other organisms without the ability to synthesize ectoine or 5-hydroxyectoine themselves are often able to accumulate those compounds from their environment and profit from their protective properties.

Due to its chiral carbons ectoine and 5-hydroxyectoine can only be efficiently produced via biological approaches.

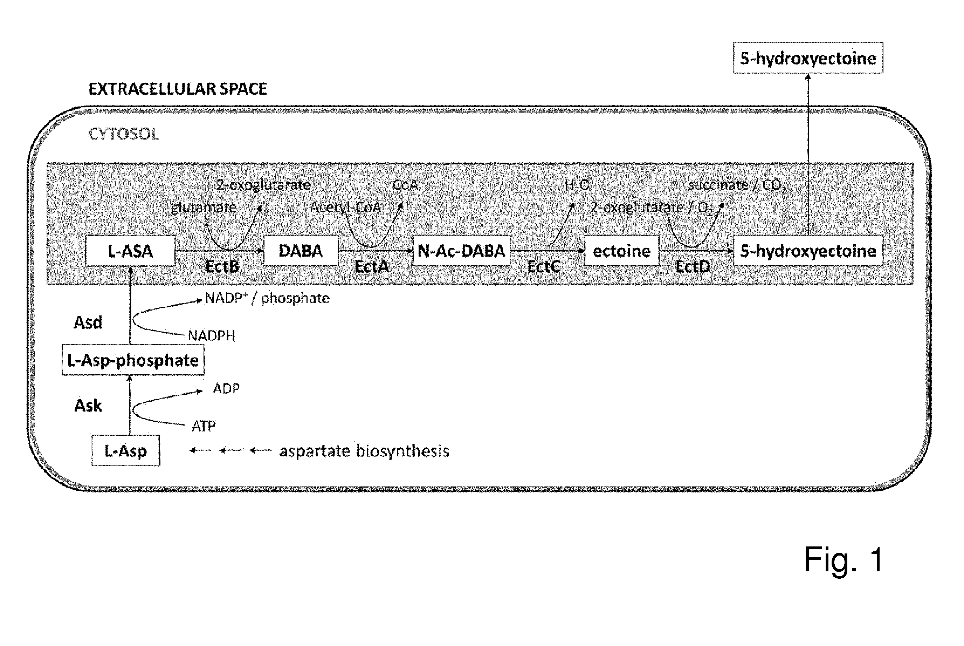

Among moderate halophilic bacteria, *Halomonas elongata* is considered one important model organism and has been studied extensively (Garcia-Estepa, R., Cánovas, D., Iglesias-Guerra, F., Ventosa, A., Csonka, L., Nieto, J., Vargas, C.: Osmoprotection of Salmonella enterica serovar Typhimurium by Nyacetyldiaminobutyrate, the precursor of the compatible solute ectoine; Syst. Appl. Microbiol. 29 (8), 2006, p. 626-633). The synthesis of ectoine takes place in three steps starting with L-aspartate-semialdehyde. L-aspartate-semialdehyde is converted by the enzyme EctB to L-2,4-diaminobutyrate and next the Ny-acetyl-2,4-diaminobutyrate is formed by the action of the enzyme EctA. In the last step ectoine is generated by the enzyme EctC by condensation. An additional hydroxylation step catalyzed by the enzyme EctD converts ectoine into 5-hydroxyectoine (Bursy, J., Pierik, A., Pica, N., Bremer, E.: Osmotically induced synthesis of the compatible solute hydroxyectoine is mediated by an evolutionarily conserved ectoine hydroxylase; J. Biol. Chem. 282 (43), 2007, p. 31147-31155). Figure 1 is a schematic overview of the 5-hydroxyectoine synthesis pathway of *H. elongata.* The abbreviations used in Figure 1 are: L-Asp: L-aspartate, L-ASA: L-aspartate-β-semialdehyde, DABA: L-aspartate-diaminobutyrate, N-Ac: N-Acetyl, Ask: aspartate kinase, Asd: aspartate-semialdehyde dehydrogenase, EctB: diaminobutyrate-2-oxoglutarate transaminase, EctA: L-2,4-diaminobutyrate acetyltransferase, EctC: ectoine synthase, EctD: ectoine hydroxylase. As a precursor for 5-hydroxyectoine synthesis L-Asp is provided by respective anabolic processes.

The synthesis of ectoine and 5-hydroxyectoine in *H. elongata* only occurs under a variety of stress conditions, e.g. salt, wherein not the entire ectoine pool is converted to 5-hydroxyectoine. In addition it is known that in *H. elongata* ectoine can also be hydrolyzed to Ny- and Nα-acetyl-L-2,4-diaminobutyrate by the ectoine hydrolase resulting in a reduced yield (Schwibbert, K., Marin-Sanguino, A., Bagyan, I., Heidrich, G., Lentzen, G., Seitz, H., Rampp, M., Schuster, S.C., Klenk, H.-P., Pfeiffer, F., Oesterhelt, D., Kunte, H.: A blueprint of ectoine metabolism from the genome of the industrial producer Halomonas elongata DSM 2581; Environ. Microbiol. 13 (8), 2010, p. 1973-1994).

Heterologous organisms for synthesizing ectoine or 5-hydroxyectoine are of interest, since these organisms not only accumulate the ectoine or 5-hydroxyectoine within the cells but can also secrete them. The relevant enzymes involved in ectoine biosynthesis have already been expressed in heterologous prokaryotic microbial systems. *Escherichia coli* was utilized for the expression of the EctABC clusters derived from different species, such as *Bacillus halodurans* (Anbu Rajan, L., Joseph, T., Thampuran, N., James, R., Ashok Kumar, K., Viswanathan, C., Bansal, K.: Cloning and heterologous expression of ectoine biosynthesis genes from Bacillus halodurans in Escherichia coli; Biotechnol. Lett. 30 (8), 2008, p. 1403-1407) or *Chromohalobacter salexigens* (Schubert, T., Maskow, T., Benndorf, D., Harms, H., Breuer, U.: Continuous synthesis and excretion of the compatible solute ectoine by a transgenic, nonhalophilic bacterium; Appl. Environ. Microbiol. 73 (10), 2007, p. 3343-3347), leading to yields of up to 6 g/l of ectoine. Another effective ectoine process reported using *E. coli* is 'bacterial milking' introduced by Sauer and Galinski (Sauer, T., Galinski, E.: Bacterial milking: A novel bioprocess for production of compatible solutes; Biotechnol. Bioeng. 57 (3), 1998, p. 306-313). Lentzen and Schwarz (Lentzen, G., Schwarz, T.: Kompatible Solute: Mikrobielle Herstellung und Anwendung, in: Antranikian, G. (Ed.), Angewandte Mikrobiologie, Springer-Verlag, Berlin, Heidelberg, 2006) developed the so far most effective 'permanent-milking' process using the genuine producer *H. elongata* with yields up to 20 g/l ectoine, a process which finds application in industrial production.

The 5-hydroxyectoine biosynthesis was optimized recently using the homologous producer *Pseudomonas stutzeri* and recombinant *E. coli* expressing the *P. stutzeri* EctABCD-ask cluster (Seip, B., Galinski, E., Kurz, M.: Natural and engineered hydroxyectoine production based on the Pseudomonas stutzeri ectABCD-ask gene cluster; Appl. Environ. Microbiol. 77 (4), 2011, p. 1368-1374). These approaches resulted in almost complete transformation of ectoine to 5-hydroxyectoine.

5-hydroxyectoine production using *H. elongata* is also possible by modification of the 'bacterial milking' process (Lentzen, G., Schwarz, T.: Extremolytes: Natural compounds from extremophiles for versatile applications; Appl. Microbiol. Biotechnol. 72 (4), 2006, p. 623-634). However, Seip et al. (see above) stated that even after optimization of the production process by increased salinity and elevated temperature 5-hydroxyectoine synthesis did not exceed 50%. Therefore, due to the inavailability of an efficient heterologous production system both compounds are currently biotechnologically produced solely using the homologous system *H. elongata* (Reshetnikov, A., Khmelenina, V., Mustakhimov, I., Trotsenko, Y.: Genes and enzymes of ectoine biosynthesis in halotolerant methanotrophs; Meth. Enzymol. 495, 2011, p. 15-30).

Therefore, it is a problem underlying the invention to provide a possibility to synthesize ectoine or a derivative thereof, in particular 5-hydroxyectoine, independently of stress parameters, such as high salt concentrations which are hostile conditions for production and subsequent downstream processing.

This problem is solved by a method for producing ectoine or a derivative thereof according to claim 1. Moreover, the problem is solved by a yeast cell according to claim 20.

The method for producing ectoine or a derivative thereof according to the invention comprises the steps of:
(a) preparing host cells from a type of yeast, the host cells containing at least one recombinant DNA sequence encoding each of one or more heterologous enzymes for biosynthesis of ectoine;
(b) causing the host cells, with the aid of the recombinant DNA sequences encoding the enzymes, to express the heterologous enzymes; and
(c) causing the host cells, with the aid of the expressed heterologous enzymes, to synthesize ectoine and/or the derivative thereof from a precursor.

In the preferred embodiment, the host cells are prepared from at least one yeast type of the orders Saccharomycetales or Schizosaccharomycetales, in particular from at least one yeast type of the genera Pichia (Hansenula and Hyphopichia are obsolete synonyms), Dekkera (also known as Brettanomyces), Saccharomyces, Kluyveromyces, Candida, Blastobotrys (also known as Arxula), Debaromyces (also known as Schwanniomyces), and Yarrowia, preferably the genus Pichia (Hansenula). More preferably, host cells are prepared from at least one methylotrophic yeast type of the genus Pichia (Hansenula); most preferably from *Pichia angusta (Hansenula polymorpha).*

The invention is based on the finding that a eukaryotic host cell from a type of yeast, in particular from *Pichia angusta (Hansenula polymorpha),* can express the heterologous enzymes for biosynthesis of ectoine or a derivative thereof, in particular 5-hydroxyectoine, and can activate these enzymes so that ectoine or the derivative thereof, in particular 5-hydroxyectoine, can be synthesized without necessity of stress conditions like high salinity. Moreover, the host cells can secret the synthesized ectoine or the derivative thereof, in particular 5-hydroxyectoine, so that it can be easily separated from the culture supernatant.

In a preferred embodiment, the step (a) of preparing host cells comprises preparing host cells from a type of yeast, the host cells containing at least one recombinant DNA sequence encoding a DABA transaminase enzyme, at least one recombinant DNA sequence encoding a DABA acetyltransferase enzyme, and at least one recombinant DNA sequence encoding an ectoine synthase enzyme; and the step (b) of causing the host cells to express the heterologous enzymes comprises the step of causing the host cells to express the heterologous enzymes DABA transaminase, DABA acetyltransferase and ectoine synthase. More preferably, the step (a) of preparing host cells comprises preparing host cells from a type of yeast, the host cells containing DNA sequences encoding the enzymes DABA-2-oxoglutarate transaminase (EctB), DABA acetyltransferase (EctA) and ectoine synthase (EctC) of the ectoine biosynthesis pathway of *Halomonas elon*gata; and the step (b) of causing the host cells to express the heterologous enzymes comprises the step of causing the host cells to express the enzymes DABA-2-oxoglutarate transaminase (EctB), DABA acetyltransferase (EctA) and ectoine synthase (EctC) of the ectoine biosynthesis pathway of Halomonas elongata. In this preferred embodiment the enzymes of the ectoine biosynthesis pathway of the prokaryotic species Halomonas elongate are expressed and active in the eukaryotic yeast cells.

In order to synthesize 5-hydroxyectoine, a preferred derivative of ectoine, the method preferably comprises the step (a) of preparing host cells containing at least one further recombinant DNA sequence encoding a heterologous enzyme for the hydroxylation of ectoine to 5-hydroxyectoine, wherein the step (b) of causing the host cells to express the heterologous enzymes comprises the step of causing the host cells to additionally express the heterologous enzyme for the hydroxylation of ectoine to 5-hydroxyectoine. In a preferred embodiment of this method, the host cells additionally contain at least one recombinant DNA sequence encoding an ectoine hydroxylase enzyme; wherein the step (b) of causing the host cells to express the heterologous enzymes comprises the step of causing the host cells to additionally express the ectoine hydroxylase enzyme. Most preferably, the method comprises preparing host cells from a type of yeast, the host cells containing additionally at least one recombinant DNA sequence encoding the ectoine hydroxylase (EctD) of the ectoine biosynthesis pathway of Halomonas elongata; wherein the step (b) of causing the host cells to express the heterologous enzymes comprises the step of causing the host cells to additionally express the ectoine hydroxylase (EctD) of the ectoine biosynthesis pathway of Halomonas elongata.

In one embodiment, each of the at least one recombinant DNA sequence encoding each of one or more heterologous enzymes for biosynthesis of ectoine is contained in an expression cassette comprising a strong constitutive or inducible homologous promoter. This use of a strong promoter of a native enzyme of the yeast provides for a high-level expression. In a preferred embodiment, the yeast is a methylotrophic yeast and the promoter is an inducible formate dehydrogenase (FMD) promoter. In this embodiment, the step (b) of causing the host cells to express the heterologous enzymes preferably comprises adding methanol to the culture. Preferably, the expression cassette comprises a methanol oxidase (MOX) terminator.

In a further embodiment, each of the at least one recombinant DNA sequence encoding each of one or more heterologous enzymes for biosynthesis of ectoine is contained in an expression cassette comprising a homologous terminator from a native gene different from the native gene of the homologous promoter. This has the advantage that the combination of the promoter and the terminator does not exist in a native gene.

In one embodiment, the step (b) of causing the host cells to express the heterologous enzymes comprises adding a derepressing or inducing agent.

In one embodiment, the step (c) of causing the host cells to synthesize ectoine and/or the derivative thereof from a precursor comprises adjusting a pH value between 3 and 7 in the culture, preferably a pH value of 5.

Preferably, the method for producing ectoine or a derivative thereof comprises the additional steps of (d) causing the host cells to export or secrete the synthesized ectoine and/or the derivative thereof to a culture supernatant, and (e) separating the exported or secreted ectoine and/or the derivative thereof from the culture supernatant.

According to the invention, a yeast cell of the orders Saccharomycetales or Schizosaccharomycetales for use as a host cell in a method for producing ectoine or a derivative thereof is provided, the yeast cell containing at least one recombinant DNA sequence encoding each of one or more heterologous enzymes for biosynthesis of ectoine. Preferably, the yeast cell is a yeast cell of the genera Pichia (Hansenula), Dekkera (Brettanomyces), Saccharomyces, Kluyveromyces, Candida, Blastobotrys (Arxula), Debaromyces (Schwanniomyces), and Yarrowia, more preferably a a methylotrophic yeast cell of the genus Pichia (Hansenula). In a preferred embodiment, the yeast cell comprises at least one recombinant DNA sequence encoding a DABA transaminase enzyme, at least one recombinant DNA sequence encoding a DABA acetyltransferase enzyme, and at least one recombinant DNA sequence encoding an ectoine synthase enzyme. In a further preferred embodiment, the yeast cell comprises at least one further recombinant DNA sequence encoding a heterologous enzyme for the hydroxylation of ectoine to 5-hydroxyectoine. Preferably, each of the at least one recombinant DNA sequence encoding each of one or more heterologous enzymes for biosynthesis of ectoine is contained in an expression cassette comprising a strong constitutive or inducible homologous promoter. Most preferably, the yeast cell is methylotrophic and the promoter is a formate dehydrogenase (FMD) or a methanol oxidase (MOX) promoter.

Preferred embodiments are characterized in the dependent claims.

In the following detailed description, preferred embodiments are described in connection with the drawings wherein:
Fig. 1 is a schematic overview of the 5-hydroxyectoine synthesis pathway of H. elongate;
Fig. 2 is a schematic overview of the targeted single copy integration of Ect genes into the genome of H. polymorpha;
Fig. 3 is a Western blot analysis of total cell extract of strain ALU3/EctBACD #1 coexpressing EctB, EctA, EctC and EctD;
Fig. 4 is a diagram showing the determination of ectoine and 5-hydroxyectoine conversion yields of ALU3/EctBACD #1 in dependence of media pH-value during cultivation in 3 ml scale;
Fig. 5 is a diagram showing the determination of ectoine and 5-hydroxyectoine conversion yields during cultivation of strain ALU3/EctBACD #1 in 3 ml scale in dependence of carbon source feeding mode; and
Fig. 6 is a diagram showing the fermentation of strain ALU3/EctBACD #1 at 1 litre scale.

The preferred embodiment is a stable whole-cell biocatalyst on the basis of the methylotrophic yeast *Hansenula polymorpha* (also known as *Pichia angusta)* which catalyzes the transformation of monomeric substrates to 5-hydroxyectoine. The genes of the 5-hydroxyectoine biosynthesis pathway of Halomonas elongata were inserted into the genome of Hansenula polymorpha. Subsequently the synthesis and secretion of ectoine and 5-hydroxyectoine were analyzed and optimized. Instead of the genes of H. elongata homologous genes of other organisms may be used for recombinant establishment of the pathway in H. polymorpha or another expression organism.

### 1. Material and Methods

### a) Strains and culture conditions

*Escherichia coli* NEB10ß (araD139 Δ(ara-leu)7697 fhuA lacX74 galK (φ80 Δ(lacZ)M15) mcrA galU recA1 endA1 nupG rpsL (StrR) Δ(mrr-hsdRMS-mcrBC); New England Biolabs, Frankfurt a. M., Germany) was used for construction and propagation of plasmid DNA. Cultivation took place in Luria Bertani media, when needed supplemented with 50 µg ampicillin per ml.

Hansenula polymorpha strain ALU3 (*ade1 leu2 odc1)* was used for protein expression as a host. Cultivation took place in either yeast extract-peptone-dextrose medium complemented with 100 µg adenine per ml (YPD-Ade) or yeast nitrogen base medium complemented with 80 µg uracil per ml (YNB-Ura). All cultivations were performed at 37°C if not indicated otherwise.

### b) Plasmid constructions

To generate the expression plasmids, synthetic open reading frames (ORFs) for the enzymes EctB, EctA, EctC and EctD from *Halomonas elongata* ATCC 33173 based on Universal Protein Resource Knowledgebase were inserted into four different vectors containing the upstream- and downstream fragment for the target locus as well as a unique selection marker, respectively (see below (c): Single copy integration system). Each ORF has been fused with the codons of a C- or N-terminal tag allowing individual detection.

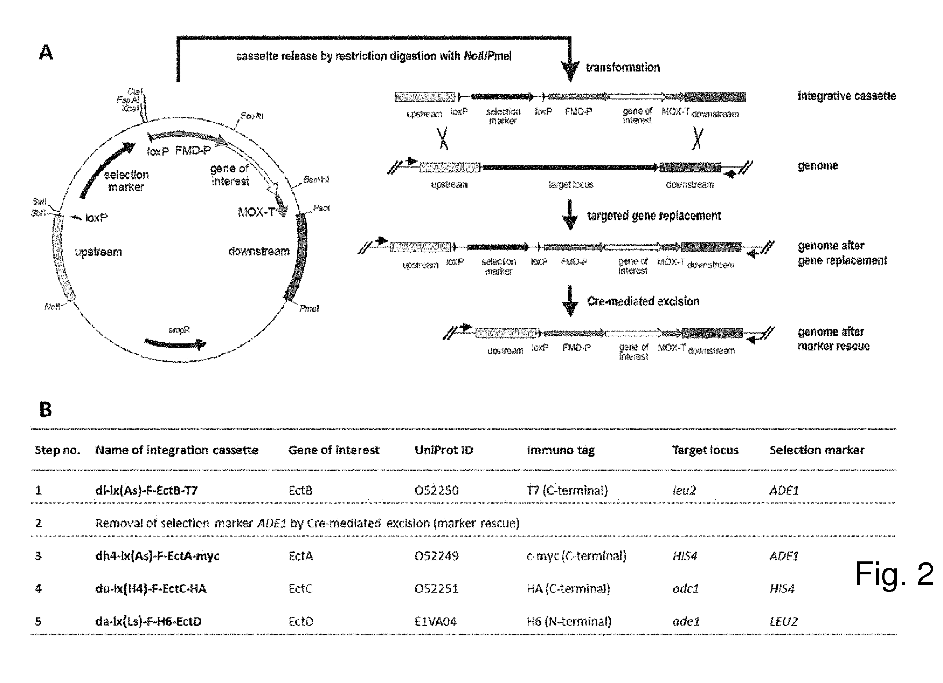

Fig. 2 is a schematic overview of the targeted single copy integration of Ect genes into the genome of *H. polymorpha.* In (A) a schematic representation of the plasmids is shown harboring elements for integration of single copy expression cassettes and their integration by targeted gene replacement as well as the genomic structure upon marker rescue. Primer hybridization sites for analytical PCR are symbolized by small black arrows. (B) is a table showing the various combinations of genes, tags, loci, and selection markers in the integration cassettes harbored by those plasmids, and the succession of integration events leading to strain ALU3/EctBACD #1 with indication of source of the Ect gene, immuno tag used for detection, target locus for single copy integration, and selection marker. Integration cassettes were integrated into the genome of *H. polymorpha* ALU3 by the indicated integration order. For removal of the selection marker within step no. 2 the Cre-recombinase was transiently expressed.

The protein sequence of EctA-myc, i.e. the combination of the proteins of the enzyme L-2,4-diaminobutyrate acetyltransferase (EctA) and the myc-tag, is shown as SEQ ID 1 in the sequence listing in the Appendix of the description. The protein sequence of EctB-T7, i.e. the combination of the proteins of the enzyme diaminobutyrate-2-oxoglutarate transaminase (EctB) and the T7-tag, is shown as SEQ ID 2. The protein sequence of EctC-HA, i.e. the combination of the proteins of the enzyme ectoine synthase (EctC) and the HA-tag, is shown as SEQ ID 3. The protein sequence of H6-EctD, i.e. the combination of the proteins of the polyhistidine-tag (His-tag or H6-tag) and the enzyme ectoine hydroxylase (EctD), is shown as SEQ ID 4.

The DNA sequence of the plasmid pDh4-lx(As)-F-EctA-myc is shown as SEQ ID 5, the DNA sequence of the plasmid pDl-lx(As)-F-EctB-T7 is shown as SEQ ID 6, the DNA sequence of the plasmid pDu-lx(H4)-F-EctC-HA is shown as SEQ ID 7 and the DNA sequence of the plasmid pDa-lx(Ls)-F-H6-EctD is shown as SEQ ID 8.

The DNA sequences of the corresponding integration cassettes Dh4-lx(As)-F-EctA-myc, Dl-lx(As)-F-EctB-T7, Du-lx(H4)-F-EctC-HA and Da-lx(Ls)-F-H6-EctD are shown as sequences SEQ ID 9 to SEQ ID 12, respectively.

*Hansenula polymorpha* was transformed by electroporation as described by Faber et al. (Faber, K.N., Haima, P., Harder, W., Veenhuis, M., AB, G.: Highly-efficient electrotransformation of the yeast Hansenula polymorpha; Curr. Genet. 25 (4), 1994, p. 305-310).

In other embodiments it would be possible to construct multi-copy expression plasmids encoding the various Ect genes and integrate them into the genome of H. polymorpha.

If another yeast expression organism is used as the host cells, host specific vector systems must be used for cloning and introduction of the various Ect genes. If genomic integration should not be possible, expression of the Ect genes from episomal plasmids may be performed.

### c) Single copy integration system

In *Hansenula polymorpha,* introduced circular plasmids are initially episomal before integrated as high-copy tandem repeats into the genome. Plasmid integration and amplification are stochastic processes, and the number of copies finally integrated is not predictable. For the defined expression of several genes of interest a modular system for integration of single expression cassettes into the genome by targeted gene replacement was used. The significant qualities of that system are as follows (see also Fig. 2, (A)):
(1) FMD-promoter and MOX-terminator as transcriptional elements with a multiple cloning site in between to confer high-level expression of any gene of interest;
(2) interchangeable yeast selection markers flanked by loxP sites suitable for Cre-mediated marker rescue;
(3) both selection marker and expression cassette are flanked by up- and downstream target sequences of approximately 1 kb size to confer double-homologous recombination at the target locus with high frequency;
(4) all modules are flanked with unique restriction sites for convenient cloning; and
(5) the integrative cassette can be isolated via restriction with NotI/PmeI.

After transformation with the cassette and selection of transformants, colonies displaying the expected prototrophy were screened by a locus-specific analytical PCR for the expected recombination event. The primer hybridization sites were chosen in a way that a PCR product length dimorphism shows the presence of the target locus with or without the integrated cassette, respectively (see Fig. 2, (A); primer hybridization sites are indicated as arrows).

The Cre-loxP system was used for site-specific removal of selection marker genes. For transient expression of the Cre recombinase, the plasmid pFPMT-2m-Cre was constructed. This derivate of pFPMT121 (described by Degelmann, A., Muller, F., Sieber, H., Jenzelewski, V., Suckow, M., Strasser, A., Gellissen, G.: Strain and process development for the production of human cytokines in Hansenula polymorpha; FEMS Yeast Res. 2 (3), 2002, p. 349-361) contains the Cre gene from pSH47 (see Güldener, U., Heck, S., Fielder, T., Beinhauer, J., Hegemann, J.H.: A new efficient gene disruption cassette for repeated use in budding yeast; Nucleic Acids Res. 24 (13), 1996, p. 2519-2524) under control of the FMD promoter.

Transformants were selected on agar plates containing 2% glycerol for immediate expression of the recombinase; subsequently, isolated colonies were picked and transferred onto plates with nonselective medium to allow plasmid loss. Cells having lost both selection marker and Cre expression plasmid were identified by replica plating onto diagnostic agar plates.

### d) Protein expression and preparation of protein material

For protein expression, the recombinant strains were subjected to derepression. The cells were cultivated in 3 ml scale in YNB containing 1-2% of a derepressing carbon source (glycerol or sorbitol) for 24-48 h at 37°C. Then methanol (MeOH) was added to 1% v/v and cultivation was continued for additional 24-48 h with repetition of the methanol induction every 24 h if applicable. After cultivation (72 h) the cells were harvested by centrifugation. Cells and culture supernatant were separately collected and stored at -20°C. The total cell extract was prepared by glass bead cell disruption. Subsequently, the samples were centrifuged for 15' at 16000 x g to separate the insoluble and soluble fractions.

### e) Protein analysis

For molecular weight determination, the samples were analyzed by SDS-PAGE according to Laemmli (Laemmli, U. K.: Cleavage of structural proteins during the assembly of the head of bacteriophage T4; Nature 227 (5259), 1970, p. 680-685). For the electrophoretic separation 4-12% Bis-Tris acryl amide XT gels (BioRad, Hercules, CA, USA) were used. The protein band patterns were transferred to nitrocellulose membranes. The protein bands were then visualized by immunoblotting. For immunological detection a His-tag-specific antibody (MicroMol, Karlsruhe, Germany), a HA-specific antibody (Sigma-Aldrich, St. Louis, MO, USA), a c-myc-specific antibody (BioLegend, San Diego, CA, USA), or a T7-specific antibody (MBL, Nagoya, Japan) was applied depending on the protein to be detected. Goat-anti-mouse-AP-conjugate or goat-anti-rabbit-AP-conjugate (both BioRad) were used as secondary antibodies. Development was performed by use of NBT/BCIP (Roche, Mannheim, Germany).

### f) HPLC detection of ectoine and 5-hydroxyectoine

For HPLC analysis samples were diluted with the HPLC eluent (Acetonitrile/phosphate buffer 71/29, pH 7.2) to an approximate concentration of 10 - 50 ng/ml and centrifuged (13000 rpm, 5 min) with subsequent filtration of the supernatant with a cutoff at 0.22 µm. HPLC analyses were carried out using a Nucleodur 100-5 NH2-RP CC 125/4 column (Macherey & Nagel, Düren, Germany) at a flow rate of 1 ml/min at 25°C. Ectoine and 5-hydroxyectoine were measured by absorbance at 210 nm using a UV/VIS detector. Standard components (ectoine, 5-hydroxyectoine and Ny-acetyl-diaminobutyrate) were kindly provided by the bitop AG.

### g) Fermentation

The fermentation was carried out in a 1.25 1 Bioflo III fermenter (New Brunswick Scientific, Edison, NJ, USA). 100 ml YPD preculture were grown for 20 h. Fermentation was started by adjusting an initial optical density of OD600 nm = 1 in a start culture volume of 1 1 using SYN6 medium, as previously described in Eilert et al. (2012), supplemented with 2% (w/v) sorbitol and 500 mg/l uracil.

The aeration rate was 1 vvm using a ring sparger. The cultivation temperature was adjusted to 37°C. Agitation was performed by two stirrer blades (speed between 500 and 1000 rpm). The sorbitol/methanol feed was started after 25 h with a feed rate of 2 g/l h⁻¹; adjustments of the sorbitol/methanol feed are described in the results section. During the derepression phase the pO2 was kept at 40 % by a stirrer cascade between 500 and 1000 rpm. pH 5 was adjusted and maintained using a 12.5 % v/v ammonia solution. Sterile diluted antifoam agent (10 % (v/v) structol J 673) was added manually as appropriate. Feed was performed using a 37.5% (w/v) sorbitol 37.5% (v/v) methanol solution or a 15% (w/v) sorbitol 60% (v/v) methanol solution. Fermentation was terminated after 189 h.

During fermentation samples were taken and analyzed offline. The optical density (OD) was determined at 600 nm wavelength, dry cell weight (dcw) was analyzed, and products were analyzed by HPLC.

### 2. Results

### a) H. polymorpha - a suitable candidate for 5-hydroxyectoine synthesis

It was shown that ectoine cannot only serve as an osmolyte but that some organisms can utilize it as a nutrient. Therefore, before introduction of the genes of the 5-hydroxyectoine biosynthesis pathway into *H. polymorpha,* a possible ability of *H. polymorpha* ALU3 to utilize ectoine had to be excluded, because degradation of the intermediate ectoine would counterproductive-ly interfere with the desired 5-hydroxyectoine synthesis. For this purpose *H. polymorpha* ALU3 was cultivated in YNB without carbon- or nitrogen-source but supplemented with 1% (w/v) ectoine. Determination of the OD595nm did not show any growth of the yeast strain indicating that *H. polymorpha* ALU3 did not utilize ectoine, neither as a carbon- nor as a nitrogen-source.

The synthesis of 5-hydroxyectoine is based on the synthesis of amino acids of the aspartate family (Pastor, J., Salvador, M., Argandoña, M., Bernal, V., Reina-Bueno, M., Csonka, L., Iborra, J., Vargas, C., Nieto, J., Cánovas, M.: Ectoines in cell stress protection: uses and biotechnological production; Biotechnol. Adv. 28 (6), 2010, p. 782-801). L-aspartate is initially converted to L-aspartate-phosphate by the aspartate kinase (Ask) and then further converted to L-aspartate-β-semialdehyde by the L-aspartate-β-semialdehyde dehydrogenase (Asd). Database analysis suggested the existence of enzymes with Ask- and Asd-activity in *H. polymorpha* (*H. polymorpha* NCYC 495 leu1.1, Ask - protein ID 17263, Asd - protein ID 76258). In addition, *H. polymorpha* does not seem to harbor the gene for ectoine hydrolase, and therefore degradation of ectoine is unlikely. For that reason, for 5-hydroxyectoine synthesis expression of the enzymes EctA, EctB, EctC and EctD in *H. polymorpha* should be sufficient without further strain modifications.

### b) Generation of recombinant H. polymorpha strains coexpressing genes of the H. elongata 5-hydroxyectoine pathway

Synthetic ORFs of EctA, EctB, EctC and EctD were designed, generated, and inserted into *H. polymorpha* single copy expression vectors with different selection markers and integration loci (see Fig. 2). To provide the option of single detection of each protein, codons for a C- or N-terminal tag were added to the ORFs (EctA-myc, EctB-T7, EctC-HA, H6-EctD). Each ORF transcription is controlled by the strong carbon source regulated FMD promoter. However, other strong regulative or constitutive promoters may be used as an alternative. *H*. *polymorpha* ALU3 *(ade1 leu2 odc1)* was transformed with NotI/PmeI fragments harboring the respective integration cassette, and plates were cultivated for six days. This procedure typically results in the isolation of strains harboring one single copy of the expression cassette at a defined integration locus. Proper integration was determined by analytical PCR using flanking PCR primers. A couple of strains resulting from this procedure were subjected to small scale methanol induction, and the total cell extracts were analyzed for the presence of gene products by SDS-PAGE/Western blot using tag-specific primary antibodies. After the first integration step the selection marker was removed using the cre/loxP recombinase system. The gene integration procedure was repeated until all four enzymes were properly co-expressed in *H. polymorpha* ALU3. The coexpression was analyzed via SDS PAGE/Western blot resulting in the strain ALU3/EctBACD #1 (odc1).

Expression of the various Ect genes in H. polymorpha from genomically integrated multicopy expression plasmids may also be an option.

Integration of respective expression cassettes into the genomes of other yeast expression organisms may require respective host-specific vectors and genetic elements, i.e. marker genes functional in those organisms and termini homologous to the integration locus of the respective organism. Depending on the particular expression organism it may be necessary to use non-integrative episomal plasmids.

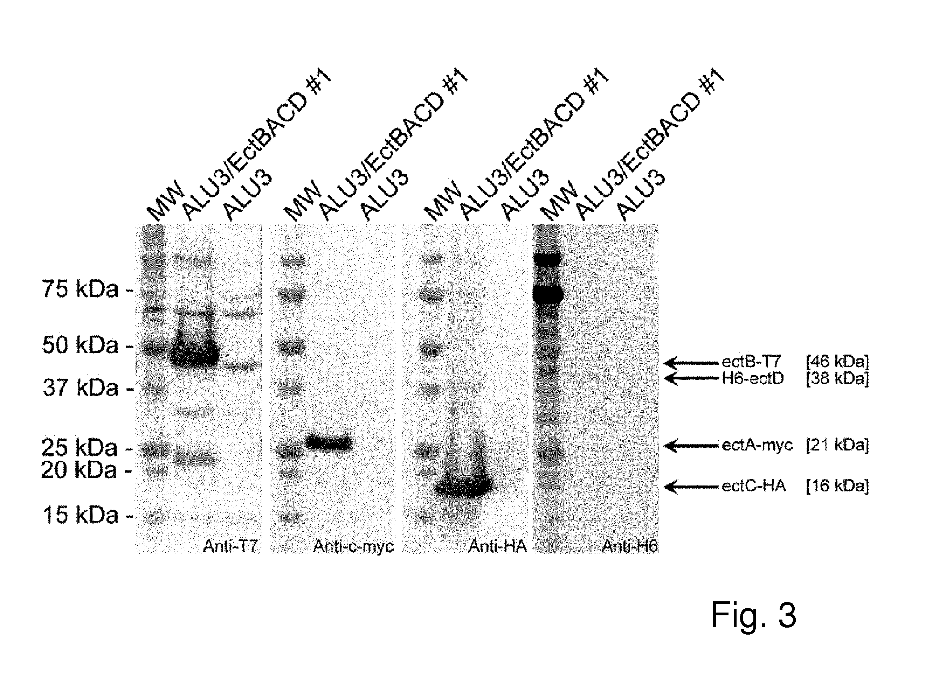

Fig. 3 shows the Western blot analysis of total cell extract of strain ALU3/EctBACD #1 coexpressing EctB, EctA, EctC and EctD. Cultivation was carried out as described above in Materials and Methods at 37°C in SYN6 at pH 5. After 96 h, a sample was taken and total cell extract has been prepared and analyzed by SDS-PAGE/Western blot under reducing conditions. The Ect proteins were then detected individually in the cell extract by four different Western blots using antibodies specific for the individual tags, respectively. The arrows indicate the positions of the bands corresponding to the various Ect genes, the corresponding calculated molecular weights are indicated in brackets. MW means molecular weight marker, and ALU3 is the wildtype strain.

### c) 5-hydroxyectoine is quantitatively synthesized from ectoine and is efficiently secreted or exported

After integration and expression of EctB, EctA, EctC and EctD, it was analyzed whether all enzymes are active. The strain ALU3/EctBACD #1 was cultivated at 37°C under derepressing/methanol induction conditions (YNB 2% (w/v) glycerol, induction after 48 h with 1% (v/v) methanol final concentration, total cultivation time 72 h). Analysis of the culture supernatant by HPLC showed that both compounds were synthesized by this strain in a ratio of 5-hydroxyectoine to ectoine of about 60:40. This ratio under non-optimized conditions was already higher than that in the homologous system *H. elongata,* where only a 50:50 ratio could be reached (see Seip et al., cited above). Preferably, the standard cultivation temperature for H. polymorpha of 37°C is used. However, in other embodiments other culturing temperatures may be applied to influence yield and ratio of 5-hydroxyectoine to ectoine.

### d) Efficient 5-hydroxyectoine synthesis is dependent on media pH

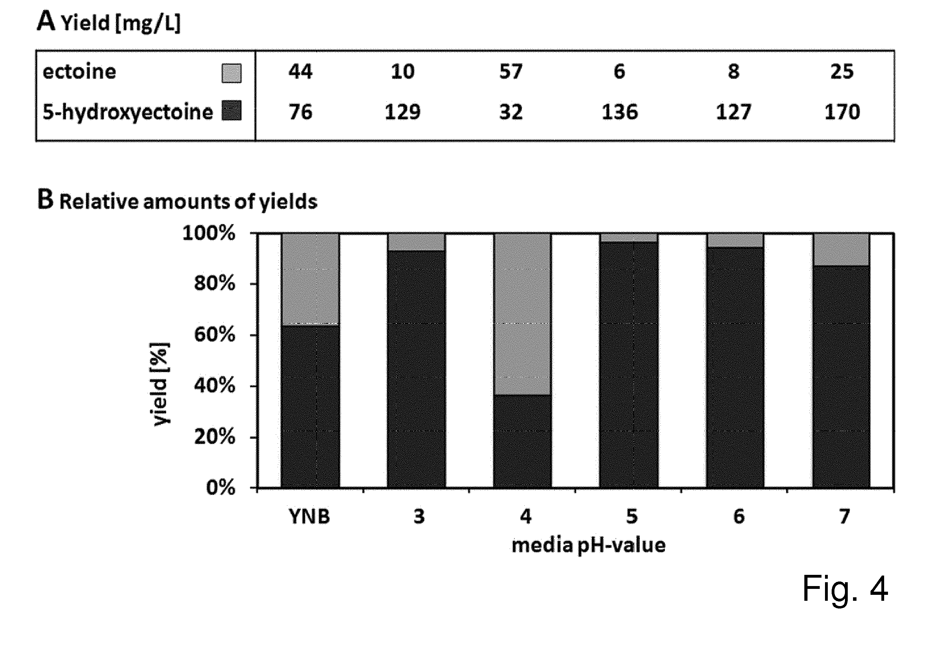

The optimal pH-values of EctA, EctB and EctC are within the range of 8.2 to 9.0 (Ono, H., Sawada, K., Khunajakr, N., Tao, T., Yamamoto, M., Hiramoto, M., Shinmyo, A., Takano, M., Murooka, Y.: Characterization of biosynthetic enzymes for ectoine as a compatible solute in a moderately halophilic eubacterium, Halomonas elongate; J. Bacteriol. 181 (1), 1999, p. 91-99); EctD has its pH optimum at 7.5 (Bursy, J., Pierik, A., Pica, N., Bremer, E.: Osmotically induced synthesis of the compatible solute hydroxyectoine is mediated by an evolutionarily conserved ectoine hydroxylase; J. Biol. Chem. 282 (43), 2007, p. 31147-31155). Hence, to further improve the 5-hydroxyectoine/ectoine ratio a variety of buffered YNB-based media of different pH-values from 3 to 7 were tested. Subsequent HPLC analysis of the culture supernatant showed a broad range of synthesis yields (see Fig. 4). In other expression organisms different conditions may be required.

Fig. 4 illustrates the determination of ectoine and 5-hydroxyectoine conversion yields of ALU3/EctBACD #1 in dependence of media pH-value during cultivation in 3ml scale. 5-hydroxyectoine producer ALU3/EctBACD #1 was cultivated at 37°C for a total of 72h in six different YNB-based media which were buffered at different pH-values ranging from 3 to 7 as indicated. Unbuffered YNB-medium with an initial pH-value of 6 was used as control medium. In all cases glycerol (2% w/v) was used as derepressing carbon source ("YNB"). After consumption of glycerol (after 48 h) a methanol induction was performed by adding methanol to a final concentration of 1% (v/v), respectively, and cultivation was continued for additional 24 h. In (A) the absolute values of ectoine and 5-hydroxyectoine in the culture supernatants as measured by HPLC are shown (light grey: ectoine, dark grey: 5-hydroxyectoine). In (B) a comparison of 5-hydroxyectoine/ectoine ratios is shown. The sum of 5-hydroxyectoine and ectoine has been defined as 100%, respectively, without considering the absolute values (which are shown in A). The values correspond to a single measurement each.

For further optimization of the culture conditions YNB pH 5 was chosen, since in this medium an increase of 5-hydroxyectoine yield (136 mg/l) and a low ectoine yield (10 mg/l) in a ratio of about 90:10 were combined. The media with pH 3, 6, 7 showed also a high potential towards 5-hydroxyectoine synthesis, however, in addition to the target compounds several side products were detected.

In the cases of other promoters controlling the Ect genes and/or other expression organisms pH values different from 5 and/or temperatures different from 37°C may be optimal.

### e) 5-hydroxyectoine formation is dependent on carbon source

Cultivations (of H. polymorpha) without methanol induction did not result in any conversion of ectoine to 5-hydroxyectoine. Thus methanol induction seems to be crucial for ectoine hydroxylation. In other yeast expression organisms this step may perform without methanol addition or by addition of another component to the culturing medium.

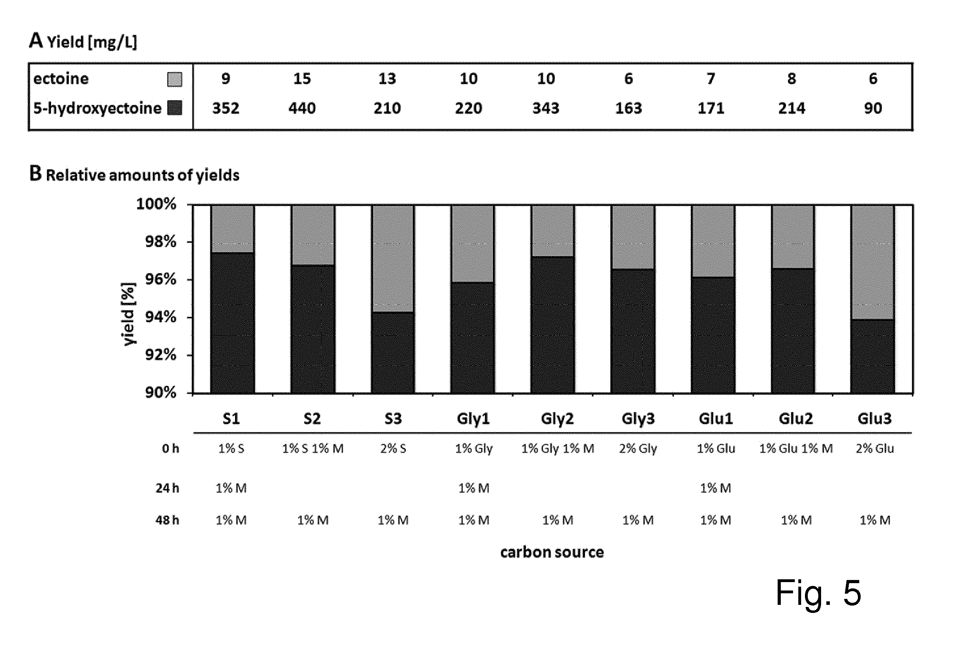

Beside glycerol also sorbitol was used as derepressing carbon source in combination with methanol (see Fig. 5). In addition glucose was tested, since at low concentrations it can also be considered a derepressing carbon source. After depletion of the carbon sources from the batch, the Ect genes were induced with methanol. However, the use of different promoters and/or different expression organisms may require the use of other carbon sources or inductors.

Fig. 5 shows the determination of ectoine and 5-hydroxyectoine conversion yields during cultivation of strain ALU3/EctBACD #1 in 3 mL scale in dependence of carbon source feeding mode. 5-hydroxyectoine producer ALU3/EctBACD #1 was cultivated at 37°C for a total of 72 h. All carbon source combinations were tested on the basis of buffered YNB medium, pH 5 (see also Fig. 3). The applied carbon source feeding profiles are indicated at the bottom of (B). In (A) the absolute values of ectoine and 5-hydroxyectoine in the culture supernatants as measured by HPLC are shown (light grey: ectoine, dark grey: 5-hydroxyectoine). In (B) a comparison of 5-hydroxyectoine/ectoine ratios is shown (for scheme and symbols see Fig. 3, (B)). Below the bars the course of the respective carbon source feeding mode and the respective carbon source(s) added at 0 h, 24 h, and 48 h (v/v, respectively) is (are) indicated. Feeding with 2% glycerol (G3) already used in the cultivations shown in Fig. 3 was the control condition. The values correspond to a single measurement each (Abbreviations: S = sorbitol; Gly = glycerol; Glu = glucose; M = methanol).

In general, a strong dependency of the synthesis yield on the methanol induction pattern was observed. Methanol feeding from cultivation start or early induction pulses starting after 24 h led to higher 5-hydroxyectoine synthesis yields (up to 440 mg/l for condition S2). Induction starting after 48 h resulted in significantly lower synthesis yields (compare conditions S3, Gly3 and Glu3). In addition, the 5-hydroxyectoine/ectoine ratio could be further improved to > 95% of 5-hydroxyectoine. In this regard condition S2 resulted in the highest 5-hydroxyectoine synthesis yield. Consequently, a fermentation process based on this condition was designed for upscaling of the 5-hydroxyectoine production in *H. polymorpha.*

### f) 5-hydroxyectoine is secreted or exported in gram per litre scale

Based on condition S2, we used the strain ALU3/EctBACD #1 for fermentation in 1 1 scale. In contrast to the previously optimized cultivation conditions a SYN6 medium, a synthetic minimal medium for yeast that is similar to YNB but achieves higher cell densities, was used. During cultivation the pH was maintained at 5 as in the best test tube conditions.

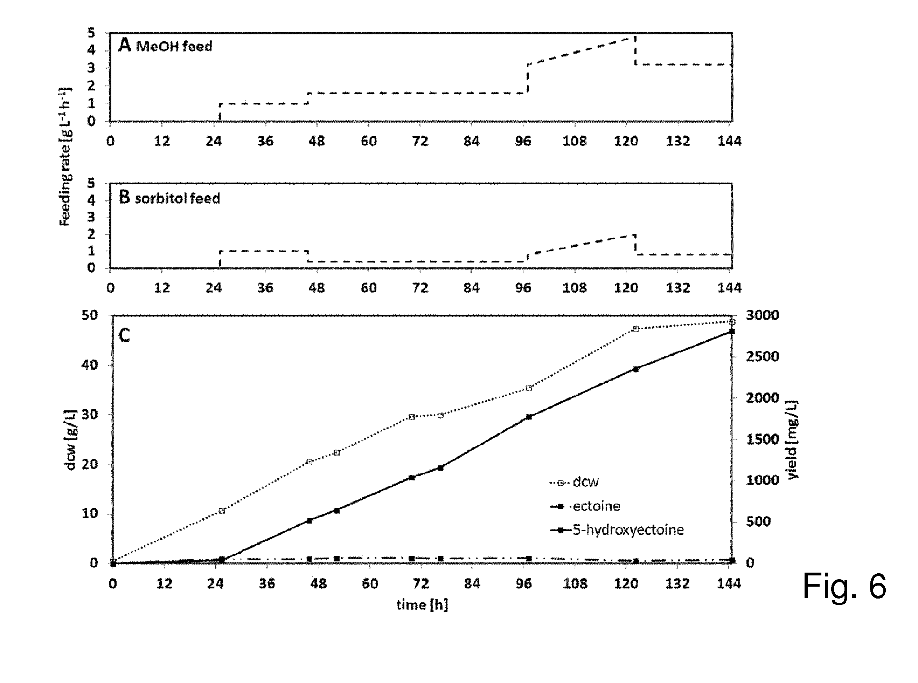

Fig. 6 is a diagram showing the fermentation of strain ALU3/EctBACD #1 at 1 litre scale. The fermentation was carried out as described in Materials and Methods at 37°C in SYN6 at pH 5. In (A) and (B) feeding modi are shown. 30 g/l sorbitol was provided as batch for initial cell growth. After consumption of the carbon source (assumed after 24 h) a [37.5% (w/v) sorbitol/37.5% (v/v) methanol] solution has been applied as a mixed feed with 2 g/l h⁻¹ was started. After 45 h the feeding solution was exchanged with a [15% (w/v) sorbitol/60% (v/v) methanol] solution without altering of the feeding rate. Between 96 h and 122 h the feeding rate with the latter feeding solution has been continuously increased from 4 to 6 g/l h⁻¹, as shown in (A) and (B). After 122 h the feeding rate has been reduced to 4 g/l h⁻¹. The different feeding solutions for the feed are indicated. In (C) corresponding courses of dry cell weight (dcw), and ectoine or 5-hydroxyectoine (mg/l) in the culture supernatant as measured by HPLC are shown. The values correspond to a single measurement each.

For initial growth, the medium was supplemented with 2% sorbitol. After 25 h, a mixed carbon source feed containing 1 g/l h⁻¹ methanol and 1 g/l h⁻¹ sorbitol was started. After 45 h the feed was switched to 1.6 g/l h⁻¹ methanol and 0.4 g/l h⁻¹ sorbitol for further activation of 5-hydroxyectoine synthesis. The carbon source feed was increased during fermentation (Fig. 6, (A) and (B)). Fermentation was stopped after 190 h. The dry cell weight (dcw) and yields for ectoine and 5-hydroxyectoine are shown in Fig. 6, (C).

Efficient conversion of ectoine to 5-hydroxyectoine starts with the mixed carbon source feed. The 5-hydroxyectoine formation thus clearly depends on methanol induction. After 144 h of fermentation the cells reached stationary phase, and the synthesis yield did not increase further. Analysis of the intracellular soluble fraction of the yeast cells showed that only fractions of ectoine and 5-hydroxyectoine were retained in the cell and thus no accumulation occurred.

Strain ALU3/EctBACD #1 produced 2.8 g/l 5-hydroxyectoine (364.5 µmol/g dcw at 144 h) in the culture supernatant with only 44 mg/l ectoine (6.3 µmol/g dcw at 144 h) as a side product. Thus the very high 5-hydroxyectoine yield of > 95% could be transferred from the test tube scale to the 1 1 scale.

It is likely that the use of promoters different from FMD promoter in H. polymorpha and/or the use of a different yeast as an expression organism may require adaptation of the upstream process adapted for the respective system.

Whole-cell biocatalysis provides an economic technique to produce compounds of chemical or pharmaceutical interest. Ectoine and 5-hydroxyectoine are biologicals of high value and mainly find application in skin care and medical products. *Hansenula polymorpha* (syn. *P. angusta*) provides a preferred system for the production of 5-hydroxyectoine.

For further optimization of the 5-hydroxyectoine synthesis in *H. polymorpha* several points need consideration. The precursor compound L-aspartate-semialdehyde is synthesized from aspartate by the two enzymes aspartatekinase (Ask) and L-aspartate-semialdehyde dehydrogenase (Asd). However, it is known that variants of Ask can be feedback sensitive to compounds of the aspartate pathway (methionine, lysine and threonine). The existence of a *H*. *polymorpha* Ask is supported by our finding that the 5-hydroxyectoine synthesis was downregulated when methionine, lysine or threonine was added during cultivation. Deletion of the putative feedback regulated Ask and expression of a feedback independent variant is an option for process improvement. Another interesting candidate for overexpression in H. polymorpha might be Ask_Lys derived from *H. elongata* of which the regulation is yet unknown but resulting 5-hydroxyectoine synthesis yields are high.

Surprisingly, it was found that in *H. polymorpha* strain ALU3/EctBACD #1 not the ectoine, but solely the 5-hydroxyectoine formation was methanol dependent.

Interestingly it was found that during fermentation the majority of ectoine as well as 5-hydroxyectoine were efficiently secreted to the culture supernatant with only a small fraction remaining inside the cell. The lack of an increase in salt resistance in the 5-hydroxyectoine producing *H. polymorpha* strains is in agreement with the finding that both ectoine and 5-hydroxyectoine are not accumulated in the cell. *H. polymorpha* is not a native producer of either of these compounds; nevertheless it obviously comprises an efficient transport system resulting in quantitative secretion of ectoine and 5-hydroxyectoine. In contrast to transporters derived from native ectoine producers, the transporter in *H. polymorpha* which is involved in ectoine and 5-hydroxyectoine secretion seems to be salt-independent.

In summary, 5-hydroxyectoine in gram per litre scale was synthesized by using *H. polymorpha* as a eukaryotic host without application of stress factors (e.g. high salinity) as for example necessary for *H. elongata.* Secretion or export of the product and a low rate of ectoine as a side product require a downstream process which is significantly simpler than the other systems described so far. Considering further possible options for improval of synthesis yield, *H. polymorpha* is a promising candidate for 5-hydroxyectoine production in industrial scale.

**Annex - Sequence Listings**

| | |
|---|---|
| SEQ ID 1 | protein sequence of EctA-myc |
| SEQ ID 2 | protein sequence of EctB-T7 |
| SEQ ID 3 | protein sequence of EctC-HA |
| SEQ ID 4 | protein sequence of H6-EctD |
| SEQ ID 5 | DNA sequence of plasmid pDh4-lx(As)-F-EctA-myc |
| SEQ ID 6 | DNA sequence of plasmid pDl-lx(As)-F-EctB-T7 |
| SEQ ID 7 | DNA sequence of plasmid pDu-lx(H4)-F-EctC-HA |
| SEQ ID 8 | DNA sequence of plasmid pDa-lx(Ls)-F-H6-EctD |
| SEQ ID 9 | DNA sequence of integration cassette Dh4-lx(As)-F-EctA-myc |
| SEQ ID 10 | DNA sequence of integration cassette Dl-lx(As)-F-EctB-T7 |
| SEQ ID 11 | DNA sequence of integration cassette Du-lx(H4)-F-EctC-HA |
| SEQ ID 12 | DNA sequence of integration cassette Da-lx(Ls)-F-H6-EctD |

## Claims

1. A method for producing ectoine or a derivative thereof, comprising:
(a) preparing host cells from a type of yeast, the host cells containing at least one recombinant DNA sequence encoding each of one or more heterologous enzymes for biosynthesis of ectoine;
(b) causing the host cells, with the aid of the recombinant DNA sequences encoding the enzymes, to express the heterologous enzymes; and
(c) causing the host cells, with the aid of the expressed heterologous enzymes, to synthesize ectoine and/or the derivative thereof from a precursor.

2. The method of claim 1, wherein the step (a) of preparing host cells comprises preparing host cells from at least one yeast type of the orders Saccharomycetales or Schizosaccharomycetales.

3. The method of claim 2, wherein the step (a) of preparing host cells comprises preparing host cells from at least one yeast type of the genera Pichia (Hansenula), Dekkera (Brettanomyces), Saccharomyces, Kluyveromyces, Candida, Blastobotrys (Arxula), Debaromyces (Schwanniomyces), and Yarrowia.

4. The method of claim 1, wherein the step (a) of preparing host cells comprises preparing host cells from at least one yeast type of the genus Pichia (Hansenula).

5. The method of claim 4, wherein the step (a) of preparing host cells comprises preparing host cells from at least one methylotrophic yeast type of the genus Pichia (Hansenula).

6. The method of claim 5, wherein the step (a) of preparing host cells comprises preparing host cells from Pichia angusta (Hansenula polymorpha).

7. The method of any of claims 1 - 6, wherein the step (a) of preparing host cells comprises preparing host cells from a type of yeast, the host cells containing
at least one recombinant DNA sequence encoding a DABA transaminase enzyme,
at least one recombinant DNA sequence encoding a DABA acetyltransferase enzyme, and
at least one recombinant DNA sequence encoding an ectoine synthase enzyme; and
wherein the step (b) of causing the host cells to express the heterologous enzymes comprises the step of causing the host cells to express the heterologous enzymes DABA transaminase, DABA acetyltransferase and ectoine synthase.

8. The method of claim 7, wherein the step (a) of preparing host cells comprises preparing host cells from a type of yeast, the host cells containing DNA sequences encoding the enzymes DABA-2-oxoglutarate transaminase (EctB), DABA acetyltransferase (EctA) and ectoine synthase (EctC) of the ectoine biosynthesis pathway of Halomonas elongata; and
wherein the step (b) of causing the host cells to express the heterologous enzymes comprises the step of causing the host cells to express the enzymes DABA-2-oxoglutarate transaminase (EctB), DABA acetyltransferase (EctA) and ectoine synthase (EctC) of the ectoine biosynthesis pathway of Halomonas elongata.

9. The method of any of claims 1 - 8, wherein the step (a) of preparing host cells comprises preparing host cells from a type of yeast, the host cells containing at least one further recombinant DNA sequence encoding a heterologous enzyme for the hydroxylation of ectoine to 5-hydroxyectoine; and
wherein the step (b) of causing the host cells to express the heterologous enzymes comprises the step of causing the host cells to additionally express the heterologous enzyme for the hydroxylation of ectoine to 5-hydroxyectoine.

10. The method of claim 9, wherein the step (a) of preparing host cells comprises preparing host cells from a type of yeast, the host cells containing additionally at least one recombinant DNA sequence encoding an ectoine hydroxylase enzyme; and
wherein the step (b) of causing the host cells to express the heterologous enzymes comprises the step of causing the host cells to additionally express the ectoine hydroxylase enzyme.

11. The method of claim 10, wherein the step (a) of preparing host cells comprises preparing host cells from a type of yeast, the host cells containing additionally at least one recombinant DNA sequence encoding the ectoine hydroxylase (EctD) of the ectoin biosynthesis pathway of Halomonas elongata; and
wherein the step (b) of causing the host cells to express the heterologous enzymes comprises the step of causing the host cells to additionally express the ectoine hydroxylase (EctD) of the ectoine biosynthesis pathway of Halomonas elongata.

12. The method of any of claims 1 - 11, wherein each of the at least one recombinant DNA sequence encoding each of one or more heterologous enzymes for biosynthesis of ectoine is contained in an expression cassette comprising a strong constitutive or inducible homologous promoter.

13. The method of claim 12, wherein the yeast is a methylotrophic yeast and the promoter is an inducible formate dehydrogenase (FMD) promoter.

14. The method of claim 13, wherein the step (b) of causing the host cells to express the heterologous enzymes comprises adding methanol to the culture.

15. The method of claims 13 or 14, wherein each of the at least one recombinant DNA sequence encoding each of one or more heterologous enzymes for biosynthesis of ectoine is contained in an expression cassette comprising a methanol oxidase (MOX) terminator.

16. The method of claims 12, wherein each of the at least one recombinant DNA sequence encoding each of one or more heterologous enzymes for biosynthesis of ectoine is contained in an expression cassette comprising a homologous terminator from a native gene different from the native gene of the homologous promoter.

17. The method of any of claims 1 - 16, wherein the step (b) of causing the host cells to express the heterologous enzymes comprises adding a derepressing or inducing agent.

18. The method of any of claims 1 - 17, wherein the step (c) of causing the host cells to synthesize ectoine and/or the derivative thereof from a precursor comprises adjusting a pH value between 3 and 7 in the culture.

19. The method of any of claims 1 - 18, comprising the additional steps of:
d) causing the host cells to export or secrete the synthesized ectoine and/or the derivative thereof to a culture supernatant, and
e) separating the exported or secreted ectoine and/or the derivative thereof from the culture supernatant.

20. A yeast cell of the orders Saccharomycetales or Schizosaccharomycetales for use as a host cell in a method for producing ectoine or a derivative thereof according to any of claims 1 to 16, the yeast cell containing at least one recombinant DNA sequence encoding each of one or more heterologous enzymes for biosynthesis of ectoine.

21. The yeast cell of claim 20, being a yeast cell of the genera Pichia (Hansenula), Dekkera (Brettanomyces), Saccharomyces, Kluyveromyces, Candida, Blastobotrys (Arxula), Debaromyces (Schwanniomyces), and Yarrowia.

22. The yeast cell of claim 21, being a methylotrophic yeast cell of the genus Pichia (Hansenula).

23. The yeast cell of claim 22, comprising at least one recombinant DNA sequence encoding a DABA transaminase enzyme, at least one recombinant DNA sequence encoding a DABA acetyltransferase enzyme, and at least one recombinant DNA sequence encoding an ectoine synthase enzyme.

24. The yeast cell of claim 23, comprising at least one further recombinant DNA sequence encoding for a heterologous enzyme for the hydroxylation of ectoine to 5-hydroxyectoine.

25. The yeast cell of any of claims 20 - 24, wherein each of the at least one recombinant DNA sequence encoding each of one or more heterologous enzymes for biosynthesis of ectoine is contained in an expression cassette comprising a strong constitutive or inducible homologous promoter.

26. The yeast cell of claim 25, wherein the yeast cell is methylotrophic and the promoter is a formate dehydrogenase (FMD) or a methanol oxidase (MOX) promoter.


SEQUENCE LISTING

<110> Artes Biotechnology GmbH

<120> A method for producing ectoine or a derivative thereof and a yeast cell for use
as a host cell in such a method

<130> A638EP

<160> 12

<170> BiSSAP 1.2

<210> 1
<211> 202
<212> PRT
<213> Hansenula polymorpha (syn. Pichia angusta)

<220>
<221> CHAIN
<222> 1..192
<223> EctA

<220>
<221> CHAIN
<222> 193..202
<223> myc tag

<400> 1
Met Asn Ala Thr Thr Glu Pro Phe Thr Pro Ser Ala Asp Leu Ala Lys
1               5                   10                  15
Pro Ser Val Ala Asp Ala Val Val Gly His Glu Ala Ser Pro Leu Phe
            20                  25                  30
Ile Arg Lys Pro Ser Pro Asp Asp Gly Trp Gly Ile Tyr Glu Leu Val
        35                  40                  45
Lys Ser Cys Pro Pro Leu Asp Val Asn Ser Ala Tyr Ala Tyr Leu Leu
    50                  55                  60
Leu Ala Thr Gln Phe Arg Asp Ser Cys Ala Val Ala Thr Asn Glu Glu
65                  70                  75                  80
Gly Glu Ile Val Gly Phe Val Ser Gly Tyr Val Lys Ser Asn Ala Pro
                85                  90                  95
Asp Thr Tyr Phe Leu Trp Gln Val Ala Val Gly Glu Lys Ala Arg Gly
            100                 105                 110
Thr Gly Leu Ala Arg Arg Leu Val Glu Ala Val Met Thr Arg Pro Glu
        115                 120                 125
Met Ala Glu Val His His Leu Glu Thr Thr Ile Thr Pro Asp Asn Gln
    130                 135                 140
Ala Ser Trp Gly Leu Phe Arg Arg Leu Ala Asp Arg Trp Gln Ala Pro

145                 150                 155                 160
Leu Asn Ser Arg Glu Tyr Phe Ser Thr Asp Gln Leu Gly Gly Glu His
                165                 170                 175
Asp Pro Glu Asn Leu Val Arg Ile Gly Pro Phe Gln Thr Asp Gln Ile
            180                 185                 190
Glu Gln Lys Leu Ile Ser Glu Glu Asp Leu
        195                 200

<210> 2
<211> 432
<212> PRT
<213> Hansenula polymorpha (syn. Pichia angusta)

<220>
<221> CHAIN
<222> 1..421
<223> EctB

<220>
<221> CHAIN
<222> 422..432
<223> T7 tag

<400> 2
Met Gln Thr Gln Ile Leu Glu Arg Met Glu Ser Asp Val Arg Thr Tyr
1               5                   10                  15
Ser Arg Ser Phe Pro Val Val Phe Thr Lys Ala Arg Asn Ala Arg Leu
            20                  25                  30
Thr Asp Glu Glu Gly Arg Glu Tyr Ile Asp Phe Leu Ala Gly Ala Gly
        35                  40                  45
Thr Leu Asn Tyr Gly His Asn Asn Pro His Leu Lys Gln Ala Leu Leu
    50                  55                  60
Asp Tyr Ile Asp Ser Asp Gly Ile Val His Gly Leu Asp Phe Trp Thr
65                  70                  75                  80
Ala Ala Lys Arg Asp Tyr Leu Glu Thr Leu Glu Glu Val Ile Leu Lys
                85                  90                  95
Pro Arg Gly Leu Asp Tyr Lys Val His Leu Pro Gly Pro Thr Gly Thr
            100                 105                 110
Asn Ala Val Glu Ala Ala Ile Arg Leu Ala Arg Val Ala Lys Gly Arg
        115                 120                 125
His Asn Ile Val Ser Phe Thr Asn Gly Phe His Gly Val Thr Met Gly
    130                 135                 140
Ala Leu Ala Thr Thr Gly Asn Arg Lys Phe Arg Glu Ala Thr Gly Gly
145                 150                 155                 160
Val Pro Thr Gln Ala Ala Ser Phe Met Pro Phe Asp Gly Tyr Leu Gly
                165                 170                 175
Ser Ser Thr Asp Thr Leu Asp Tyr Phe Glu Lys Leu Leu Gly Asp Lys
            180                 185                 190
Ser Gly Gly Leu Asp Val Pro Ala Ala Val Ile Val Glu Thr Val Gln

195                 200                 205
Gly Glu Gly Gly Ile Asn Val Ala Gly Leu Glu Trp Leu Lys Arg Leu
    210                 215                 220
Glu Ser Ile Cys Arg Ala Asn Asp Ile Leu Leu Ile Ile Asp Asp Ile
225                 230                 235                 240
Gln Ala Gly Cys Gly Arg Thr Gly Lys Phe Phe Ser Phe Glu His Ala
                245                 250                 255
Gly Ile Thr Pro Asp Ile Val Thr Asn Ser Lys Ser Leu Ser Gly Tyr
            260                 265                 270
Gly Leu Pro Phe Ala His Val Leu Met Arg Pro Glu Leu Asp Lys Trp
        275                 280                 285
Lys Pro Gly Gln Tyr Asn Gly Thr Phe Arg Gly Phe Asn Leu Ala Phe
    290                 295                 300
Ala Thr Ala Ala Ala Ala Met Arg Lys Tyr Trp Ser Asp Asp Thr Phe
305                 310                 315                 320
Glu Arg Asp Val Gln Arg Lys Ala Arg Ile Val Glu Glu Arg Phe Gly
                325                 330                 335
Lys Ile Ala Ala Trp Leu Ser Glu Asn Gly Ile Glu Ala Ser Glu Arg
            340                 345                 350
Gly Arg Gly Leu Met Arg Gly Ile Asp Val Gly Ser Gly Asp Ile Ala
        355                 360                 365
Asp Lys Ile Thr His Gln Ala Phe Glu Asn Gly Leu Ile Ile Glu Thr
    370                 375                 380
Ser Gly Gln Asp Gly Glu Val Val Lys Cys Leu Cys Pro Leu Thr Ile
385                 390                 395                 400
Pro Asp Glu Asp Leu Val Glu Gly Leu Asp Ile Leu Glu Thr Ser Thr
                405                 410                 415
Lys Gln Ala Phe Ser Met Ala Ser Met Thr Gly Gly Gln Gln Met Gly
            420                 425                 430

<210> 3
<211> 146
<212> PRT
<213> Hansenula polymorpha (syn. Pichia angusta)

<220>
<221> CHAIN
<222> 1..137
<223> EctC

<220>
<221> CHAIN
<222> 138..146
<223> HA tag

<400> 3
Met Ile Val Arg Asn Leu Glu Glu Ala Arg Gln Thr Asp Arg Leu Val

1                   5                   10                  15
Thr Ala Glu Asn Gly Asn Trp Asp Ser Thr Arg Leu Ser Leu Ala Glu
                20                  25                  30
Asp Gly Gly Asn Cys Ser Phe His Ile Thr Arg Ile Phe Glu Gly Thr
        35                  40                  45
Glu Thr His Ile His Tyr Lys His His Phe Glu Ala Val Tyr Cys Ile
    50                  55                  60
Glu Gly Glu Gly Glu Val Glu Thr Leu Ala Asp Gly Lys Ile Trp Pro
65                  70                  75                  80
Ile Lys Pro Gly Asp Ile Tyr Ile Leu Asp Gln His Asp Glu His Leu
                85                  90                  95
Leu Arg Ala Ser Lys Thr Met His Leu Ala Cys Val Phe Thr Pro Gly
            100                 105                 110
Leu Thr Gly Asn Glu Val His Arg Glu Asp Gly Ser Tyr Ala Pro Ala
        115                 120                 125
Asp Glu Ala Asp Asp Gln Lys Pro Leu Tyr Pro Tyr Asp Val Pro Asp
    130                 135                 140
Tyr Ala
145

<210> 4
<211> 340
<212> PRT
<213> Hansenula polymorpha (syn. Pichia angusta)

<220>
<221> CHAIN
<222> 1..1
<223> start methionine

<220>
<221> CHAIN
<222> 2..9
<223> His tag and flanking residues

<220>
<221> CHAIN
<222> 10..340
<223> EctD(2-332)

<400> 4
Met Lys His His His His His His Gln Ser Val Gln Thr Ser Ser Asn
1               5                   10                  15
Arg Pro Leu Pro Gln Ala Asn Leu His Ile Ala Thr Glu Thr Pro Glu
            20                  25                  30
Ala Asp Ser Arg Ile Arg Ser Ala Pro Arg Pro Gly Gln Asp Pro Tyr
        35                  40                  45
Pro Thr Arg Leu Ser Glu Pro Leu Asp Leu Pro Trp Leu Asn Arg Arg
    50                  55                  60

Glu Pro Val Val Lys Gly Glu Glu Ala Asp Gly Pro Leu Ser Ala Ala
65                  70                  75                  80
Gln Leu Asp Thr Phe Glu Arg Gln Gly Phe Ile Phe Glu Pro Asp Phe
                85                  90                  95
Leu Lys Gly Glu Glu Leu Glu Ala Leu Arg His Glu Leu Asn Ala Leu
            100                 105                 110
Leu Ala Arg Asp Asp Phe Arg Gly Arg Asp Phe Ala Ile Thr Glu Pro
        115                 120                 125
Gln Gly Asn Glu Ile Arg Ser Leu Phe Ala Val His Tyr Leu Ser Arg
    130                 135                 140
Val Phe Ser Arg Leu Ala Asn Asp Glu Arg Leu Met Gly Arg Ala Arg
145                 150                 155                 160
Gln Ile Leu Gly Gly Glu Pro Tyr Val His Gln Ser Arg Ile Asn Tyr
                165                 170                 175
Lys Pro Gly Phe Glu Gly Lys Gly Phe Asn Trp His Ser Asp Phe Glu
            180                 185                 190
Thr Trp His Ala Glu Asp Gly Met Pro Ala Met His Ala Val Ser Ala
        195                 200                 205
Ser Ile Val Leu Thr Asp Asn His Thr Phe Asn Gly Pro Leu Met Leu
    210                 215                 220
Val Pro Gly Ser His Arg Val Phe Val Pro Cys Leu Gly Glu Thr Pro
225                 230                 235                 240
Glu Asp His His Arg Gln Ser Leu Lys Thr Gln Glu Phe Gly Val Pro
                245                 250                 255
Ser Arg Gln Ala Leu Arg Glu Leu Ile Asp Arg His Gly Ile Glu Ala
            260                 265                 270
Pro Thr Gly Ala Ala Gly Gly Leu Leu Leu Phe Asp Cys Asn Thr Leu
        275                 280                 285
His Gly Ser Asn Ala Asn Met Ser Pro Asp Pro Arg Ser Asn Ala Phe
    290                 295                 300
Phe Val Tyr Asn Arg Arg Asp Asn Arg Cys Val Glu Pro Tyr Ala Ala
305                 310                 315                 320
Ser Lys Arg Arg Pro Arg Phe Leu Ala His Glu Pro Asp Glu Ala Trp
                325                 330                 335
Ser Pro Asp Gly
            340

<210> 5
<211> 8724
<212> DNA
<213> Hansenula polymorpha (syn. Pichia angusta)

<220>
<221> source
<222> 1..8724
<223> /mol_type="unassigned DNA"
      /organism="Hansenula polymorpha (syn. Pichia angusta)"

<220>
<221> promoter

<222> 7..1072
<223> /note="FMD promoter"

<220>
<221> misc_feature
<222> 1079..1112
<223> /note="loxP site"

<220>
<221> gene
<222> 1304..2215
<223> /note="Synthetic ADE1 gene; codons optimized for Hansenula
      polymorpha"

<220>
<221> misc_feature
<222> 2479..3510
<223> /note="HIS4 upstream region for homologous recombination of the
      integration cassette"

<220>
<221> gene
<222> 4603..5475
<223> /note="AmpR; Ampicillin resistence gene"

<220>
<221> misc_feature
<222> 6673..7770
<223> /note="HIS4 downstream region for homologous recombination of the
      integration cassette"

<220>
<221> terminator
<222> 7778..8105
<223> /note="MOX-T; teminator of the MOX gene"

<220>
<221> gene
<222> 8115..8723
<223> /note="EctA-myc fusion ORF (EctA coding region/myc tag coding
      region)"

<400> 5
aattcccgat gaaggcagag agcgcaagga ggcggtattt atagtgccat tcccctctct      60

gagagacccg gatggtagtc gagtgtatcg gagacagctt gatgtagact ccgtgcctgc     120

cggctcctct tattggcgga caccagtgag acaccccgga acttgctgtt tttctgcaaa     180

atccggggtg accagtggga gcctatttgc acacacgagc gggacacccc actctggtga     240

agagtgccaa agtcattctt tttcccgttg cggggcagcc gattgcatgt tttaggaaaa 300

tattaccttt gctacaccct gtcagattta ccctccacac atatatattc cgtcacctcc 360

agggactatt attcgtcgtt gcgccgccag cggaagatat ccagaagctg ttttccgaga 420

gactcggttg gcgcctggta tatttgatgg atgtcgcgct gcctcacgtc ccggtaccca 480

ggaacgcggt gggatctcgg gcccatcgaa gactgtgctc cagactgctc gcccagcagg 540

tgtttcttga tcgccgcctc taaattgtcc gcgcatcgcc ggtaacattt ttccagctcg 600

gagtttgcgt ttagatacag tttctgcgat gccaaaggag cctgcagatt ataacctcgg 660

atgctgtcat tcagcgcttt taatttgacc tccagatagt tgctgtattt ctgttcccat 720

tggctgctgc gcagcttcgt ataactcgag ttattgttgc gctctgcctc ggcgtactgg 780

ctcatgatct ggatcttgtc cgtgtcgctt ttcttcgagt gtttctcgca aacgatgtgc 840

acggcctgca gtgtccaatc ggagtcgagc tggcgccgaa actggcggat ctgagcctcc 900

acactgccct gtttctctat ccacggcgga accgcctcct gccgtttcag aatgttgttc 960

aagtggtact ctgtgcggtc aatgaaggcg ttattgccgg tgaaatcttt gggaagcggt 1020

tttcctcggg gaagattacg aaattccccg cgtcgttgcg cttcctggat ctcgatcgat 1080

aacttcgtat agcatacatt atacgaagtt atatgcgcac tctagattcc ttgcgtacaa 1140

acacctttcc gtcagcgaca cgccttactt tgcgcacaca tccgaacgag cctcgtccga 1200

tgagctccaa cacttggaaa tcgtccatcg aatagtatat ttacaaactt ccggatacag 1260

aaaataatta aattatttgg gacagcttgg tttatttcaa cacttaagcc caggttttac 1320

cggtcaaagc ctcgtaagcc tctttgtact tagcggcggt cttagcagca atctcctcgg 1380

tcatagacac accctcttta ccgttcagct ggtgagaagt caaccagtct ctcaggaact 1440

gcttgtcgaa agactcctga gatcttccca actcgtactt cttggcgtcc caaaatctcg 1500

aagagtctgg ggtcaaaacc tcgtcaacca gaaccagctc gttgttctcg ttcagtccga 1560

actcaaactt ggtgtcggcc aggataatac ctctcgacag agcgtagtct ctagccttag 1620

agtacagggc aatagccttc tgggcaacct cagcacacaa ctcctctcca accaactgag 1680

cagcctgctc tggagaaatg ttctcgtcgt gctgtccctg ctctgctttg gtagatgggg 1740

tgaagattgg cttctcgaaa gcctcagact cctgcaatcc agacttaact ggcagtccgt 1800

gaacggtaga cgacttcttg tactctttcc aggcagaacc ggtgatgtaa cctctcacaa 1860

tggcctccaa tggaaccagc ttgtgcttct tcaccagcag agatctacca gccaactgag 1920

ccttgtactt tggctcagac aactgctctg gcaacttggc aaagatgtcc ttgtcagagg 1980

aagcaaccag gtggtttgga ataacgtcag acagcaggtt gaaccagaac acagacagct 2040

gggtcagaat ctttcccttg tccttgatgc cgttctccat aatcacgtcg taggcagaga 2100

ttctgtcggt ggcaacaaac agcagcatgt tgtcgtccac ctggtagatg tctctcactt 2160

tacctctggc aaccaatggc agaataccgt ccaggttagt ctgggtcacg gtcatatgtg 2220

taataataat taagtaaaat tagtttagta aattaattga ctcgtgaggg tcgatcgacg 2280

cgatattggc tctccttttt atacgagttt ttgtcaactt tcctctgcta tgctcaatcc 2340

agtcaccgtc cacaaagact tctttgggtt tcctggggcc ttggttctga ccatcagtct 2400

acctttggtg gttgttttcc tgtatgcggc gtcgacggcg cgccataact tcgtatagca 2460

tacattatac gaagttatcc tgcaggttat gaatttacgg tgagatgtga gtcaagctga 2520

aaaatatctt gatttttttt tttttgacat gccacgactt tggtagcatg ggacgatata 2580

tcactatata cactgtgagt cagttattga acaaaaactg catatcggcc gccgatagcc 2640

tgctagccgc gttctggtcc tgatccagcg tggccttgac catgtcagcc ttcttctgct 2700

gcagctcaat gatccgcgac tcgatgctgt cctcgatgac caacttcaca attctgatag 2760

gccggtgctg tccaatccgg tgaattctgt ctgcggcctg acccgtgtcc aaagcagggt 2820

tccaccaact atccaggata aatacttgat tggcctcgac aagattcaga gccacacctc 2880

cggccttcaa agacaccaaa aatacctcca ccgacggatt ggtcataaag tgctggatca 2940

cagagtcacg ctggatcggg gtcatggatc cctgcaattt cgcaatggaa aaacccgccc 3000

ttttgagtcg ccactccacc aggtccagca tcgacgtgaa ctgcgaaaag acaatgcttt 3060

taacagtttg tctgtccgaa cgggacttgt acaactcttc cattagggcc tcaattttag 3120

tggaagactt ccattcgcct cccatgtcta ttcggttcac aatagagccc ttctcaacga 3180

cctcctggtt catctccagc gctggtgcct ccagatcaat actcagccca atgtggcaca 3240

ctggacactc gagacgctta tcgtcgccgt tgaacgactc cacgtactcc gtgatacaga 3300

tccggcagaa cttgtggtga catttggact ctatcggctc ctctgcttcg tcatcacaca 3360

gctggcagac aatggccccg ctgttgagga gatccctgga tgctttctta aatcgcttga 3420

gaaccagatc cggatgatcc gcaagctgtc tcatacgcgt gattagggta aagatgttgg 3480

catagttgtt cagaaccaca ccttgtgcag cggccgcgca tgcggtctct agcttggcac 3540

tggccgtcgt tttacaacgt cgtgactggg aaaaccctgg cgttacccaa cttaatcgcc 3600

ttgcagcaca tccccctttc gccagctggc gtaatagcga agaggcccgc accgatcgcc 3660

cttcccaaca gttgcgcagc ctgaatggcg aatggcgcct gatgcggtat tttctcctta 3720

cgcatctgtg cggtatttca caccgcatat gctcgtcaaa gcaaccatag tacgcgccct 3780

gtagcggcgc attaagcgcg gcgggtgtgg tggttacgcg cagcgtgacc gctacacttg 3840

ccagcgccct agcgcccgct cctttcgctt tcttcccttc ctttctcgcc acgttcgccg 3900

gctttccccg tcaagctcta aatcgggggc tccctttagg gttccgattt agtgctttac 3960

ggcacctcga ccccaaaaaa cttgatttgg gtgatggttc acgtagtggg ccatcgccct 4020

gatagacggt ttttcgccct ttgacgttgg agtccacgtt ctttaatagt ggactcttgt 4080

tccaaactgg aacaacactc aaccctatct cgggctattc ttttgattta taagggattt 4140

tgccgatttc ggcctattgg ttaaaaaatg agctgattta acaaaaattt aacgcgaatt 4200

ttaacaaaat attaacgttt acaattttat ggtgcactct cagtacaatc tgctctgatg 4260

ccgcatagtt aagccagccc cgacacccgc caacacccgc tgacgcgccc tgacgggctt 4320

gtctgctccc ggcatccgct tacagacaag ctgtgaccgt ctccgggagc tgcatgtgtc 4380

agaggttttc accgtcatca ccgaaacgcg cgaggacgaa agggcctcgt gatacgccta 4440

tttttatagg ttaatgtcat gataataatg gtttcttaga cgtcaggtgg cacttttcgg 4500

ggaaatgtgc gcggaacccc tatttgttta tttttctaaa tacattcaaa tatgtatccg 4560

ctcatgagac aataaccctg ataaatgctt caataatatt gaaaaaggaa gagtatgagt 4620

attcaacatt tccgtgtcgc ccttattccc ttttttgcgg cattttgcct tcctgttttt 4680

gctcacccag aaacgctggt gaaagtaaaa gatgctgaag atcagttggg tgcacgagtg 4740

ggttacatcg aactggatct caacagcggt aagatccttg agagttttcg ccccgaagaa 4800

cgttttccaa tgatgagcac ttttaaagtt ctgctatgtg gcgcggtatt atcccgtatt 4860

gacgccgggc aagagcaact cggtcgccgc atacactatt ctcagaatga cttggttgag 4920

tactcaccag tcacagaaaa gcatcttacg gatggcatga cagtaagaga attatgcagt 4980

gctgccataa ccatgagtga taacactgcg gccaacttac ttctgacaac gatcggagga 5040

ccgaaggagc taaccgcttt tttgcacaac atgggggatc atgtaactcg ccttgatcgt 5100

tgggaaccgg agctgaatga agccatacca aacgacgagc gtgacaccac gatgcctgta 5160

gcaatggcaa caacgttgcg caaactatta actggcgaac tacttactct agcttcccgg 5220

caacaattaa tagactggat ggaggcggat aaagttgcag gaccacttct gcgctcggcc 5280

cttccggctg gctggtttat tgctgataaa tctggagccg gtgagcgtgg gtctcgcggt 5340

atcattgcag cactggggcc agatggtaag ccctcccgta tcgtagttat ctacacgacg 5400

gggagtcagg caactatgga tgaacgaaat agacagatcg ctgagatagg tgcctcactg 5460

attaagcatt ggtaactgtc agaccaagtt tactcatata tactttagat tgatttaaaa 5520

cttcattttt aatttaaaag gatctaggtg aagatccttt ttgataatct catgaccaaa 5580

atcccttaac gtgagttttc gttccactga gcgtcagacc ccgtagaaaa gatcaaagga 5640

tcttcttgag atcctttttt tctgcgcgta atctgctgct tgcaaacaaa aaaaccaccg 5700

ctaccagcgg tggtttgttt gccggatcaa gagctaccaa ctctttttcc gaaggtaact 5760

ggcttcagca gagcgcagat accaaatact gtccttctag tgtagccgta gttaggccac 5820

cacttcaaga actctgtagc accgcctaca tacctcgctc tgctaatcct gttaccagtg 5880

gctgctgcca gtggcgataa gtcgtgtctt accgggttgg actcaagacg atagttaccg 5940

gataaggcgc agcggtcggg ctgaacgggg ggttcgtgca cacagcccag cttggagcga 6000

acgacctaca ccgaactgag atacctacag cgtgagcatt gagaaagcgc cacgcttccc 6060

gaagggagaa aggcggacag gtatccggta agcggcaggg tcggaacagg agagcgcacg 6120

agggagcttc cagggggaaa cgcctggtat ctttatagtc ctgtcgggtt tcgccacctc 6180

tgacttgagc gtcgattttt gtgatgctcg tcaggggggc ggagcctatg gaaaaacgcc 6240

agcaacgcgg cctttttacg gttcctggcc ttttgctggc cttttgctca catgttcttt 6300

cctgcgttat cccctgattc tgtggataac cgtattaccg cctttgagtg agctgatacc 6360

gctcgccgca gccgaacgac cgagcgcagc gagtcagtga gcgaggaagc ggaagagcgc 6420

ccaatacgca aaccgcctct ccccgcgcgt tggccgattc attaatgcag ctggcacgac 6480

aggtttcccg actggaaagc gggcagtgag cgcaacgcaa ttaatgtgag ttagctcact 6540

cattaggcac cccaggcttt acactttatg cttccggctc gtatgttgtg tggaattgtg 6600

agcggataac aatttcacac aggaaacagc tatgaccatg attacgaatt gagctcggta 6660

cccggcctcg aggtttaaac gaggattctg gccctacaac tgcaaagaag ctgtgcgagg 6720

gcacgctcgt cgtttgggcc agccaatgtg ggcaacaatc tcgccaacct ggacgttttg 6780

ggctcttctc cagtgcctga gaacaatatc gagatgatct ctaaggactc gattatcttc 6840

tccaacatga aggttgtgcg atcgcccaac aggcaaggga ctcttgttgc tgctctgttg 6900

ttggataacc agatatttga gctcaacaca aagtccatca gcaaaaaagg cccattggat 6960

ttgacaacta ctgagctctt tcccttgtta gaggcgctct acccaaaacc cgagctgttt 7020

gtgctgggtc ttgggttcag caaccggatg ttacacccag acacgaaaag acgcttttcc 7080

gagttgggga tcatgctcga agtaggcgac accaagagcg cagtgctcag ctacgatctt 7140

ctggctacgg aacgttctcc aaaacaggta ggggcactca tatttccatc ggtgttctga 7200

aatatgcgcg aaacaactat ttaagggctc tgaccaggaa ttgcgtgctg aacacatagg 7260

agtaggcagc aagggtaagg acgctcagac tgttgaacac gaggctgatt ccatggtact 7320

tgccgaactt cttggtcaaa gctgcgattt tttcgttggt ctcgggagtt ctctcgctct 7380

ctggcagttc ctcgagcgca aagcgttcag tcttcagccg gttgcacact ggcatcagcc 7440

acaagtaatt ggtgagacct gagagcgaag aggccgccag caatgcaatt ccgacggttg 7500

ggatcgtgta tggtgcagag agaccgacca ccacggggcc gatcgactgc gtcaggaagt 7560

ggatcgggaa cagcttggat tgcaggaggc caaactcctt gcggggcagg accttgaacg 7620

ctctgacaga ggacacaaat gagtggaacg aagtggctcc gaaaacatac gagtaagcaa 7680

gcaggtgcac agcagtcagc atggtaaaaa ttaattgcac ttattattta tttggtcgtg 7740

gctttattta atcagtgata attgtcacgt taattaacaa tctccggaat ggtgatctga 7800

tcgttcctga aaacctcgac attggctccc tcctgacaca ggtactcgta caggttccag 7860

gtaaacgagt cgtagttgtc gatcatgaca acgttcttag aagcggccgg cattttgaag 7920

gtgactaata gcctaagaaa atatttaatt taattttcat taaattttcc tatactcgct 7980

atttcagctt ttcatctcat cacttcataa acgatataaa ccagaaaaag aactattttc 8040

aaacacgctt ctcaaaagcg gtatgtcctt ccacgtctcc ttagaatctg gcaagtccgc 8100

gagggggatc cttactacag gtcttcctca gaaatcagct tctgctcaat ctggtcggtc 8160

tggaatggac caattctaac caagttctct gggtcgtgct caccacccaa ctggtcggta 8220

gaaaagtact ctctagagtt caatggggcc tgccatctgt cagccaatct tctaaacagg 8280

ccccaagaag cctggttgtc tggagtaatg gtagtctcca agtggtgaac ctcagccatc 8340

tctggtctgg tcataacggc ctcaaccaat cttctagcca gaccagtacc tctagccttc 8400

tcaccaacag caacctgcca caaaaagtag gtgtccggag cgttagactt cacgtaacca 8460

gacacgaaac caacgatctc accttcctcg ttggtagcaa cagcacaaga gtctctgaac 8520

tgggtagcca gcaacaggta agcgtaagca gagttaacgt ccagcggtgg acaagactta 8580

accaactcgt agatacccca gccgtcgtct ggagatggtt ttctgatgaa cagtggagaa 8640

gcctcgtgac caacaacagc gtcagcaaca gatggcttag ccaagtcagc agatggagtg 8700

aatggctcgg tggtagcgtt catg 8724

<210> 6
<211> 9360
<212> DNA
<213> Hansenula polymorpha (syn. Pichia angusta)

<220>
<221> source
<222> 1..9360
<223> /mol_type="unassigned DNA"
      /organism="Hansenula polymorpha (syn. Pichia angusta)"

<220>
<221> gene
<222> 190..1062

<223> /note="AmpR; Ampicillin resistence gene"

<220>
<221> misc_feature
<222> 2260..3360
<223> /note="LEU2 downstream region for homologous recombination of the
      integration cassette"

<220>
<221> terminator
<222> 3368..3695
<223> /note="MOX-T; teminator of the MOX gene"

<220>
<221> gene
<222> 3705..5003
<223> /note="EctB-T7 fusion ORF (EctB coding region/T7 tag coding
      region)"

<220>
<221> promoter
<222> 5011..6076
<223> /note="FMD promoter"

<220>
<221> misc_feature
<222> 6083..6116
<223> /note="loxP site"

<220>
<221> gene
<222> 6308..7219
<223> /note="Synthetic ADE1 gene; codons optimized for Hansenula
      polymorpha"

<220>
<221> misc_feature
<222> 7449..7482
<223> /note="loxP site"

<220>
<221> misc_feature
<222> 7483..8457
<223> /note="LEU2 upstream region for homologous recombination of the
      integration cassette"

<400> 6
ggacgaaagg gcctcgtgat acgcctattt ttataggtta atgtcatgat aataatggtt      60

tcttagacgt caggtggcac ttttcgggga aatgtgcgcg gaacccctat ttgtttattt      120

ttctaaatac attcaaatat gtatccgctc atgagacaat aaccctgata aatgcttcaa 180

taatattgaa aaaggaagag tatgagtatt caacatttcc gtgtcgccct tattcccttt 240

tttgcggcat tttgccttcc tgtttttgct cacccagaaa cgctggtgaa agtaaaagat 300

gctgaagatc agttgggtgc acgagtgggt tacatcgaac tggatctcaa cagcggtaag 360

atccttgaga gttttcgccc cgaagaacgt tttccaatga tgagcacttt taaagttctg 420

ctatgtggcg cggtattatc ccgtattgac gccgggcaag agcaactcgg tcgccgcata 480

cactattctc agaatgactt ggttgagtac tcaccagtca cagaaaagca tcttacggat 540

ggcatgacag taagagaatt atgcagtgct gccataacca tgagtgataa cactgcggcc 600

aacttacttc tgacaacgat cggaggaccg aaggagctaa ccgctttttt gcacaacatg 660

ggggatcatg taactcgcct tgatcgttgg gaaccggagc tgaatgaagc cataccaaac 720

gacgagcgtg acaccacgat gcctgtagca atggcaacaa cgttgcgcaa actattaact 780

ggcgaactac ttactctagc ttcccggcaa caattaatag actggatgga ggcggataaa 840

gttgcaggac cacttctgcg ctcggccctt ccggctggct ggtttattgc tgataaatct 900

ggagccggtg agcgtgggtc tcgcggtatc attgcagcac tggggccaga tggtaagccc 960

tcccgtatcg tagttatcta cacgacgggg agtcaggcaa ctatggatga acgaaataga 1020

cagatcgctg agataggtgc ctcactgatt aagcattggt aactgtcaga ccaagtttac 1080

tcatatatac tttagattga tttaaaactt catttttaat ttaaaaggat ctaggtgaag 1140

atcctttttg ataatctcat gaccaaaatc ccttaacgtg agttttcgtt ccactgagcg 1200

tcagaccccg tagaaaagat caaaggatct tcttgagatc ctttttttct gcgcgtaatc 1260

tgctgcttgc aaacaaaaaa accaccgcta ccagcggtgg tttgtttgcc ggatcaagag 1320

ctaccaactc tttttccgaa ggtaactggc ttcagcagag cgcagatacc aaatactgtc 1380

cttctagtgt agccgtagtt aggccaccac ttcaagaact ctgtagcacc gcctacatac 1440

ctcgctctgc taatcctgtt accagtggct gctgccagtg gcgataagtc gtgtcttacc 1500

gggttggact caagacgata gttaccggat aaggcgcagc ggtcgggctg aacggggggt 1560

tcgtgcacac agcccagctt ggagcgaacg acctacaccg aactgagata cctacagcgt 1620

gagcattgag aaagcgccac gcttcccgaa gggagaaagg cggacaggta tccggtaagc 1680

ggcagggtcg gaacaggaga gcgcacgagg gagcttccag ggggaaacgc ctggtatctt 1740

tatagtcctg tcgggtttcg ccacctctga cttgagcgtc gatttttgtg atgctcgtca 1800

ggggggcgga gcctatggaa aaacgccagc aacgcggcct ttttacggtt cctggccttt 1860

tgctggcctt ttgctcacat gttctttcct gcgttatccc ctgattctgt ggataaccgt 1920

attaccgcct ttgagtgagc tgataccgct cgccgcagcc gaacgaccga gcgcagcgag 1980

tcagtgagcg aggaagcgga agagcgccca atacgcaaac cgcctctccc cgcgcgttgg 2040

ccgattcatt aatgcagctg gcacgacagg tttcccgact ggaaagcggg cagtgagcgc 2100

aacgcaatta atgtgagtta gctcactcat taggcacccc aggctttaca ctttatgctt 2160

ccggctcgta tgttgtgtgg aattgtgagc ggataacaat ttcacacagg aaacagctat 2220

gaccatgatt acgaattgag ctcggtaccc ggcctcgagg tttaaaccga ttcctgctgc 2280

cgcacctcgc gagccagctg gaggctgacg gggagacgac gccaaaatgc tggatgcgcg 2340

cttttcgctg aggttgtctc tgtcgtcgcg ctggcgtctg cgccaaaaca ggaacaggag 2400

caggagtccc aagagcagca aagctccgag gatcgacccg acagcgattc cggccttcgc 2460

gcccttgctg agccccgtgg acctgttgtt ggcgctcgag gcacttgagg acgacgcggc 2520

agccgacgcc gaggtggctg tagcgacgga ctgcgtgatg tagatgatcg aggtctgcat 2580

gtttccaggt gaaacggacg tagttatccg cactgattcg gatgagggtg tggacgaaga 2640

ggtggttgaa gacgacgacg agctctggga gaaagaaaag gacgaaatcg agcttgttgt 2700

agtcgagcta gcggacagac ttgaggacgt tggagacgag tctgtggaag acatggtgga 2760

gctctgtggg gaggaacttt gcgtggagga agacgatgtt gaggagctgg acagactgga 2820

actggaggag gaggtggtgg aggtggagga ggtggaagtg gcatcgtctg tggacgagtg 2880

cgacgactgc gccgacgaac tgtcgtcgtc gttttcctca gactcgtcaa catagacgga 2940

ataagagtcg ctaccaccac acttctccat gggataacca gtgcaggacg ttgtgcacga 3000

gccgtcgggg ttgtccgaag gtacgtcgtc tccgcaataa cagtcctggc cgttgatcaa 3060

tgcagcaact gcgtgtcccg aacactgctc cgtgcaatgg ctggagctct gccatgtgta 3120

cgagtccttc ttggtcaagc cggaaatggc atccgagctg tagcaaccta aatacgtaaa 3180

gtctgctagg acttgggtga tcacgaatac gacaccgaat ctcatgttga catggaaaag 3240

ctaaaaaata aacaatagag cgtcgcgtcg aaaaaaataa aatctgtgag gcgtgcgtgt 3300

ttaggatttt tgggattatt taggaattat tcgagatcct actttttttt ctcccttatt 3360

taattaacaa tctccggaat ggtgatctga tcgttcctga aaacctcgac attggctccc 3420

tcctgacaca ggtactcgta caggttccag gtaaacgagt cgtagttgtc gatcatgaca 3480

acgttcttag aagcggccgg cattttgaag gtgactaata gcctaagaaa atatttaatt 3540

taattttcat taaattttcc tatactcgct atttcagctt ttcatctcat cacttcataa 3600

acgatataaa ccagaaaaag aactattttc aaacacgctt ctcaaaagcg gtatgtcctt 3660

ccacgtctcc ttagaatctg gcaagtccgc gagggggatc cttattagcc catctgctgg 3720

ccaccggtca tcgaggccat cgagaaggcc tgcttggtcg aggtttccag gatgtccagg 3780

ccttccacca ggtcctcgtc tgggatggtc agtgggcaca ggcacttcac cacctcgccg 3840

tcctggcccg aggtctcgat gatcaggccg ttctcgaagg cctggtgggt gatcttgtcg 3900

gcgatgtcgc ccgagcccac gtcgatgcct ctcatcaggc ctctgcctct ttccgaagcc 3960

tcgatgccgt tctccgacag ccaagcggcg atcttgccga atctctcttc cacgattctg 4020

gcctttctct gcacgtctct ctcgaaggtg tcgtccgacc agtactttct catggctgcg 4080

gcagcggtgg cgaaggccag gttgaagcct ctgaaggtgc cgttgtactg gcctggcttc 4140

cacttgtcca gctctggtct catcagcacg tgggcgaatg gcaggccgta gcccgacagc 4200

gacttcgagt tggtcacgat gtctggggtg atgccggcgt gctcgaacga gaagaacttg 4260

ccggttctgc cgcagccggc ctggatatcg tcgatgatca gcaggatgtc gttggctctg 4320

cagatcgatt ccagtctctt cagccattcc agaccggcca cgttgatgcc gccctcgccc 4380

tgcacggttt ccacgatcac ggcggctggc acgtccaggc cacccgactt gtcgcccagc 4440

agcttctcga agtagtccag ggtgtcggtc gacgagccca ggtagccgtc gaatggcatg 4500

aacgaggcag cctgggttgg gacgcctccg gtggcctctc tgaactttct gttgccggtg 4560

gtagccagag cgcccatggt cacaccgtgg aagccgttgg tgaacgacac gatgttgtgt 4620

ctgcccttgg ccactctggc cagtctgatg gcagcttcca cggcgttggt gccggttggg 4680

cctggcaggt gcaccttgta gtccaggcct cttggcttca ggatcacctc ttccagggtt 4740

tccaggtagt ctctcttggc ggcggtccaa aagtccaggc cgtgcacgat gccgtccgag 4800

tcgatgtagt ccagcagggc ctgcttcagg tgtgggttgt tgtggccgta gttcagggtg 4860

cctgcgccag ccagaaagtc gatgtactct ctgccttcct cgtcggtcag tctggcgttt 4920

ctggccttgg tgaacaccac tgggaacgat ctcgagtagg ttctcacgtc cgattccatt 4980

ctctccagga tctgggtctg catgaattcc cgatgaaggc agagagcgca aggaggcggt 5040

atttatagtg ccattcccct ctctgagaga cccggatggt agtcgagtgt atcggagaca 5100

gcttgatgta gactccgtgc ctgccggctc ctcttattgg cggacaccag tgagacaccc 5160

cggaacttgc tgtttttctg caaaatccgg ggtgaccagt gggagcctat ttgcacacac 5220

gagcgggaca ccccactctg gtgaagagtg ccaaagtcat tctttttccc gttgcggggc 5280

agccgattgc atgttttagg aaaatattac ctttgctaca ccctgtcaga tttaccctcc 5340

acacatatat attccgtcac ctccagggac tattattcgt cgttgcgccg ccagcggaag 5400

atatccagaa gctgttttcc gagagactcg gttggcgcct ggtatatttg atggatgtcg 5460

cgctgcctca cgtcccggta cccaggaacg cggtgggatc tcgggcccat cgaagactgt 5520

gctccagact gctcgcccag caggtgtttc ttgatcgccg cctctaaatt gtccgcgcat 5580

cgccggtaac atttttccag ctcggagttt gcgtttagat acagtttctg cgatgccaaa 5640

ggagcctgca gattataacc tcggatgctg tcattcagcg cttttaattt gacctccaga 5700

tagttgctgt atttctgttc ccattggctg ctgcgcagct tcgtataact cgagttattg 5760

ttgcgctctg cctcggcgta ctggctcatg atctggatct tgtccgtgtc gcttttcttc 5820

gagtgtttct cgcaaacgat gtgcacggcc tgcagtgtcc aatcggagtc gagctggcgc 5880

cgaaactggc ggatctgagc ctccacactg ccctgtttct ctatccacgg cggaaccgcc 5940

tcctgccgtt tcagaatgtt gttcaagtgg tactctgtgc ggtcaatgaa ggcgttattg 6000

ccggtgaaat ctttgggaag cggttttcct cggggaagat tacgaaattc cccgcgtcgt 6060

tgcgcttcct ggatctcgat cgataacttc gtatagcata cattatacga agttatatgc 6120

gcactctaga ttccttgcgt acaaacacct ttccgtcagc gacacgcctt actttgcgca 6180

cacatccgaa cgagcctcgt ccgatgagct ccaacacttg gaaatcgtcc atcgaatagt 6240

atatttacaa acttccggat acagaaaata attaaattat ttgggacagc ttggtttatt 6300

tcaacactta agcccaggtt ttaccggtca aagcctcgta agcctctttg tacttagcgg 6360

cggtcttagc agcaatctcc tcggtcatag acacaccctc tttaccgttc agctggtgag 6420

aagtcaacca gtctctcagg aactgcttgt cgaaagactc ctgagatctt cccaactcgt 6480

acttcttggc gtcccaaaat ctcgaagagt ctggggtcaa aacctcgtca accagaacca 6540

gctcgttgtt ctcgttcagt ccgaactcaa acttggtgtc ggccaggata atacctctcg 6600

acagagcgta gtctctagcc ttagagtaca gggcaatagc cttctgggca acctcagcac 6660

acaactcctc tccaaccaac tgagcagcct gctctggaga aatgttctcg tcgtgctgtc 6720

cctgctctgc tttggtagat ggggtgaaga ttggcttctc gaaagcctca gactcctgca 6780

atccagactt aactggcagt ccgtgaacgg tagacgactt cttgtactct ttccaggcag 6840

aaccggtgat gtaacctctc acaatggcct ccaatggaac cagcttgtgc ttcttcacca 6900

gcagagatct accagccaac tgagccttgt actttggctc agacaactgc tctggcaact 6960

tggcaaagat gtccttgtca gaggaagcaa ccaggtggtt tggaataacg tcagacagca 7020

ggttgaacca gaacacagac agctgggtca gaatctttcc cttgtccttg atgccgttct 7080

ccataatcac gtcgtaggca gagattctgt cggtggcaac aaacagcagc atgttgtcgt 7140

ccacctggta gatgtctctc actttacctc tggcaaccaa tggcagaata ccgtccaggt 7200

tagtctgggt cacggtcata tgtgtaataa taattaagta aaattagttt agtaaattaa 7260

ttgactcgtg agggtcgatc gacgcgatat tggctctcct ttttatacga gtttttgtca 7320

actttcctct gctatgctca atccagtcac cgtccacaaa gacttctttg ggtttcctgg 7380

ggccttggtt ctgaccatca gtctaccttt ggtggttgtt ttcctgtatg cggcgtcgac 7440

ggcgcgccat aacttcgtat agcatacatt atacgaagtt atcctgcagg gattgcaaaa 7500

tgatgcaact attttgcgcc ggtaccggga aaaattgaaa aaccatccac ttactcattc 7560

ctgtcttttt atttcgtatt accaaaccgc ttacgtactc acccactcag atctgctcag 7620

acacaaactg gtcgatgtcg acctcctcga gtggagcagt ggccgcattg ttcacctttg 7680

gcttctcctc tctgagctca agacgtttca gcttactaag agggccccag ccgtgcacag 7740

gtgggctaag tttgaatgga tccaaaaact tgacacactg tttgaacgag tcgcccaatg 7800

tggcaatctc gtggatgatg tcttccacac agatgatgcc gtagtctcca agctggtctt 7860

caatcaaatt gttgtcatcc aagttcacct tcttggttgt tccgtcctcg acgatactag 7920

ccgtggcacg tttctggatc aagtttcgga cagtagcaag cgatggagtg ccgatggcga 7980

tgtacggact ggtcagtctg aggagaggtt tgacggcagc gttcaacttg atgaaagttc 8040

ctgtgtacac gtgctctaga cgcagcagtt caagcacttt tctggctttg gagggcactt 8100

tggcaccatg cgggccgggg atacggacca caaacaaaag acgctcatcg gactcttccg 8160

gcacagcaga gctccgttca tgctgagaca cgcgcttcac acggtactgt tctctttctg 8220

atgcgcgatg tttagccact agggtttctg ctctgataaa ccttgccttt ctttgctgtt 8280

gtttgcgttt cttctgttcc tcgagcttct tacgtgcctg ctcctgtttc tctagacgga 8340

gtctgtcggc gttcttccgc ttcttgagca acacctctgg gttggtgttc agtttagtcg 8400

attcagccat gataaaaaaa aaaaaaattg tgcagaaaaa tttatttggc agtgttgcgg 8460

ccgcgcatgc ggtctctagc ttggcactgg ccgtcgtttt acaacgtcgt gactgggaaa 8520

accctggcgt tacccaactt aatcgccttg cagcacatcc ccctttcgcc agctggcgta 8580

atagcgaaga ggcccgcacc gatcgccctt cccaacagtt gcgcagcctg aatggcgaat 8640

ggcgcctgat gcggtatttt ctccttacgc atctgtgcgg tatttcacac cgcatatgct 8700

cgtcaaagca accatagtac gcgccctgta gcggcgcatt aagcgcggcg ggtgtggtgg 8760

ttacgcgcag cgtgaccgct acacttgcca gcgccctagc gcccgctcct ttcgctttct 8820

tcccttcctt tctcgccacg ttcgccggct ttccccgtca agctctaaat cgggggctcc 8880

ctttagggtt ccgatttagt gctttacggc acctcgaccc caaaaaactt gatttgggtg 8940

atggttcacg tagtgggcca tcgccctgat agacggtttt tcgccctttg acgttggagt 9000

ccacgttctt taatagtgga ctcttgttcc aaactggaac aacactcaac cctatctcgg 9060

gctattcttt tgatttataa gggattttgc cgatttcggc ctattggtta aaaaatgagc 9120

tgatttaaca aaaatttaac gcgaatttta acaaaatatt aacgtttaca attttatggt     9180

gcactctcag tacaatctgc tctgatgccg catagttaag ccagccccga cacccgccaa     9240

cacccgctga cgcgccctga cgggcttgtc tgctcccggc atccgcttac agacaagctg     9300

tgaccgtctc cgggagctgc atgtgtcaga ggttttcacc gtcatcaccg aaacgcgcga     9360

<210> 7
<211> 10130
<212> DNA
<213> Hansenula polymorpha (syn. Pichia angusta)

<220>
<221> source
<222> 1..10130
<223> /mol_type="unassigned DNA"
      /organism="Hansenula polymorpha (syn. Pichia angusta)"

<220>
<221> gene
<222> 190..1062
<223> /note="AmpR; Ampicillin resistence gene"

<220>
<221> misc_feature
<222> 2260..3270
<223> /note="ODC1 downstream region for homologous recombination of the
      integration cassette"

<220>
<221> terminator
<222> 3278..3605
<223> /note="MOX-T; teminator of the MOX gene"

<220>
<221> gene
<222> 3615..4055
<223> /note="EctC-HA fusion ORF (EctC coding region/HA tag coding
      region)"

<220>
<221> promoter
<222> 4063..5128
<223> /note="FMD promoter"

<220>
<221> misc_feature

<222> 5127..8160
<223> /note="loxP site"

<220>
<221> misc_feature
<222> 5135..5168
<223> /note="loxP site"

<220>
<221> gene
<222> 5168..7972
<223> /note="HIS4 of H. polymorpha"

<220>
<221> misc_feature
<222> 8161..9227
<223> /note="ODC1 upstream region for homologous recombination of the
      integration cassette"

<400> 7
ggacgaaagg gcctcgtgat acgcctattt ttataggtta atgtcatgat aataatggtt      60

tcttagacgt caggtggcac ttttcgggga aatgtgcgcg gaacccctat ttgtttattt     120

ttctaaatac attcaaatat gtatccgctc atgagacaat aaccctgata aatgcttcaa     180

taatattgaa aaaggaagag tatgagtatt caacatttcc gtgtcgccct tattcccttt     240

tttgcggcat tttgccttcc tgtttttgct cacccagaaa cgctggtgaa agtaaaagat     300

gctgaagatc agttgggtgc acgagtgggt tacatcgaac tggatctcaa cagcggtaag     360

atccttgaga gttttcgccc cgaagaacgt tttccaatga tgagcacttt taaagttctg     420

ctatgtggcg cggtattatc ccgtattgac gccgggcaag agcaactcgg tcgccgcata     480

cactattctc agaatgactt ggttgagtac tcaccagtca cagaaaagca tcttacggat     540

ggcatgacag taagagaatt atgcagtgct gccataacca tgagtgataa cactgcggcc     600

aacttacttc tgacaacgat cggaggaccg aaggagctaa ccgctttttt gcacaacatg     660

ggggatcatg taactcgcct tgatcgttgg gaaccggagc tgaatgaagc cataccaaac     720

gacgagcgtg acaccacgat gcctgtagca atggcaacaa cgttgcgcaa actattaact     780

ggcgaactac ttactctagc ttcccggcaa caattaatag actggatgga ggcggataaa     840

gttgcaggac cacttctgcg ctcggccctt ccggctggct ggtttattgc tgataaatct     900

ggagccggtg agcgtgggtc tcgcggtatc attgcagcac tggggccaga tggtaagccc 960

tcccgtatcg tagttatcta cacgacgggg agtcaggcaa ctatggatga acgaaataga 1020

cagatcgctg agataggtgc ctcactgatt aagcattggt aactgtcaga ccaagtttac 1080

tcatatatac tttagattga tttaaaactt catttttaat ttaaaaggat ctaggtgaag 1140

atcctttttg ataatctcat gaccaaaatc ccttaacgtg agttttcgtt ccactgagcg 1200

tcagaccccg tagaaaagat caaaggatct tcttgagatc ctttttttct gcgcgtaatc 1260

tgctgcttgc aaacaaaaaa accaccgcta ccagcggtgg tttgtttgcc ggatcaagag 1320

ctaccaactc tttttccgaa ggtaactggc ttcagcagag cgcagatacc aaatactgtc 1380

cttctagtgt agccgtagtt aggccaccac ttcaagaact ctgtagcacc gcctacatac 1440

ctcgctctgc taatcctgtt accagtggct gctgccagtg gcgataagtc gtgtcttacc 1500

gggttggact caagacgata gttaccggat aaggcgcagc ggtcgggctg aacggggggt 1560

tcgtgcacac agcccagctt ggagcgaacg acctacaccg aactgagata cctacagcgt 1620

gagcattgag aaagcgccac gcttcccgaa gggagaaagg cggacaggta tccggtaagc 1680

ggcagggtcg gaacaggaga gcgcacgagg gagcttccag ggggaaacgc ctggtatctt 1740

tatagtcctg tcgggtttcg ccacctctga cttgagcgtc gatttttgtg atgctcgtca 1800

ggggggcgga gcctatggaa aaacgccagc aacgcggcct ttttacggtt cctggccttt 1860

tgctggcctt ttgctcacat gttctttcct gcgttatccc ctgattctgt ggataaccgt 1920

attaccgcct ttgagtgagc tgataccgct cgccgcagcc gaacgaccga gcgcagcgag 1980

tcagtgagcg aggaagcgga agagcgccca atacgcaaac cgcctctccc cgcgcgttgg 2040

ccgattcatt aatgcagctg gcacgacagg tttcccgact ggaaagcggg cagtgagcgc 2100

aacgcaatta atgtgagtta gctcactcat taggcacccc aggctttaca ctttatgctt 2160

ccggctcgta tgttgtgtgg aattgtgagc ggataacaat ttcacacagg aaacagctat 2220

gaccatgatt acgaattgag ctcggtaccc ggcctcgagg tttaaacagg ccgtctcgat 2280

ttgactacct cacaacagca agagctttgt tgcatggaga gcttgcctcc aaaccgtgga 2340

gcttacgaac acattcagga atagctcttc tataccaatc aatcaaaaaa gaagggtgga 2400

attttattcc acgacaagta cttcctccaa tagtggcaaa cagcgcaatt ggagtgattc 2460

tttattctac ctatctcaca ctattacaac gttttaataa gagaaatatg aactcaattg 2520

atgatcctag tcctgtcgac actttcaaag ctggttttgt tgcgggagct gctgcgagct 2580

tggctgcatc accgctagat gcactttacg cccgatcgaa ttactcagag cttgtcagtg 2640

gaaaacagaa gagcatgtgg gagtatgcct tatacaaact aaaacaaatt ggtttagttg 2700

gaatatttgc cggcttcagt ttaaatttca taaaggaaag ccttggattt gcattctact 2760

tttcagtatt tgaactagtg aaaaaccagg ggtaccacaa gacaaaagca gctatacagt 2820

ggtatcaata ttggcgctcg agactttttc atgatgaaga aaaccaggag aagaattcaa 2880

gatttttcaa agctttgaat ttgacgttta tccttcttgc tggagcttcc gccgcaacta 2940

cactgcttgc aatccaatat ccactcaata aaattcaaaa ggtccacctg actagactcg 3000

aatctctaga tctctacaac gaagcacatc aactggatcg gcgtcgcttc ttcaaactct 3060

actataattc ttacattgag acatttgata ttattctaca acgatggcga aaatctcacc 3120

tctccctctt tggcttctgc tatagaggct ttttcagatc gtccttcact agtattcccg 3180

cgacttctat tgggcttttc gtattcgaaa tatcccgtca gcattttagc gaaaatcaat 3240

tttgacgatg tatatagaac tgaaagccgt taattaacaa tctccggaat ggtgatctga 3300

tcgttcctga aaacctcgac attggctccc tcctgacaca ggtactcgta caggttccag 3360

gtaaacgagt cgtagttgtc gatcatgaca acgttcttag aagcggccgg cattttgaag 3420

gtgactaata gcctaagaaa atatttaatt taattttcat taaattttcc tatactcgct 3480

atttcagctt ttcatctcat cacttcataa acgatataaa ccagaaaaag aactattttc 3540

aaacacgctt ctcaaaagcg gtatgtcctt ccacgtctcc ttagaatctg gcaagtccgc 3600

gagggggatc cctattaggc gtagtctggc acgtcgtatg ggtacaatgg tttctggtcg 3660

tcagcctcgt cagctggagc gtaagaaccg tcctctctgt gaacctcgtt accagtcaaa 3720

cctggggtaa aaacacaggc caggtgcata gtcttagaag ctctcaacag gtgctcgtcg 3780

tgctggtcca aaatgtagat gtcgcctggc ttaataggcc agatcttacc gtcagccaaa 3840

gtctcaacct cgccctcacc ctcaatacag tacacggcct cgaagtggtg tttgtagtgg 3900

atgtgagtct cggttccctc gaagattctg gtgatgtgga aagagcagtt accaccgtcc 3960

tcagccaaag acaatctggt agagtcccag ttaccgttct cagcagtaac caatctgtcg 4020

gtctgtctag cttcttccaa gtttctcacg atcatgaatt cccgatgaag gcagagagcg 4080

caaggaggcg gtatttatag tgccattccc ctctctgaga gacccggatg gtagtcgagt 4140

gtatcggaga cagcttgatg tagactccgt gcctgccggc tcctcttatt ggcggacacc 4200

agtgagacac cccggaactt gctgtttttc tgcaaaatcc ggggtgacca gtgggagcct 4260

atttgcacac acgagcggga caccccactc tggtgaagag tgccaaagtc attctttttc 4320

ccgttgcggg gcagccgatt gcatgtttta ggaaaatatt acctttgcta caccctgtca 4380

gatttaccct ccacacatat atattccgtc acctccaggg actattattc gtcgttgcgc 4440

cgccagcgga agatatccag aagctgtttt ccgagagact cggttggcgc ctggtatatt 4500

tgatggatgt cgcgctgcct cacgtcccgg tacccaggaa cgcggtggga tctcgggccc 4560

atcgaagact gtgctccaga ctgctcgccc agcaggtgtt tcttgatcgc cgcctctaaa 4620

ttgtccgcgc atcgccggta acatttttcc agctcggagt ttgcgtttag atacagtttc 4680

tgcgatgcca aaggagcctg cagattataa cctcggatgc tgtcattcag cgcttttaat 4740

ttgacctcca gatagttgct gtatttctgt tcccattggc tgctgcgcag cttcgtataa 4800

ctcgagttat tgttgcgctc tgcctcggcg tactggctca tgatctggat cttgtccgtg 4860

tcgcttttct tcgagtgttt ctcgcaaacg atgtgcacgg cctgcagtgt ccaatcggag 4920

tcgagctggc gccgaaactg gcggatctga gcctccacac tgccctgttt ctctatccac 4980

ggcggaaccg cctcctgccg tttcagaatg ttgttcaagt ggtactctgt gcggtcaatg 5040

aaggcgttat tgccggtgaa atctttggga agcggttttc ctcggggaag attacgaaat 5100

tccccgcgtc gttgcgcttc ctggatctcg atcgataact tcgtatagca tacattatac 5160

gaagttatat gcgcactcta gagcaattcc gacggttggg atcgtgtatg gtgcagagag 5220

accgaccacc acggggccga tcgactgcgt caggaagtgg atcgggaaca gcttggattg 5280

caggaggcca aactccttgc ggggcaggac cttgaacgct ctgacagagg acacaaatga 5340

gtggaacgaa gtggctccga aaacatacga gtaagcaagc aggtgcacag cagtcagcat 5400

ggtaaaaatt aattgcactt attatttatt tggtcgtggc tttatttaat cagtgataat 5460

tgtcacgcta tatgtagcca agtttttcca ttctaatctt gacggcgttg cggtgcgcgt 5520

cgagaccctc gacggcagcc aggtccatca cggccttgcc aatgctcttg agaccctcct 5580

tggtgaccac ctgcgatgta atgaatttct ggaacgtggt ggtgttaaca cccgagtaca 5640

ttctggcgta gccgtacgta ggcagcgtgt ggttggttcc cgaggagtag tcaccgcagc 5700

tttccggcga ataggcaccc acaaacacag agccagcatt gtcgaccttg tcgacccatt 5760

tctcggcgtc ctggatctgc aggataaggt gctccggagc gtatctgttg ctcatctcga 5820

acgcctcatc gtagctcttc acaatgatcg tggtggagtg agcaatgcac tggcgcacga 5880

tctccacacg tggcagctgc agcgcctggc gatggacggc ctcgtcgatc ttgtcgatct 5940

cggcagacgt catgtccacg gcgatcaata tcacttgcga gtcaacgccg tgttctgcct 6000

gcgacagcag gtccgaggcc acaaagtctg ggtcggcgta cttgtccgcg acaaccagaa 6060

cctcggatgg cccggcaggc atatcaatgg cacacatggc gttggaatca ttggaaacga 6120

gcatcttggc tgccgtcaca aactggtttc cggggcccat gatcttgtca catttaggca 6180

cagactcggt accgtacgcc attgcggcca ccgcctgggc ccctccggca agcacaatgc 6240

acttggcacc caccttgtgc gccacataga ccacctctgg cgtcagacgg ccgtccttgc 6300

cgggaggaga cgcaaacaca atatctttgc aaccggcaac gagagcaggc acagagagca 6360

taagggacgt ggacggcagc acggcagttc cgccggggat gtaacagcca acacgctcga 6420

tcggacgtgc aaatcgagag cacacaacgc cgggacacgt ttccacgcgt agagtctctt 6480

tctggttctg cgcttcgtgg aaggtcttga tattggcaaa cgagatgtca atggccttgc 6540

gcacgtcttc gctgatgttc atcatttctg ctgggaatgg agcgttcaat acaggagagc 6600

taagctgcac cttgtcgaac ttggctgtga gctccaccaa cgcagcgtct ccttttgttc 6660

tcactttctc gacgatcggc gtaaccaggt tcatgatatc cgcagacttt tggattggtc 6720

tttgaagaca tttttcgata tcttctttgg aggcagtggt ggcatccaca cggttcatca 6780

cgatgcggcc atctccgttc acaagccgct ttggcaattt tggcaccgtt ggcaccgttg 6840

gttcctctag ctttttttcc gtagcgtcct gctcaacata cttcgccttt gcgtcgccct 6900

tacgtctcgt caccttcagg gccttgatgt ccaaattccg ctcaacgtct gcaagcgaaa 6960

cgccgtactt cacacatctg gcaagcgcaa agtagaccaa atcggcagct tcccacgcaa 7020

tctcgtcctt cgaggtggct tccaccagct cgtcggcttc ttccctaatc ttggcaccga 7080

gcagcttctc atcctcaaac agtctcttcg tgtaggagcc ttcaggagca ttcgccttgc 7140

ggtcccaaag agtggcttcc agagctgcca gacccttgga cgagccaaaa caggaggcag 7200

tgtcgaggtg gcagaaacca gcaccggcct gttccacaac aaatttcaaa caatcgctat 7260

cgcagtctag ctccagttgg agcaattttt gcgtggctcc cgatgtctgg cctttgtacc 7320

acaagccgtg ctttctggac tggtagacac ccgtgccggt ccttattgcc tcagcaatgg 7380

acttcttgga cgagtacacc aaaccaagag cctggttcct gctgtcagtg acaagagttg 7440

tgtataagcc gtctggtctg tcggtgttca tggcagcgag atagcactcc acaaacggcg 7500

ttttggtgcc cgcgttttcg tactccttag tcagctgatc gagctcaata acagcagtga 7560

agcccttggc gatctgccgg agtggttgtt caaaataacc aaagactctt ctttttccaa 7620

ttttcacttc agtctcccca aaaacacaga tattctcggc agcatctttt ccatccttga 7680

tgaccagtct ggcgttggga atacggcttc taatctgctc agcaacagca gaggtgacaa 7740

aaagaccgta cacaccgttg ttcaaaagag ccagaacaat gtctatttgg ctatcatttt 7800

ccacagaccc cagagcccag tcgacatata cctggcctag atactcttta gccaacgcga 7860

taatttcctg cacagcggca gccgcatcaa gcgacacaag aacctcgctc acaaacggca 7920

cgctcaaaac gtcccttggt tgagcaacgc tcacaaggat aggagaaacc atttatgaat 7980

ttacggtgag atgtgagtca agctgaaaaa tatcttgatt tttttttttt tgacatgcca 8040

cgactttggt agcatgggac gatatatcac tatatacact gtgagtcagt tattgaacaa 8100

aaactgcata tcgtcgacgg cgcgccataa cttcgtatag catacattat acgaagttat 8160

cctgcagggt tgattattat tcagggaaat gttgaaaatt tttcgataat tcattagttt 8220

atttttgata caccatggat caactcaact ataaagaaca gcaagaattc cagaaaattg 8280

tggagcagaa gcaaatgaat gacttcatga gagtaagtga acgccttctg aaaactaaca 8340

ttgcagctct acaccagttt ggtggacaga tgttttaatg attgcgtcaa cgacttcacc 8400

tccaacaaac ttagctctag agaagaaaca tgtttgaaca agtgcgctga aaagttcctc 8460

aagcactccg aaagagttgg tttaagattc caggaacaga accagatgtt gatgaaccag 8520

atgagaggcg gcaattaatg agctttaatt gctggatgat tcccggaata ggctcaacaa 8580

gttttgcgtt tcttccatca tttcaatcgt ccatttgtaa gtttctgtct gattcatatc 8640

gttgtacata attgcgttgc tgaacatcag ttgaagctca aattccaact cgtcgaatga 8700

ctttatttca cccgtcctaa tagctttacc caaactctta atgtccacgg gatgccgtat 8760

tagcttgtag tagttgggct cttcgttttc attcactggt tgcatgaaca tagacgcaaa 8820

ccggttggat gatatctgtg tgagaagttg tgcagacaga gcattgaagc gcttagtaca 8880

accagggggg acgctttcgg atccgcgtcg cttgctccca cgttcttcat ctccacttgc 8940

tggcgtagat ggtgtagcag gttctggagt tggcgatggt atttctttag aagccgactt 9000

ggattccaat ttcgtttggg gttgggattc tacattcgac tctgaggttg aagctgggcc 9060

ttcttgaggt tcggcatgag tttccaataa ctctgttttg ctttttccca aagcagtgac 9120

cggctgagat tgagccgctg attctgcctc gggctccgca acttcatcga tctctttgtc 9180

tgcgtcatta tgcgtcacgc cctcttcagg ttcttgtgcg tcacctgcgg ccgcgcatgc 9240

ggtctctagc ttggcactgg ccgtcgtttt acaacgtcgt gactgggaaa accctggcgt 9300

tacccaactt aatcgccttg cagcacatcc ccctttcgcc agctggcgta atagcgaaga 9360

ggcccgcacc gatcgccctt cccaacagtt gcgcagcctg aatggcgaat ggcgcctgat 9420

gcggtatttt ctccttacgc atctgtgcgg tatttcacac cgcatatgct cgtcaaagca 9480

accatagtac gcgccctgta gcggcgcatt aagcgcggcg ggtgtggtgg ttacgcgcag 9540

cgtgaccgct acacttgcca gcgccctagc gcccgctcct ttcgctttct tcccttcctt 9600

tctcgccacg ttcgccggct ttccccgtca agctctaaat cgggggctcc ctttagggtt 9660

ccgatttagt gctttacggc acctcgaccc caaaaaactt gatttgggtg atggttcacg 9720

tagtgggcca tcgccctgat agacggtttt tcgccctttg acgttggagt ccacgttctt 9780

taatagtgga ctcttgttcc aaactggaac aacactcaac cctatctcgg gctattcttt 9840

tgatttataa gggattttgc cgatttcggc ctattggtta aaaaatgagc tgatttaaca 9900

aaaatttaac gcgaatttta acaaaatatt aacgtttaca attttatggt gcactctcag     9960

tacaatctgc tctgatgccg catagttaag ccagccccga cacccgccaa cacccgctga    10020

cgcgccctga cgggcttgtc tgctcccggc atccgcttac agacaagctg tgaccgtctc    10080

cgggagctgc atgtgtcaga ggttttcacc gtcatcaccg aaacgcgcga               10130

<210> 8
<211> 9374
<212> DNA
<213> Hansenula polymorpha (syn. Pichia angusta)

<220>
<221> source
<222> 1..9374
<223> /mol_type="unassigned DNA"
      /organism="Hansenula polymorpha (syn. Pichia angusta)"

<220>
<221> gene
<222> 6..1028
<223> /note="H6-EctD fusion ORF (start methionine/His tag and flanking
      residues coding region/EctD coding region)"

<220>
<221> terminator
<222> 1038..1365
<223> /note="MOX-T; terminator of MOX gene"

<220>
<221> misc_feature
<222> 1371..2436
<223> /note="ADE1 downstream region for homologous integration of the
      integration cassette"

<220>
<221> gene
<222> 1371..2436
<223> /note="AmpR; Ampicillin resistence gene"

<220>
<221> misc_feature
<222> 5602..6658
<223> /note="ADE1 upstream region for homologous integration of the
      integration cassette"

<220>
<221> misc_feature

<222> 6665..6698
<223> /note="loxP site"

<220>
<221> gene
<222> 6962..8050
<223> /note="Synthetic H. polymorpha LEU2 gene; codons optimized for
      Hansenula polymorpha"

<220>
<221> misc_feature
<222> 8267..8300
<223> /note="loxP site"

<220>
<221> promoter
<222> 8307..9372
<223> /note="FMD promoter"

<400> 8
aattcatgaa acaccatcat caccaccacc agtctgttca gacctcgtct aacagaccat      60

tgccacaggc taacctgcac attgctaccg agactccaga ggctgactcg agaattagat      120

cggctccaag accaggacag gacccatacc caaccagatt gtctgagcca ttggacttgc      180

cttggctgaa cagaagagag ccagttgtta aaggcgagga ggctgacggt ccattgtctg      240

ctgctcagtt ggacaccttt gagagacagg gcttcatttt cgagccagac tttttgaagg      300

gcgaggagtt ggaggctttg agacacgagc tgaacgcttt gttggctaga gatgacttca      360

gaggtagaga cttcgctatt accgagccac agggcaacga gattagatcg ctgttcgccg      420

ttcactacct gtcgagagtg ttttcgagac tggccaacga cgagagattg atgggtagag      480

ccagacagat tttgggtgga gagccatacg ttcaccagtc gagaatcaac tacaagccag      540

gctttgaggg aaagggtttc aactggcact ctgactttga gacttggcac gctgaggacg      600

gaatgccagc tatgcacgcc gtttctgcct ctattgtgct gaccgacaac cacactttta      660

acggcccact gatgttggtt ccaggctctc acagagtgtt cgttccatgt ctgggagaga      720

ctccagagga ccaccacaga cagtctctga aaacccagga gttcggagtt ccatcgagac      780

aggctctgag agagctgatt gacagacacg gtattgaggc tccaactggt gctgccggtg      840

gtttgttgct gttcgactgc aacactctgc acggttctaa cgccaacatg tcgccagacc      900

caagatcgaa cgccttcttc gtgtacaata gaagagacaa cagatgcgtt gagccatacg 960

ccgcttctaa gagaagacca agattcttgg ctcacgagcc agacgaggct tggtcgccag 1020

acggttaata aggatccccc tcgcggactt gccagattct aaggagacgt ggaaggacat 1080

accgcttttg agaagcgtgt ttgaaaatag ttcttttctt ggtttatatc gtttatgaag 1140

tgatgagatg aaaagctgaa atagcgagta taggaaaatt taatgaaaat taaattaaat 1200

attttcttag gctattagtc accttcaaaa tgccggccgc ttctaagaac gttgtcatga 1260

tcgacaacta cgactcgttt acctggaacc tgtacgagta cctgtgtcag gagggagcca 1320

atgtcgaggt tttcaggaac gatcagatca ccattccgga gattgttaat taagtgttga 1380

aataaaccaa gctgtcccaa ataatttaat tattttctgt atccggaagt ttgtaaatat 1440

actattcgat ggacgatttc caagtgttgg agctcatcgg acgaggctcg ttcggatgtg 1500

tgcgcaaagt aaggcgtgtc gctgacggaa aggtgtttgt acgcaaggaa atatcatacg 1560

gggccatgaa tagcaaggag aagcaccagc ttagcaccga gttcaggatc ctacgagaac 1620

tgagacatcc caacattgtg cagtatctcc accatgacca tgtccaggat gagcaccagg 1680

ttcatttgta tatggagtac tgcgacggag gcgatctatc ggtgctgata aagaagtaca 1740

aggagagaaa cgagtttata ccggaaacac tgatctggca gatatttacc caggtgttga 1800

atgcgcttta caaatgtcac tatggaacgt cgatagagcc ggtcagcaat cttttccagt 1860

cgtctcccga actagaaccg aaacctgtgg accatcaggt gattattcat cgcgacatca 1920

aaccagacaa cattttcctt ttgtcggacg ggtattcgat caaactgggg gatttcggtc 1980

ttgcaaagtg gctccaacac gaaaacgagt ttgccaagac ctatgtgggc acgccatact 2040

acatgtctcc cgaggtgctg atggatagac cgtacgaccc cgtctgcgac gtgtggtcgc 2100

tcggatgcgt catgtatgaa ttgtgtgctc tacgtcctcc tttccaggcc aagactcact 2160

tggcattgca ggaaaaaatc aaggctggca cgttccagcc gatccctgac cattactcgc 2220

atagactaca gatggtcatc aacgcatgtc ttattgtcga ccccatggaa cgcgctaccg 2280

tcaaccaact gctacaggac gcctctttca aattgttcag aaaagagtgg gaaatgacgc 2340

aatgggagaa ccggctgcgc gagcacgaaa cagagctgct tcttaaggaa cgcaagctgg 2400

tcaagtttga ggacgctctc aacgaagaat gcgtttaaac ctcgaggccg ggtaccgagc 2460

tcaattcgta atcatggtca tagctgtttc ctgtgtgaaa ttgttatccg ctcacaattc 2520

cacacaacat acgagccgga agcataaagt gtaaagcctg gggtgcctaa tgagtgagct 2580

aactcacatt aattgcgttg cgctcactgc ccgctttcca gtcgggaaac ctgtcgtgcc 2640

agctgcatta atgaatcggc caacgcgcgg ggagaggcgg tttgcgtatt gggcgctctt 2700

ccgcttcctc gctcactgac tcgctgcgct cggtcgttcg gctgcggcga gcggtatcag 2760

ctcactcaaa ggcggtaata cggttatcca cagaatcagg ggataacgca ggaaagaaca 2820

tgtgagcaaa aggccagcaa aaggccagga accgtaaaaa ggccgcgttg ctggcgtttt 2880

tccataggct ccgcccccct gacgagcatc acaaaaatcg acgctcaagt cagaggtggc 2940

gaaacccgac aggactataa agataccagg cgtttccccc tggaagctcc ctcgtgcgct 3000

ctcctgttcc gaccctgccg cttaccggat acctgtccgc ctttctccct tcgggaagcg 3060

tggcgctttc tcaatgctca cgctgtaggt atctcagttc ggtgtaggtc gttcgctcca 3120

agctgggctg tgtgcacgaa ccccccgttc agcccgaccg ctgcgcctta tccggtaact 3180

atcgtcttga gtccaacccg gtaagacacg acttatcgcc actggcagca gccactggta 3240

acaggattag cagagcgagg tatgtaggcg gtgctacaga gttcttgaag tggtggccta 3300

actacggcta cactagaagg acagtatttg gtatctgcgc tctgctgaag ccagttacct 3360

tcggaaaaag agttggtagc tcttgatccg gcaaacaaac caccgctggt agcggtggtt 3420

tttttgtttg caagcagcag attacgcgca gaaaaaaagg atctcaagaa gatcctttga 3480

tcttttctac ggggtctgac gctcagtgga acgaaaactc acgttaaggg attttggtca 3540

tgagattatc aaaaaggatc ttcacctaga tccttttaaa ttaaaaatga agttttaaat 3600

caatctaaag tatatatgag taaacttggt ctgacagtta ccaatgctta atcagtgagg 3660

cacctatctc agcgatctgt ctatttcgtt catccatagt tgcctgactc cccgtcgtgt 3720

agataactac gatacgggag ggcttaccat ctggccccag tgctgcaatg ataccgcgag 3780

acccacgctc accggctcca gatttatcag caataaacca gccagccgga agggccgagc 3840

gcagaagtgg tcctgcaact ttatccgcct ccatccagtc tattaattgt tgccgggaag 3900

ctagagtaag tagttcgcca gttaatagtt tgcgcaacgt tgttgccatt gctacaggca 3960

tcgtggtgtc acgctcgtcg tttggtatgg cttcattcag ctccggttcc caacgatcaa 4020

ggcgagttac atgatccccc atgttgtgca aaaaagcggt tagctccttc ggtcctccga 4080

tcgttgtcag aagtaagttg gccgcagtgt tatcactcat ggttatggca gcactgcata 4140

attctcttac tgtcatgcca tccgtaagat gcttttctgt gactggtgag tactcaacca 4200

agtcattctg agaatagtgt atgcggcgac cgagttgctc ttgcccggcg tcaatacggg 4260

ataataccgc gccacatagc agaactttaa aagtgctcat cattggaaaa cgttcttcgg 4320

ggcgaaaact ctcaaggatc ttaccgctgt tgagatccag ttcgatgtaa cccactcgtg 4380

cacccaactg atcttcagca tcttttactt tcaccagcgt ttctgggtga gcaaaaacag 4440

gaaggcaaaa tgccgcaaaa aagggaataa gggcgacacg gaaatgttga atactcatac 4500

tcttcctttt tcaatattat tgaagcattt atcagggtta ttgtctcatg agcggataca 4560

tatttgaatg tatttagaaa aataaacaaa taggggttcc gcgcacattt ccccgaaaag 4620

tgccacctga cgtctaagaa accattatta tcatgacatt aacctataaa aataggcgta 4680

tcacgaggcc ctttcgtcct cgcgcgtttc ggtgatgacg gtgaaaacct ctgacacatg 4740

cagctcccgg agacggtcac agcttgtctg taagcggatg ccgggagcag acaagcccgt 4800

cagggcgcgt cagcgggtgt tggcgggtgt cggggctggc ttaactatgc ggcatcagag 4860

cagattgtac tgagagtgca ccataaaatt gtaaacgtta atattttgtt aaaattcgcg 4920

ttaaattttt gttaaatcag ctcatttttt aaccaatagg ccgaaatcgg caaaatccct 4980

tataaatcaa aagaatagcc cgagataggg ttgagtgttg ttccagtttg gaacaagagt 5040

ccactattaa agaacgtgga ctccaacgtc aaagggcgaa aaaccgtcta tcagggcgat 5100

ggcccactac gtgaaccatc acccaaatca agttttttgg ggtcgaggtg ccgtaaagca 5160

ctaaatcgga accctaaagg gagcccccga tttagagctt gacggggaaa gccggcgaac 5220

gtggcgagaa aggaagggaa gaaagcgaaa ggagcgggcg ctagggcgct ggcaagtgta 5280

gcggtcacgc tgcgcgtaac caccacaccc gccgcgctta atgcgccgct acagggcgcg 5340

tactatggtt gctttgacga gcatatgcgg tgtgaaatac cgcacagatg cgtaaggaga 5400

aaataccgca tcaggcgcca ttcgccattc aggctgcgca actgttggga agggcgatcg 5460

gtgcgggcct cttcgctatt acgccagctg gcgaaagggg gatgtgctgc aaggcgatta 5520

agttgggtaa cgccagggtt ttcccagtca cgacgttgta aaacgacggc cagtgccaag 5580

ctagagaccg catgcgcggc cgccacacca ctgaaggctc ccgaagacag atgcacaggt 5640

ctgagcgaga gatatcttgc ttgcagggag taagtgaatg gaacccaaca cagatcgcca 5700

aatgcgagca tgaaaccgaa tccgtcggtg gtaatgtcca tcatggaaag cacgccttcc 5760

tcgttgagca ctccgtcgaa aatgtagaag gattgcatcg caacgacgaa caagagcgag 5820

ttagccaccg ttccagtttc cagatactgt ttttggatgc atgagaggtc tatcagcagc 5880

caaagcagca ttcctggccg tagctcgcaa aagagcttga tgtcccaggg tcctattctg 5940

gggttgagct ctctgccgat aaaccagtca aaaaggacgt ttttggagtt tccaccctcc 6000

gcaagaatgc gctcgtacgt tcctgcacca ttgggccgtc tcagcgggaa aaaggagcag 6060

acgtagacaa acgtgctgag aataagagaa aattcccacg aaacgaccat caggggcaaa 6120

aaattgtcat aaataaactg aagttcgggc acattgcccc ttgtgtacaa cgctcttgct 6180

gccaaagctg cacagaccaa aacactcagc tccttaccgt tgatcagata cgttaaatgt 6240

gttccgtcac gcagcgttgt gcctttttta tagtagccag gaacaataag atccagcaat 6300

gcaagaccaa aaaaccagac caggtatgct atccagcatt tggcactcag aaacgtttcc 6360

ttggtgagtt gttctgcaag caagtttttg atcgtgttga tctgcaaatt gcacccctcg 6420

acaacatacc gatcattgca cgccgcatac aggaaaacaa ccaccaaagg tagactgatg 6480

gtcagaacca aggccccagg aaacccaaag aagtctttgt ggacggtgac tggattgagc 6540

atagcagagg aaagttgaca aaaactcgta taaaaaggag agccaatatc gcgtcgatcg 6600

accctcacga gtcaattaat ttactaaact aattttactt aattattatt acacatcctg 6660

caggataact tcgtataatg tatgctatac gaagttatgg cgcgccgtcg actgtgcacg 6720

gctggggccc tcttagtaag ctgaaacgtc ttgagctcag agaggagaag ccaaaggtga 6780

acaatgcggc cactgctcca ctcgaggagg tagacatcga ccagtttgtg tctgagcaga 6840

tctgagtggg tgagtacgta agcggtttgg taatacgaaa taaaaagaca ggaatgagta 6900

agtggatggt ttttcaattt ttcccggtac cggcgcaaaa tagttgcatc attttgcaat 6960

catgtcgaag aacatcgttc tgttgccagg tgaccacgtt ggaccagagg ttgtggctga 7020

ggctgttaag gttttggagg ccgtttcttc tgccatcggc gtgaagttca acttctcgaa 7080

gcacctgatt ggtggtgctt ctattgacgc ctacggcgtt ccattgtctg acgaggcttt 7140

ggaggctgct aaaaaggctg acgctgttct gttgggtgct gttggtggtc caaaatgggg 7200

aaccggctct gttagaccag agcagggact gctgaagatc agaaaagagc tgaacctgta 7260

cgctaacctt agaccatgtt cttttgcctc tgacgctctg ttgaagctgt ctccactgaa 7320

gtctgagatc gtgaagggaa ccgacttcgt ggttgtgaga gagctggttg gcggtatcta 7380

cttcggcgac agaaaagagg acgctggcga cggcgttgct tctgacactg agagctactc 7440

tgttccagag gtgcagagaa tcaccagaat ggccgctttt ctggctttgc agtctgaccc 7500

accattgcca ttgtggtctt tggacaaggc taacgtgttg gcttcttcga gactgtggag 7560

aaagaccgtg gaggaaacta tcaagaacga gttcccacag ttgactgttc agcaccagct 7620

gattgactct gccgccatga tcttggttaa gtcgccaacc aagctgaacg gtgttatcgt 7680

gaccaacaac atgttcggcg acatcatttc ggacgaggcc tctgttattc caggttctct 7740

gggattgctg ccatctgcat ctttggcttc cctgccagac accaacaagg ctttcggtct 7800

gtacgagcca tgtcacggtt ctgctccaga cttgggtcca ggaaaggtta acccactggc 7860

caccattttg tctgctgcca tgatgctgaa gctgtctttg gacttggttg acgccggtag 7920

agctattgag caggccgtga agaacgtttt ggacgccggt attatgactg ctgacctggg 7980

cggttcttct tctactcagg aagttggcga cgctgttgca caggaagttg ccaagctgct 8040

gaagaactaa ataaggagaa aaaaaaagta ggatctcgaa taattcctaa ataatcccaa 8100

aaatcctaaa cacgcacgcc tcacagattt tatttttttc gacgcgacgc tctattgttt 8160

attttttagc ttttccatgt caacatgaga ttcggtgtcg tattcgtgat cacccaagtc 8220

ctagcagact ttacgtattt aggttgctac actctagagt gcgcatataa cttcgtataa 8280

tgtatgctat acgaagttat cgatcgagat ccaggaagcg caacgacgcg gggaatttcg 8340

taatcttccc cgaggaaaac cgcttcccaa agatttcacc ggcaataacg ccttcattga 8400

ccgcacagag taccacttga acaacattct gaaacggcag gaggcggttc cgccgtggat 8460

agagaaacag ggcagtgtgg aggctcagat ccgccagttt cggcgccagc tcgactccga 8520

ttggacactg caggccgtgc acatcgtttg cgagaaacac tcgaagaaaa gcgacacgga 8580

caagatccag atcatgagcc agtacgccga ggcagagcgc aacaataact cgagttatac 8640

gaagctgcgc agcagccaat gggaacagaa atacagcaac tatctggagg tcaaattaaa 8700

agcgctgaat gacagcatcc gaggttataa tctgcaggct cctttggcat cgcagaaact 8760

gtatctaaac gcaaactccg agctggaaaa atgttaccgg cgatgcgcgg acaatttaga 8820

ggcggcgatc aagaaacacc tgctgggcga gcagtctgga gcacagtctt cgatgggccc 8880

gagatcccac cgcgttcctg ggtaccggga cgtgaggcag cgcgacatcc atcaaatata 8940

ccaggcgcca accgagtctc tcggaaaaca gcttctggat atcttccgct ggcggcgcaa 9000

cgacgaataa tagtccctgg aggtgacgga atatatatgt gtggagggta aatctgacag 9060

ggtgtagcaa aggtaatatt ttcctaaaac atgcaatcgg ctgccccgca acgggaaaaa 9120

gaatgacttt ggcactcttc accagagtgg ggtgtcccgc tcgtgtgtgc aaataggctc 9180

ccactggtca ccccggattt tgcagaaaaa cagcaagttc cggggtgtct cactggtgtc 9240

cgccaataag aggagccggc aggcacggag tctacatcaa gctgtctccg atacactcga 9300

ctaccatccg ggtctctcag agaggggaat ggcactataa ataccgcctc cttgcgctct 9360

ctgccttcat cggg 9374

<210> 9
<211> 5559
<212> DNA
<213> Hansenula polymorpha (syn. Pichia angusta)

<220>
<221> source
<222> 1..5559
<223> /mol_type="unassigned DNA"
      /organism="Hansenula polymorpha (syn. Pichia angusta)"

<220>
<221> misc_feature
<222> 1..1094
<223> /note="HIS4 upstream region for homologous recombination of the

integration cassette"

<220>
<221> terminator
<222> 1102..1429
<223> /note="MOX-T; terminator of MOX gene"

<220>
<221> gene
<222> 1439..2047
<223> /note="EctA-myc fusion ORF (EctA coding region/myc tag coding
      region)"

<220>
<221> promoter
<222> 2055..3120
<223> /note="FMD promoter"

<220>
<221> misc_feature
<222> 3127..3160
<223> /note="loxP site"

<220>
<221> gene
<222> 3352..4263
<223> /note="Synthetic ADE1 gene; codons optimized for Hansenula
      polymorpha"

<220>
<221> misc_feature
<222> 4493..4526
<223> /note="loxP site"

<220>
<221> misc_feature
<222> 4527..5558
<223> /note="HIS4 downstream region for homologous recombination of the
      integration cassette"

<400> 9
aaacgaggat tctggcccta caactgcaaa gaagctgtgc gagggcacgc tcgtcgtttg      60

ggccagccaa tgtgggcaac aatctcgcca acctggacgt tttgggctct tctccagtgc     120

ctgagaacaa tatcgagatg atctctaagg actcgattat cttctccaac atgaaggttg     180

tgcgatcgcc caacaggcaa gggactcttg ttgctgctct gttgttggat aaccagatat     240

ttgagctcaa cacaaagtcc atcagcaaaa aaggcccatt ggatttgaca actactgagc     300

tctttccctt gttagaggcg ctctacccaa aacccgagct gtttgtgctg ggtcttgggt 360

tcagcaaccg gatgttacac ccagacacga aaagacgctt ttccgagttg gggatcatgc 420

tcgaagtagg cgacaccaag agcgcagtgc tcagctacga tcttctggct acggaacgtt 480

ctccaaaaca ggtaggggca ctcatatttc catcggtgtt ctgaaatatg cgcgaaacaa 540

ctatttaagg gctctgacca ggaattgcgt gctgaacaca taggagtagg cagcaagggt 600

aaggacgctc agactgttga acacgaggct gattccatgg tacttgccga acttcttggt 660

caaagctgcg attttttcgt tggtctcggg agttctctcg ctctctggca gttcctcgag 720

cgcaaagcgt tcagtcttca gccggttgca cactggcatc agccacaagt aattggtgag 780

acctgagagc gaagaggccg ccagcaatgc aattccgacg gttgggatcg tgtatggtgc 840

agagagaccg accaccacgg ggccgatcga ctgcgtcagg aagtggatcg ggaacagctt 900

ggattgcagg aggccaaact ccttgcgggg caggaccttg aacgctctga cagaggacac 960

aaatgagtgg aacgaagtgg ctccgaaaac atacgagtaa gcaagcaggt gcacagcagt 1020

cagcatggta aaaattaatt gcacttatta tttatttggt cgtggcttta tttaatcagt 1080

gataattgtc acgttaatta acaatctccg gaatggtgat ctgatcgttc ctgaaaacct 1140

cgacattggc tccctcctga cacaggtact cgtacaggtt ccaggtaaac gagtcgtagt 1200

tgtcgatcat gacaacgttc ttagaagcgg ccggcatttt gaaggtgact aatagcctaa 1260

gaaaatattt aatttaattt tcattaaatt ttcctatact cgctatttca gcttttcatc 1320

tcatcacttc ataaacgata taaaccagaa aaagaactat tttcaaacac gcttctcaaa 1380

agcggtatgt ccttccacgt ctccttagaa tctggcaagt ccgcgagggg gatccttact 1440

acaggtcttc ctcagaaatc agcttctgct caatctggtc ggtctggaat ggaccaattc 1500

taaccaagtt ctctgggtcg tgctcaccac ccaactggtc ggtagaaaag tactctctag 1560

agttcaatgg ggcctgccat ctgtcagcca atcttctaaa caggccccaa gaagcctggt 1620

tgtctggagt aatggtagtc tccaagtggt gaacctcagc catctctggt ctggtcataa 1680

cggcctcaac caatcttcta gccagaccag tacctctagc cttctcacca acagcaacct 1740

gccacaaaaa gtaggtgtcc ggagcgttag acttcacgta accagacacg aaaccaacga 1800

tctcaccttc ctcgttggta gcaacagcac aagagtctct gaactgggta gccagcaaca 1860

ggtaagcgta agcagagtta acgtccagcg gtggacaaga cttaaccaac tcgtagatac 1920

cccagccgtc gtctggagat ggttttctga tgaacagtgg agaagcctcg tgaccaacaa 1980

cagcgtcagc aacagatggc ttagccaagt cagcagatgg agtgaatggc tcggtggtag 2040

cgttcatgaa ttcccgatga aggcagagag cgcaaggagg cggtatttat agtgccattc 2100

ccctctctga gagacccgga tggtagtcga gtgtatcgga gacagcttga tgtagactcc 2160

gtgcctgccg gctcctctta ttggcggaca ccagtgagac accccggaac ttgctgtttt 2220

tctgcaaaat ccggggtgac cagtgggagc ctatttgcac acacgagcgg gacaccccac 2280

tctggtgaag agtgccaaag tcattctttt tcccgttgcg gggcagccga ttgcatgttt 2340

taggaaaata ttacctttgc tacaccctgt cagatttacc ctccacacat atatattccg 2400

tcacctccag ggactattat tcgtcgttgc gccgccagcg gaagatatcc agaagctgtt 2460

ttccgagaga ctcggttggc gcctggtata tttgatggat gtcgcgctgc ctcacgtccc 2520

ggtacccagg aacgcggtgg gatctcgggc ccatcgaaga ctgtgctcca gactgctcgc 2580

ccagcaggtg tttcttgatc gccgcctcta aattgtccgc gcatcgccgg taacattttt 2640

ccagctcgga gtttgcgttt agatacagtt tctgcgatgc caaaggagcc tgcagattat 2700

aacctcggat gctgtcattc agcgctttta atttgacctc cagatagttg ctgtatttct 2760

gttcccattg gctgctgcgc agcttcgtat aactcgagtt attgttgcgc tctgcctcgg 2820

cgtactggct catgatctgg atcttgtccg tgtcgctttt cttcgagtgt ttctcgcaaa 2880

cgatgtgcac ggcctgcagt gtccaatcgg agtcgagctg gcgccgaaac tggcggatct 2940

gagcctccac actgccctgt ttctctatcc acggcggaac cgcctcctgc cgtttcagaa 3000

tgttgttcaa gtggtactct gtgcggtcaa tgaaggcgtt attgccggtg aaatctttgg 3060

gaagcggttt tcctcgggga agattacgaa attccccgcg tcgttgcgct tcctggatct 3120

cgatcgataa cttcgtatag catacattat acgaagttat atgcgcactc tagattcctt 3180

gcgtacaaac acctttccgt cagcgacacg ccttactttg cgcacacatc cgaacgagcc 3240

tcgtccgatg agctccaaca cttggaaatc gtccatcgaa tagtatattt acaaacttcc 3300

ggatacagaa aataattaaa ttatttggga cagcttggtt tatttcaaca cttaagccca 3360

ggttttaccg gtcaaagcct cgtaagcctc tttgtactta gcggcggtct tagcagcaat 3420

ctcctcggtc atagacacac cctctttacc gttcagctgg tgagaagtca accagtctct 3480

caggaactgc ttgtcgaaag actcctgaga tcttcccaac tcgtacttct tggcgtccca 3540

aaatctcgaa gagtctgggg tcaaaacctc gtcaaccaga accagctcgt tgttctcgtt 3600

cagtccgaac tcaaacttgg tgtcggccag gataatacct ctcgacagag cgtagtctct 3660

agccttagag tacagggcaa tagccttctg ggcaacctca gcacacaact cctctccaac 3720

caactgagca gcctgctctg gagaaatgtt ctcgtcgtgc tgtccctgct ctgctttggt 3780

agatggggtg aagattggct tctcgaaagc ctcagactcc tgcaatccag acttaactgg 3840

cagtccgtga acggtagacg acttcttgta ctctttccag gcagaaccgg tgatgtaacc 3900

tctcacaatg gcctccaatg gaaccagctt gtgcttcttc accagcagag atctaccagc 3960

caactgagcc ttgtactttg gctcagacaa ctgctctggc aacttggcaa agatgtcctt 4020

gtcagaggaa gcaaccaggt ggtttggaat aacgtcagac agcaggttga accagaacac 4080

agacagctgg gtcagaatct ttcccttgtc cttgatgccg ttctccataa tcacgtcgta 4140

ggcagagatt ctgtcggtgg caacaaacag cagcatgttg tcgtccacct ggtagatgtc 4200

tctcacttta cctctggcaa ccaatggcag aataccgtcc aggttagtct gggtcacggt 4260

catatgtgta ataataatta agtaaaatta gtttagtaaa ttaattgact cgtgagggtc 4320

gatcgacgcg atattggctc tcctttttat acgagttttt gtcaactttc ctctgctatg 4380

ctcaatccag tcaccgtcca caaagacttc tttgggtttc ctggggcctt ggttctgacc 4440

atcagtctac ctttggtggt tgttttcctg tatgcggcgt cgacggcgcg ccataacttc 4500

gtatagcata cattatacga agttatcctg caggttatga atttacggtg agatgtgagt 4560

caagctgaaa aatatcttga tttttttttt tttgacatgc cacgactttg gtagcatggg 4620

acgatatatc actatataca ctgtgagtca gttattgaac aaaaactgca tatcggccgc 4680

cgatagcctg ctagccgcgt tctggtcctg atccagcgtg gccttgacca tgtcagcctt 4740

cttctgctgc agctcaatga tccgcgactc gatgctgtcc tcgatgacca acttcacaat 4800

tctgataggc cggtgctgtc caatccggtg aattctgtct gcggcctgac ccgtgtccaa 4860

agcagggttc caccaactat ccaggataaa tacttgattg gcctcgacaa gattcagagc 4920

cacacctccg gccttcaaag acaccaaaaa tacctccacc gacggattgg tcataaagtg 4980

ctggatcaca gagtcacgct ggatcggggt catggatccc tgcaatttcg caatggaaaa 5040

acccgccctt ttgagtcgcc actccaccag gtccagcatc gacgtgaact gcgaaaagac 5100

aatgctttta acagtttgtc tgtccgaacg ggacttgtac aactcttcca ttagggcctc 5160

aattttagtg gaagacttcc attcgcctcc catgtctatt cggttcacaa tagagccctt 5220

ctcaacgacc tcctggttca tctccagcgc tggtgcctcc agatcaatac tcagcccaat 5280

gtggcacact ggacactcga gacgcttatc gtcgccgttg aacgactcca cgtactccgt 5340

gatacagatc cggcagaact tgtggtgaca tttggactct atcggctcct ctgcttcgtc 5400

atcacacagc tggcagacaa tggccccgct gttgaggaga tccctggatg ctttcttaaa 5460

tcgcttgaga accagatccg gatgatccgc aagctgtctc atacgcgtga ttagggtaaa 5520

gatgttggca tagttgttca gaaccacacc ttgtgcagc 5559

<210> 10
<211> 6195
<212> DNA
<213> Hansenula polymorpha (syn. Pichia angusta)

<220>
<221> source
<222> 1..6195
<223> /mol_type="unassigned DNA"
      /organism="Hansenula polymorpha (syn. Pichia angusta)"

<220>
<221> misc_feature
<222> 1..1097
<223> /note="LEU2 downstream region for homologous recombination of the
      integration cassette"

<220>
<221> terminator
<222> 1105..1432
<223> /note="MOX-T; terminator of MOX gene"

<220>
<221> gene
<222> 1442..2740
<223> /note="EctB-T7 fusion ORF (EctB coding region/T7 tag coding
      region)"

<220>
<221> promoter
<222> 2748..3813
<223> /note="FMD promoter"

<220>
<221> misc_feature
<222> 3820..3853
<223> /note="loxP site"

<220>
<221> gene
<222> 4045..4956
<223> /note="Synthetic ADE1 gene; codons optimized for Hansenula
      polymorpha"

<220>
<221> misc_feature
<222> 5186..5219
<223> /note="loxP site"

<220>
<221> misc_feature
<222> 5220..6194
<223> /note="LEU2 upstream region for homologous recombination of the
      integration cassette"

<400> 10
aaaccgattc ctgctgccgc acctcgcgag ccagctggag gctgacgggg agacgacgcc      60

aaaatgctgg atgcgcgctt ttcgctgagg ttgtctctgt cgtcgcgctg gcgtctgcgc     120

caaaacagga acaggagcag gagtcccaag agcagcaaag ctccgaggat cgacccgaca     180

gcgattccgg ccttcgcgcc cttgctgagc cccgtggacc tgttgttggc gctcgaggca     240

cttgaggacg acgcggcagc cgacgccgag gtggctgtag cgacggactg cgtgatgtag     300

atgatcgagg tctgcatgtt tccaggtgaa acggacgtag ttatccgcac tgattcggat     360

gagggtgtgg acgaagaggt ggttgaagac gacgacgagc tctgggagaa agaaaaggac     420

gaaatcgagc ttgttgtagt cgagctagcg gacagacttg aggacgttgg agacgagtct     480

gtggaagaca tggtggagct ctgtggggag gaactttgcg tggaggaaga cgatgttgag 540

gagctggaca gactggaact ggaggaggag gtggtggagg tggaggaggt ggaagtggca 600

tcgtctgtgg acgagtgcga cgactgcgcc gacgaactgt cgtcgtcgtt ttcctcagac 660

tcgtcaacat agacggaata agagtcgcta ccaccacact tctccatggg ataaccagtg 720

caggacgttg tgcacgagcc gtcggggttg tccgaaggta cgtcgtctcc gcaataacag 780

tcctggccgt tgatcaatgc agcaactgcg tgtcccgaac actgctccgt gcaatggctg 840

gagctctgcc atgtgtacga gtccttcttg gtcaagccgg aaatggcatc cgagctgtag 900

caacctaaat acgtaaagtc tgctaggact tgggtgatca cgaatacgac accgaatctc 960

atgttgacat ggaaaagcta aaaaataaac aatagagcgt cgcgtcgaaa aaaataaaat 1020

ctgtgaggcg tgcgtgttta ggatttttgg gattatttag gaattattcg agatcctact 1080

tttttttctc ccttatttaa ttaacaatct ccggaatggt gatctgatcg ttcctgaaaa 1140

cctcgacatt ggctccctcc tgacacaggt actcgtacag gttccaggta aacgagtcgt 1200

agttgtcgat catgacaacg ttcttagaag cggccggcat tttgaaggtg actaatagcc 1260

taagaaaata tttaatttaa ttttcattaa attttcctat actcgctatt tcagcttttc 1320

atctcatcac ttcataaacg atataaacca gaaaaagaac tattttcaaa cacgcttctc 1380

aaaagcggta tgtccttcca cgtctcctta gaatctggca agtccgcgag gggatccttt 1440

attagcccat ctgctggcca ccggtcatcg aggccatcga gaaggcctgc ttggtcgagg 1500

tttccaggat gtccaggcct tccaccaggt cctcgtctgg gatggtcagt gggcacaggc 1560

acttcaccac ctcgccgtcc tggcccgagg tctcgatgat caggccgttc tcgaaggcct 1620

ggtgggtgat cttgtcggcg atgtcgcccg agcccacgtc gatgcctctc atcaggcctc 1680

tgcctctttc cgaagcctcg atgccgttct ccgacagcca agcggcgatc ttgccgaatc 1740

tctcttccac gattctggcc tttctctgca cgtctctctc gaaggtgtcg tccgaccagt 1800

actttctcat ggctgcggca gcggtggcga aggccaggtt gaagcctctg aaggtgccgt 1860

tgtactggcc tggcttccac ttgtccagct ctggtctcat cagcacgtgg gcgaatggca 1920

ggccgtagcc cgacagcgac ttcgagttgg tcacgatgtc tggggtgatg ccggcgtgct 1980

cgaacgagaa gaacttgccg gttctgccgc agccggcctg gatatcgtcg atgatcagca 2040

ggatgtcgtt ggctctgcag atcgattcca gtctcttcag ccattccaga ccggccacgt 2100

tgatgccgcc ctcgccctgc acggtttcca cgatcacggc ggctggcacg tccaggccac 2160

ccgacttgtc gcccagcagc ttctcgaagt agtccagggt gtcggtcgac gagcccaggt 2220

agccgtcgaa tggcatgaac gaggcagcct gggttgggac gcctccggtg gcctctctga 2280

actttctgtt gccggtggta gccagagcgc ccatggtcac accgtggaag ccgttggtga 2340

acgacacgat gttgtgtctg cccttggcca ctctggccag tctgatggca gcttccacgg 2400

cgttggtgcc ggttgggcct ggcaggtgca ccttgtagtc caggcctctt ggcttcagga 2460

tcacctcttc cagggtttcc aggtagtctc tcttggcggc ggtccaaaag tccaggccgt 2520

gcacgatgcc gtccgagtcg atgtagtcca gcagggcctg cttcaggtgt gggttgttgt 2580

ggccgtagtt cagggtgcct gcgccagcca gaaagtcgat gtactctctg ccttcctcgt 2640

cggtcagtct ggcgtttctg gccttggtga acaccactgg gaacgatctc gagtaggttc 2700

tcacgtccga ttccattctc tccaggatct gggtctgcat gaattcccga tgaaggcaga 2760

gagcgcaagg aggcggtatt tatagtgcca ttcccctctc tgagagaccc ggatggtagt 2820

cgagtgtatc ggagacagct tgatgtagac tccgtgcctg ccggctcctc ttattggcgg 2880

acaccagtga gacaccccgg aacttgctgt ttttctgcaa aatccggggt gaccagtggg 2940

agcctatttg cacacacgag cgggacaccc cactctggtg aagagtgcca aagtcattct 3000

ttttcccgtt gcggggcagc cgattgcatg ttttaggaaa atattacctt tgctacaccc 3060

tgtcagattt accctccaca catatatatt ccgtcacctc caggactatt tattcgtcgt 3120

tgcgccgcca gcggaagata tccagaagct gttttccgag agactcggtt ggcgcctggt 3180

atatttgatg gatgtcgcgc tgcctcacgt cccggtaccc aggaacgcgg tgggatctcg 3240

ggcccatcga agactgtgct ccagactgct cgcccagcag gtgtttcttg atcgccgcct 3300

ctaaattgtc cgcgcatcgc cggtaacatt tttccagctc ggagtttgcg tttagataca 3360

gtttctgcga tgccaaagga gcctgcagat tataacctcg gatgctgtca ttcagcgctt 3420

ttaatttgac ctccagatag ttgctgtatt tctgttccca ttggctgctg cgcagcttcg 3480

tataactcga gttattgttg cgctctgcct cggcgtactg gctcatgatc tggatcttgt 3540

ccgtgtcgct tttcttcgag tgtttctcgc aaacgatgtg cacggcctgc agtgtccaat 3600

cggagtcgag ctggcgccga aactggcgga tctgagcctc cacactgccc tgtttctcta 3660

tccacggcgg aaccgcctcc tgccgtttca gaatgttgtt caagtggtac tctgtgcggt 3720

caatgaaggc gttattgccg gtgaaatctt tgggaagcgg ttttcctcgg ggaagattac 3780

gaaattcccc gcgtcgttgc gcttcctgga tctcgatcga taacttcgta tagcatacat 3840

tatacgaagt tatatgcgca ctctagattc cttgcgtaca aacacctttc cgtcagcgac 3900

acgccttact ttgcgcacac atccgaacga gcctcgtccg atgagctcca acacttggaa 3960

atcgtccatc gaatagtata tttacaaact tccggataca gaaaataatt aaattatttg 4020

ggacagcttg gtttatttca acacttaagc ccaggtttta ccggtcaaag cctcgtaagc 4080

ctctttgtac ttagcggcgg tcttagcagc aatctcctcg gtcatagaca caccctcttt 4140

accgttcagc tggtgagaag tcaaccagtc tctcaggaac tgcttgtcga aagactcctg 4200

agatcttccc aactcgtact tcttggcgtc ccaaaatctc gaagagtctg gggtcaaaac 4260

ctcgtcaacc agaaccagct cgttgttctc gttcagtccg aactcaaact tggtgtcggc 4320

caggataata cctctcgaca gagcgtagtc tctagcctta gagtacaggg caatagcctt 4380

ctgggcaacc tcagcacaca actcctctcc aaccaactga gcagcctgct ctggagaaat 4440

gttctcgtcg tgctgtccct gctctgcttt ggtagatggg gtgaagattg gcttctcgaa 4500

agcctcagac tcctgcaatc cagacttaac tggcagtccg tgaacggtag acgacttctt 4560

gtactctttc caggcagaac cggtgatgta acctctcaca atggcctcca atggaaccag 4620

cttgtgcttc ttcaccagca gagatctacc agccaactga gccttgtact ttggctcaga 4680

caactgctct ggcaacttgg caaagatgtc cttgtcagag gaagcaacca ggtggtttgg 4740

aataacgtca gacagcaggt tgaaccagaa cacagacagc tgggtcagaa tctttccctt 4800

gtccttgatg ccgttctcca taatcacgtc gtaggcagag attctgtcgg tggcaacaaa 4860

cagcagcatg ttgtcgtcca cctggtagat gtctctcact ttacctctgg caaccaatgg 4920

cagaataccg tccaggttag tctgggtcac ggtcatatgt gtaataataa ttaagtaaaa 4980

ttagtttagt aaattaattg actcgtgagg gtcgatcgac gcgatattgg ctctcctttt 5040

tatacgagtt tttgtcaact ttcctctgct atgctcaatc cagtcaccgt ccacaaagac 5100

ttctttgggt ttcctggggc cttggttctg accatcagtc tacctttggt ggttgttttc 5160

ctgtatgcgg cgtcgacggc gcgccataac ttcgtatagc atacattata cgaagttatc 5220

ctgcagggat tgcaaaatga tgcaactatt ttgcgccggt accgggaaaa attgaaaaac 5280

catccactta ctcattcctg tctttttatt tcgtattacc aaaccgctta cgtactcacc 5340

cactcagatc tgctcagaca caaactggtc gatgtcgacc tcctcgagtg gagcagtggc 5400

cgcattgttc acctttggct tctcctctct gagctcaaga cgtttcagct tactaagagg 5460

gccccagccg tgcacaggtg ggctaagttt gaatggatcc aaaaacttga cacactgttt 5520

gaacgagtcg cccaatgtgg caatctcgtg gatgatgtct tccacacaga tgatgccgta 5580

gtctccaagc tggtcttcaa tcaaattgtt gtcatccaag ttcaccttct tggttgttcc 5640

gtcctcgacg atactagccg tggcacgttt ctggatcaag tttcggacag tagcaagcga 5700

tggagtgccg atggcgatgt acggactggt cagtctgagg agaggtttga cggcagcgtt 5760

caacttgatg aaagttcctg tgtacacgtg ctctagacgc agcagttcaa gcacttttct 5820

ggctttggag ggcactttgg caccatgcgg gccggggata cggaccacaa acaaaagacg 5880

ctcatcggac tcttccggca cagcagagct ccgttcatgc tgagacacgc gcttcacacg 5940

gtactgttct ctttctgatg cgcgatgttt agccactagg gtttctgctc tgataaacct 6000

tgcctttctt tgctgttgtt tgcgtttctt ctgttcctcg agcttcttac gtgcctgctc 6060

ctgtttctct agacggagtc tgtcggcgtt cttccgcttc ttgagcaaca cctctgggtt 6120

ggtgttcagt ttagtcgatt cagccatgat aaaaaaaaaa aaaattgtgc agaaaaattt 6180

atttggcagt gttgc 6195

<210> 11
<211> 6965
<212> DNA
<213> Hansenula polymorpha (syn. Pichia angusta)

<220>
<221> source

<222> 1..6965
<223> /mol_type="unassigned DNA"
      /organism="Hansenula polymorpha (syn. Pichia angusta)"

<220>
<221> misc_feature
<222> 1..1007
<223> /note="ODC1 downstream region for homologous recombination of the
      integration cassette"

<220>
<221> terminator
<222> 1015..1342
<223> /note="MOX-T; terminator of MOX gene"

<220>
<221> gene
<222> 1352..1792
<223> /note="EctC-HA fusion ORF (EctC coding region/HA tag coding
      region)"

<220>
<221> promoter
<222> 1800..2865
<223> /note="FMD promoter"

<220>
<221> misc_feature
<222> 2872..2905
<223> /note="loxP site"

<220>
<221> gene
<222> 3205..5709
<223> /note="H. polymorpha HIS4"

<220>
<221> misc_feature
<222> 5864..5897
<223> /note="loxP site"

<220>
<221> misc_feature
<222> 5898..6964
<223> /note="ODC1 upstream region for homologous recombination of the
      integration cassette"

<400> 11
aaacaggccg tctcgatttg actacctcac aacagcaaga gctttgttgc atggagagct        60

tgcctccaaa ccgtggagct tacgaacaca ttcaggaata gctcttctat accaatcaat 120

caaaaaagaa gggtggaatt ttattccacg acaagtactt cctccaatag tggcaaacag 180

cgcaattgga gtgattcttt attctaccta tctcacacta ttacaacgtt ttaataagag 240

aaatatgaac tcaattgatg atcctagtcc tgtcgacact ttcaaagctg gttttgttgc 300

gggagctgct gcgagcttgg ctgcatcacc gctagatgca ctttacgccc gatcgaatta 360

ctcagagctt gtcagtggaa aacagaagag catgtgggag tatgccttat acaaactaaa 420

acaaattggt ttagttggaa tatttgccgg cttcagttta aatttcataa aggaaagcct 480

tggatttgca ttctactttt cagtatttga actagtgaaa aaccaggggt accacaagac 540

aaaagcagct atacagtggt atcaatattg gcgctcgaga ctttttcatg atgaagaaaa 600

ccaggagaag aattcaagat ttttcaaagc tttgaatttg acgtttatcc ttcttgctgg 660

agcttccgcc gcaactacac tgcttgcaat ccaatatcca ctcaataaaa ttcaaaaggt 720

ccacctgact agactcgaat ctctagatct ctacaacgaa gcacatcaac tggatcggcg 780

tcgcttcttc aaactctact ataattctta cattgagaca tttgatatta ttctacaacg 840

atggcgaaaa tctcacctct ccctctttgg cttctgctat agaggctttt tcagatcgtc 900

cttcactagt attcccgcga cttctattgg gcttttcgta ttcgaaatat cccgtcagca 960

ttttagcgaa aatcaatttt gacgatgtat atagaactga aagccgttaa ttaacaatct 1020

ccggaatggt gatctgatcg ttcctgaaaa cctcgacatt ggctccctcc tgacacaggt 1080

actcgtacag gttccaggta aacgagtcgt agttgtcgat catgacaacg ttcttagaag 1140

cggccggcat tttgaaggtg actaatagcc taagaaaata tttaatttaa ttttcattaa 1200

attttcctat actcgctatt tcagcttttc atctcatcac ttcataaacg atataaacca 1260

gaaaaagaac tattttcaaa cacgcttctc aaaagcggta tgtccttcca cgtctcctta 1320

gaatctggca agtccgcgag ggggatccct attaggcgta gtctggcacg tcgtatgggt 1380

acaatggttt ctggtcgtca gcctcgtcag ctggagcgta agaaccgtcc tctctgtgaa 1440

cctcgttacc agtcaaacct ggggtaaaaa cacaggccag gtgcatagtc ttagaagctc 1500

tcaacaggtg ctcgtcgtgc tggtccaaaa tgtagatgtc gcctggctta ataggccaga 1560

tcttaccgtc agccaaagtc tcaacctcgc cctcaccctc aatacagtac acggcctcga 1620

agtggtgttt gtagtggatg tgagtctcgg ttccctcgaa gattctggtg atgtggaaag 1680

agcagttacc accgtcctca gccaaagaca atctggtaga gtcccagtta ccgttctcag 1740

cagtaaccaa tctgtcggtc tgtctagctt cttccaagtt tctcacgatc atgaattccc 1800

gatgaaggca gagagcgcaa ggaggcggta tttatagtgc cattcccctc tctgagagac 1860

ccggatggta gtcgagtgta tcggagacag cttgatgtag actccgtgcc tgccggctcc 1920

tcttattggc ggacaccagt gagacacccc ggaacttgct gtttttctgc aaaatccggg 1980

gtgaccagtg ggagcctatt tgcacacacg agcgggacac cccactctgg tgaagagtgc 2040

caaagtcatt ctttttcccg ttgcggggca gccgattgca tgttttagga aaatattacc 2100

tttgctacac cctgtcagat ttaccctcca cacatatata ttccgtcacc tccagggact 2160

attattcgtc gttgcgccgc cagcggaaga tatccagaag ctgttttccg agagactcgg 2220

ttggcgcctg gtatatttga tggatgtcgc gctgcctcac gtcccggtac ccaggaacgc 2280

ggtgggatct cgggcccatc gaagactgtg ctccagactg ctcgcccagc aggtgtttct 2340

tgatcgccgc ctctaaattg tccgcgcatc gccggtaaca tttttccagc tcggagtttg 2400

cgtttagata cagtttctgc gatgccaaag gagcctgcag attataacct cggatgctgt 2460

cattcagcgc ttttaatttg acctccagat agttgctgta tttctgttcc cattggctgc 2520

tgcgcagctt cgtataactc gagttattgt tgcgctctgc ctcggcgtac tggctcatga 2580

tctggatctt gtccgtgtcg cttttcttcg agtgtttctc gcaaacgatg tgcacggcct 2640

gcagtgtcca atcggagtcg agctggcgcc gaaactggcg gatctgagcc tccacactgc 2700

cctgtttctc tatccacggc ggaaccgcct cctgccgttt cagaatgttg ttcaagtggt 2760

actctgtgcg gtcaatgaag gcgttattgc cggtgaaatc tttgggaagc ggttttcctc 2820

ggggaagatt acgaaattcc ccgcgtcgtt gcgcttcctg gatctcgatc gataacttcg 2880

tatagcatac attatacgaa gttatatgcg cactctagag caattccgac ggttgggatc 2940

gtgtatggtg cagagagacc gaccaccacg gggccgatcg actgcgtcag gaagtggatc 3000

gggaacagct tggattgcag gaggccaaac tccttgcggg gcaggacctt gaacgctctg 3060

acagaggaca caaatgagtg gaacgaagtg gctccgaaaa catacgagta agcaagcagg 3120

tgcacagcag tcagcatggt aaaaattaat tgcacttatt atttatttgg tcgtggcttt 3180

atttaatcag tgataattgt cacgctatat gtagccaagt ttttccattc taatcttgac 3240

ggcgttgcgg tgcgcgtcga gaccctcgac ggcagccagg tccatcacgg ccttgccaat 3300

gctcttgaga ccctccttgg tgaccacctg cgatgtaatg aatttctgga acgtggtggt 3360

gttaacaccc gagtacattc tggcgtagcc gtacgtaggc agcgtgtggt tggttcccga 3420

ggagtagtca ccgcagcttt ccggcgaata ggcacccaca aacacagagc cagcattgtc 3480

gaccttgtcg acccatttct cggcgtcctg gatctgcagg ataaggtgct ccggagcgta 3540

tctgttgctc atctcgaacg cctcatcgta gctcttcaca atgatcgtgg tggagtgagc 3600

aatgcactgg cgcacgatct ccacacgtgg cagctgcagc gcctggcgat ggacggcctc 3660

gtcgatcttg tcgatctcgg cagacgtcat gtccacggcg atcaatatca cttgcgagtc 3720

aacgccgtgt tctgcctgcg acagcaggtc cgaggccaca aagtctgggt cggcgtactt 3780

gtccgcgaca accagaacct cggatggccc ggcaggcata tcaatggcac acatggcgtt 3840

ggaatcattg gaaacgagca tcttggctgc cgtcacaaac tggtttccgg ggcccatgat 3900

cttgtcacat ttaggcacag actcggtacc gtacgccatt gcggccaccg cctgggcccc 3960

tccggcaagc acaatgcact tggcacccac cttgtgcgcc acatagacca cctctggcgt 4020

cagacggccg tccttgccgg gaggagacgc aaacacaata tctttgcaac cggcaacgag 4080

agcaggcaca gagagcataa gggacgtgga cggcagcacg gcagttccgc cggggatgta 4140

acagccaaca cgctcgatcg gacgtgcaaa tcgagagcac acaacgccgg gacacgtttc 4200

cacgcgtaga gtctctttct ggttctgcgc ttcgtggaag gtcttgatat tggcaaacga 4260

gatgtcaatg gccttgcgca cgtcttcgct gatgttcatc atttctgctg ggaatggagc 4320

gttcaataca ggagagctaa gctgcacctt gtcgaacttg gctgtgagct ccaccaacgc 4380

agcgtctcct tttgttctca ctttctcgac gatcggcgta accaggttca tgatatccgc 4440

agacttttgg attggtcttt gaagacattt ttcgatatct tctttggagg cagtggtggc 4500

atccacacgg ttcatcacga tgcggccatc tccgttcaca agccgctttg gcaattttgg 4560

caccgttggc accgttggtt cctctagctt tttttccgta gcgtcctgct caacatactt 4620

cgcctttgcg tcgcccttac gtctcgtcac cttcagggcc ttgatgtcca aattccgctc 4680

aacgtctgca agcgaaacgc cgtacttcac acatctggca agcgcaaagt agaccaaatc 4740

ggcagcttcc cacgcaatct cgtccttcga ggtggcttcc accagctcgt cggcttcttc 4800

cctaatcttg gcaccgagca gcttctcatc ctcaaacagt ctcttcgtgt aggagccttc 4860

aggagcattc gccttgcggt cccaaagagt ggcttccaga gctgccagac ccttggacga 4920

gccaaaacag gaggcagtgt cgaggtggca gaaaccagca ccggcctgtt ccacaacaaa 4980

tttcaaacaa tcgctatcgc agtctagctc cagttggagc aatttttgcg tggctcccga 5040

tgtctggcct ttgtaccaca agccgtgctt tctggactgg tagacacccg tgccggtcct 5100

tattgcctca gcaatggact tcttggacga gtacaccaaa ccaagagcct ggttcctgct 5160

gtcagtgaca agagttgtgt ataagccgtc tggtctgtcg gtgttcatgg cagcgagata 5220

gcactccaca aacggcgttt tggtgcccgc gttttcgtac tccttagtca gctgatcgag 5280

ctcaataaca gcagtgaagc ccttggcgat ctgccggagt ggttgttcaa aataaccaaa 5340

gactcttctt tttccaattt tcacttcagt ctccccaaaa acacagatat tctcggcagc 5400

atcttttcca tccttgatga ccagtctggc gttgggaata cggcttctaa tctgctcagc 5460

aacagcagag gtgacaaaaa gaccgtacac accgttgttc aaaagagcca gaacaatgtc 5520

tatttggcta tcattttcca cagaccccag agcccagtcg acatatacct ggcctagata 5580

ctctttagcc aacgcgataa tttcctgcac agcggcagcc gcatcaagcg acacaagaac 5640

ctcgctcaca aacggcacgc tcaaaacgtc ccttggttga gcaacgctca caaggatagg 5700

agaaaccatt tatgaattta cggtgagatg tgagtcaagc tgaaaaatat cttgattttt 5760

ttttttttga catgccacga ctttggtagc atgggacgat atatcactat atacactgtg 5820

agtcagttat tgaacaaaaa ctgcatatcg tcgacggcgc gccataactt cgtatagcat 5880

acattatacg aagttatcct gcaggttgga ttattattca gggaaatgtt gaaaattttt 5940

cgataattca ttagtttatt tttgatacac catggatcaa ctcaactata aagaacagca 6000

agaattccag aaaattgtgg agcagaagca aatgaatgac ttcatgagag taagtgaacg 6060

ccttctgaaa actaacattg cagctctaca ccagtttggt ggacagatgt tttaatgatt 6120

gcgtcaacga cttcacctcc aacaaactta gctctagaga agaaacatgt ttgaacaagt 6180

gcgctgaaaa gttcctcaag cactccgaaa gagttggttt aagattccag gaacagaacc 6240

agatgttgat gaaccagatg agaggcggca attaatgagc tttaattgct ggatgattcc 6300

cggaataggc tcaacaagtt ttgcgtttct tccatcattt caatcgtcca tttgtaagtt 6360

tctgtctgat tcatatcgtt gtacataatt gcgttgctga acatcagttg aagctcaaat 6420

tccaactcgt cgaatgactt tatttcaccc gtcctaatag ctttacccaa actcttaatg 6480

tccacgggat gccgtattag cttgtagtag ttgggctctt cgttttcatt cactggttgc 6540

atgaacatag acgcaaaccg gttggatgat atctgtgtga gaagttgtgc agacagagca 6600

ttgaagcgct tagtacaacc aggggggacg ctttcggatc cgcgtcgctt gctcccacgt 6660

tcttcatctc cacttgctgg cgtagatggt gtagcaggtt ctggagttgg cgatggtatt 6720

tctttagaag ccgacttgga ttccaatttc gtttggggtt gggattctac attcgactct 6780

gaggttgaag ctgggccttc ttgaggttcg gcatgagttt ccaataactc tgttttgctt 6840

ttttccaaag cagtgaccgg ctgagattga gccgctgatt ctgcctcggg ctccgcaact 6900

tcatcgatct ctttgtctgc gtcattatgc gtcacgccct cttcaggttc ttgtgcgtca 6960

cctgc 6965

<210> 12
<211> 6213
<212> DNA
<213> Hansenula polymorpha (syn. Pichia angusta)

<220>
<221> source
<222> 1..6213
<223> /mol_type="unassigned DNA"
      /organism="Hansenula polymorpha (syn. Pichia angusta)"

<220>
<221> misc_feature
<222> 5..1061
<223> /note="ADE1 upstream region for homologous recombination of the
      integration cassette"

<220>
<221> misc_feature
<222> 1068..1101
<223> /note="loxP site"

<220>
<221> gene
<222> 1365..2453
<223> /note="Synthetic H. polymorpha LEU2 gene; codons optimized for
      Hansenula polymorpha"

<220>
<221> misc_feature
<222> 2670..2703
<223> /note="loxP site"

<220>
<221> promoter
<222> 2710..3775
<223> /note="FMD promoter"

<220>
<221> gene
<222> 3783..4805
<223> /note="H6-EctD fusion ORF (start methionine/His tag and flanking
      residues coding region/EctD coding region)"

<220>
<221> terminator
<222> 4815..5142
<223> /note="MOX-T; terminator of MOX gene"

<220>
<221> misc_feature
<222> 5148..6213
<223> /note="ADE1 downstream region for homologous recombination of the
      integration cassette"

<400> 12
ggccgccaca ccactgaagg ctcccgaaga cagatgcaca ggtctgagcg agagatatct      60

tgcttgcagg gagtaagtga atggaaccca acacagatcg ccaaatgcga gcatgaaacc     120

gaatccgtcg gtggtaatgt ccatcatgga aagcacgcct tcctcgttga gcactccgtc     180

gaaaatgtag aaggattgca tcgcaacgac gaacaagagc gagttagcca ccgttccagt     240

ttccagatac tgtttttgga tgcatgagag gtctatcagc agccaaagca gcattcctgg     300

ccgtagctcg caaaagagct tgatgtccca gggtcctatt ctggggttga gctctctgcc     360

gataaaccag tcaaaaagga cgtttttgga gtttccaccc tccgcaagaa tgcgctcgta 420

cgttcctgca ccattgggcc gtctcagcgg gaaaaaggag cagacgtaga caaacgtgct 480

gagaataaga gaaaattccc acgaaacgac catcaggggc aaaaaattgt cataaataaa 540

ctgaagttcg ggcacattgc cccttgtgta caacgctctt gctgccaaag ctgcacagac 600

caaaacactc agctccttac cgttgatcag atacgttaaa tgtgttccgt cacgcagcgt 660

tgtgcctttt ttatagtagc caggaacaat aagatccagc aatgcaagac caaaaaacca 720

gaccaggtat gctatccagc atttggcact cagaaacgtt tccttggtga gttgttctgc 780

aagcaagttt ttgatcgtgt tgatctgcaa attgcacccc tcgacaacat accgatcatt 840

gcacgccgca tacaggaaaa caaccaccaa aggtagactg atggtcagaa ccaaggcccc 900

aggaaaccca aagaagtctt tgtggacggt gactggattg agcatagcag aggaaagttg 960

acaaaaactc gtataaaaag gagagccaat atcgcgtcga tcgaccctca cgagtcaatt 1020

aatttactaa actaatttta cttaattatt attacacatc ctgcaggata acttcgtata 1080

atgtatgcta tacgaagtta tggcgcgccg tcgactgtgc acggctgggg ccctcttagt 1140

aagctgaaac gtcttgagct cagagaggag aagccaaagg tgaacaatgc ggccactgct 1200

ccactcgagg aggtagacat cgaccagttt gtgtctgagc agatctgagt gggtgagtac 1260

gtaagcggtt tggtaatacg aaataaaaag acaggaatga gtaagtggat ggtttttcaa 1320

tttttcccgg taccggcgca aaatagttgc atcattttgc aatcatgtcg aagaacatcg 1380

ttctgttgcc aggtgaccac gttggaccag aggttgtggc tgaggctgtt aaggttttgg 1440

aggccgtttc ttctgccatc ggcgtgaagt tcaacttctc gaagcacctg attggtggtg 1500

cttctattga cgcctacggc gttccattgt ctgacgaggc tttggaggct gctaaaaagg 1560

ctgacgctgt tctgttgggt gctgttggtg gtccaaaatg gggaaccggc tctgttagac 1620

cagagcaggg actgctgaag atcagaaaag agctgaacct gtacgctaac cttagaccat 1680

gttcttttgc ctctgacgct ctgttgaagc tgtctccact gaagtctgag atcgtgaagg 1740

gaaccgactt cgtggttgtg agagagctgg ttggcggtat ctacttcggc gacagaaaag 1800

aggacgctgg cgacggcgtt gcttctgaca ctgagagcta ctctgttcca gaggtgcaga 1860

gaatcaccag aatggccgct tttctggctt tgcagtctga cccaccattg ccattgtggt 1920

ctttggacaa ggctaacgtg ttggcttctt cgagactgtg gagaaagacc gtggaggaaa 1980

ctatcaagaa cgagttccca cagttgactg ttcagcacca gctgattgac tctgccgcca 2040

tgatcttggt taagtcgcca accaagctga acggtgttat cgtgaccaac aacatgttcg 2100

gcgacatcat ttcggacgag gcctctgtta ttccaggttc tctgggattg ctgccatctg 2160

catctttggc ttccctgcca gacaccaaca aggctttcgg tctgtacgag ccatgtcacg 2220

gttctgctcc agacttgggt ccaggaaagg ttaacccact ggccaccatt ttgtctgctg 2280

ccatgatgct gaagctgtct ttggacttgg ttgacgccgg tagagctatt gagcaggccg 2340

tgaagaacgt tttggacgcc ggtattatga ctgctgacct gggcggttct tcttctactc 2400

aggaagttgg cgacgctgtt gcacaggaag ttgccaagct gctgaagaac taaataaggg 2460

agaaaaaaaa gtaggatctc gaataattcc taaataatcc caaaaatcct aaacacgcac 2520

gcctcacaga ttttattttt ttcgacgcga cgctctattg tttatttttt agcttttcca 2580

tgtcaacatg agattcggtg tcgtattcgt gatcacccaa gtcctagcag actttacgta 2640

tttaggttgc tacactctag agtgcgcata taacttcgta taatgtatgc tatacgaagt 2700

tatcgatcga gatccaggaa gcgcaacgac gcggggaatt tcgtaatctt ccccgaggaa 2760

aaccgcttcc caaagatttc accggcaata acgccttcat tgaccgcaca gagtaccact 2820

tgaacaacat tctgaaacgg caggaggcgg ttccgccgtg gatagagaaa cagggcagtg 2880

tggaggctca gatccgccag tttcggcgcc agctcgactc cgattggaca ctgcaggccg 2940

tgcacatcgt ttgcgagaaa cactcgaaga aaagcgacac ggacaagatc cagatcatga 3000

gccagtacgc cgaggcagag cgcaacaata actcgagtta tacgaagctg cgcagcagcc 3060

aatgggaaca gaaatacagc aactatctgg aggtcaaatt aaaagcgctg aatgacagca 3120

tccgaggtta taatctgcag gctcctttgg catcgcagaa actgtatcta aacgcaaact 3180

ccgagctgga aaaatgttac cggcgatgcg cggacaattt agaggcggcg atcaagaaac 3240

acctgctggg cgagcagtct ggagcacagt cttcgatggg cccgagatcc caccgcgttc 3300

ctgggtaccg ggacgtgagg cagcgcgaca tccatcaaat ataccaggcg ccaaccgagt 3360

ctctcggaaa acagcttctg gatatcttcc gctggcggcg caacgacgaa taatagtccc 3420

tggaggtgac ggaatatata tgtgtggagg gtaaatctga cagggtgtag caaaggtaat 3480

attttcctaa aacatgcaat cggctgcccc gcaacgggaa aaagaatgac tttggcactc 3540

ttcaccagag tggggtgtcc cgctcgtgtg tgcaaatagg ctcccactgg tcaccccgga 3600

ttttgcagaa aaacagcaag ttccggggtg tctcactggt gtccgccaat aagaggagcc 3660

ggcaggcacg gagtctacat caagctgtct ccgatacact cgactaccat ccgggtctct 3720

cagagagggg aatggcacta taaataccgc ctccttgcgc tctctgcctt catcgggaat 3780

tcatgaaaca ccatcatcac caccaccagt ctgttcagac ctcgtctaac agaccattgc 3840

cacaggctaa cctgcacatt gctaccgaga ctccagaggc tgactcgaga attagatcgg 3900

ctccaagacc aggacaggac ccatacccaa ccagattgtc tgagccattg gacttgcctt 3960

ggctgaacag aagagagcca gttgttaaag gcgaggaggc tgacggtcca ttgtctgctg 4020

ctcagttgga cacctttgag agacagggct tcattttcga gccagacttt ttgaagggcg 4080

aggagttgga ggctttgaga cacgagctga acgctttgtt ggctagagat gacttcagag 4140

gtagagactt cgctattacc gagccacagg gcaacgagat tagatcgctg ttcgccgttc 4200

actacctgtc gagagtgttt tcgagactgg ccaacgacga gagattgatg ggtagagcca 4260

gacagatttt gggtggagag ccatacgttc accagtcgag aatcaactac aagccaggct 4320

ttgagggaaa gggtttcaac tggcactctg actttgagac ttggcacgct gaggacggaa 4380

tgccagctat gcacgccgtt tctgcctcta ttgtgctgac cgacaaccac acttttaacg 4440

gcccactgat gttggttcca ggctctcaca gagtgttcgt tccatgtctg ggagagactc 4500

cagaggacca ccacagacag tctctgaaaa cccaggagtt cggagttcca tcgagacagg 4560

ctctgagaga gctgattgac agacacggta ttgaggctcc aactggtgct gccggtggtt 4620

tgttgctgtt cgactgcaac actctgcacg gttctaacgc caacatgtcg ccagacccaa 4680

gatcgaacgc cttcttcgtg tacaatagaa gagacaacag atgcgttgag ccatacgccg 4740

cttctaagag aagaccaaga ttcttggctc acgagccaga cgaggcttgg tcgccagacg 4800

gttaataagg atccccctcg cggacttgcc agattctaag gagacgtgga aggacatacc 4860

gcttttgaga agcgtgtttg aaaatagttc tttttctggt ttatatcgtt tatgaagtga 4920

tgagatgaaa agctgaaata gcgagtatag gaaaatttaa tgaaaattaa attaaatatt 4980

ttcttaggct attagtcacc ttcaaaatgc cggccgcttc taagaacgtt gtcatgatcg 5040

acaactacga ctcgtttacc tggaacctgt acgagtacct gtgtcaggag ggagccaatg 5100

tcgaggtttt caggaacgat cagatcacca ttccggagat tgttaattaa gtgttgaaat 5160

aaaccaagct gtcccaaata atttaattat tttctgtatc cggaagtttg taaatatact 5220

attcgatgga cgatttccaa gtgttggagc tcatcggacg aggctcgttc ggatgtgtgc 5280

gcaaagtaag gcgtgtcgct gacggaaagg tgtttgtacg caaggaaata tcatacgggg 5340

ccatgaatag caaggagaag caccagctta gcaccgagtt caggatccta cgagaactga 5400

gacatcccaa cattgtgcag tatctccacc atgaccatgt ccaggatgag caccaggttc 5460

atttgtatat ggagtactgc gacggaggcg atctatcggt gctgataaag aagtacaagg 5520

agagaaacga gtttataccg gaaacactga tctggcagat atttacccag gtgttgaatg 5580

cgctttacaa atgtcactat ggaacgtcga tagagccggt cagcaatctt ttccagtcgt 5640

ctcccgaact agaaccgaaa cctgtggacc atcaggtgat tattcatcgc gacatcaaac 5700

cagacaacat tttccttttg tcggacgggt attcgatcaa actgggggat ttcggtcttg 5760

caaagtggct ccaacacgaa aacgagtttg ccaagaccta tgtgggcacg ccatactaca 5820

tgtctcccga ggtgctgatg gatagaccgt acgaccccgt ctgcgacgtg tggtcgctcg 5880

gatgcgtcat gtatgaattg tgtgctctac gtcctccttt ccaggccaag actcacttgg 5940

cattgcagga aaaaatcaag gctggcacgt tccagccgat ccctgaccat tactcgcata 6000

gactacagat ggtcatcaac gcatgtctta ttgtcgaccc catggaacgc gctaccgtca 6060

accaactgct acaggacgcc tctttcaaat tgttcagaaa agagtgggaa atgacgcaat 6120

gggagaaccg gctgcgcgag cacgaaacag agctgcttct taaggaacgc aagctggtca 6180

agtttgagga cgctctcaac gaagaatgcg ttt 6213

SEQUENCE LISTING

<110> Artes Biotechnology GmbH

<120> A method for producing ectoine or a derivative thereof and a yeast
cell for use as a host cell in such a method

<130> A638EP

<160> 12

<170> BiSSAP 1.2

<210> 1
<211> 202
<212> PRT
<213> Hansenula polymorpha (syn. Pichia angusta)

<220>
<221> CHAIN
<222> 1..192
<223> EctA

<220>
<221> CHAIN
<222> 193..202
<223> myc tag

<400> 1
Met Asn Ala Thr Thr Glu Pro Phe Thr Pro Ser Ala Asp Leu Ala Lys
1               5                   10                  15
Pro Ser Val Ala Asp Ala Val Val Gly His Glu Ala Ser Pro Leu Phe
            20                  25                  30
Ile Arg Lys Pro Ser Pro Asp Asp Gly Trp Gly Ile Tyr Glu Leu Val
        35                  40                  45
Lys Ser Cys Pro Pro Leu Asp Val Asn Ser Ala Tyr Ala Tyr Leu Leu
    50                  55                  60
Leu Ala Thr Gln Phe Arg Asp Ser Cys Ala Val Ala Thr Asn Glu Glu
65                  70                  75                  80
Gly Glu Ile Val Gly Phe Val Ser Gly Tyr Val Lys Ser Asn Ala Pro
                85                  90                  95
Asp Thr Tyr Phe Leu Trp Gln Val Ala Val Gly Glu Lys Ala Arg Gly
            100                 105                 110
Thr Gly Leu Ala Arg Arg Leu Val Glu Ala Val Met Thr Arg Pro Glu
        115                 120                 125
Met Ala Glu Val His His Leu Glu Thr Thr Ile Thr Pro Asp Asn Gln
    130                 135                 140
Ala Ser Trp Gly Leu Phe Arg Arg Leu Ala Asp Arg Trp Gln Ala Pro
145                 150                 155                 160
Leu Asn Ser Arg Glu Tyr Phe Ser Thr Asp Gln Leu Gly Gly Glu His
                165                 170                 175
Asp Pro Glu Asn Leu Val Arg Ile Gly Pro Phe Gln Thr Asp Gln Ile
            180                 185                 190
Glu Gln Lys Leu Ile Ser Glu Glu Asp Leu
        195                 200

<210> 2
<211> 432
<212> PRT
<213> Hansenula polymorpha (syn. Pichia angusta)

<220>
<221> CHAIN

<222> 1..421
<223> EctB

<220>
<221> CHAIN
<222> 422..432
<223> T7 tag

<400> 2
Met Gln Thr Gln Ile Leu Glu Arg Met Glu Ser Asp Val Arg Thr Tyr
1               5                   10                  15
Ser Arg Ser Phe Pro Val Val Phe Thr Lys Ala Arg Asn Ala Arg Leu
            20                  25                  30
Thr Asp Glu Glu Gly Arg Glu Tyr Ile Asp Phe Leu Ala Gly Ala Gly
        35                  40                  45
Thr Leu Asn Tyr Gly His Asn Asn Pro His Leu Lys Gln Ala Leu Leu
    50                  55                  60
Asp Tyr Ile Asp Ser Asp Gly Ile Val His Gly Leu Asp Phe Trp Thr
65                  70                  75                  80
Ala Ala Lys Arg Asp Tyr Leu Glu Thr Leu Glu Glu Val Ile Leu Lys
                85                  90                  95
Pro Arg Gly Leu Asp Tyr Lys Val His Leu Pro Gly Pro Thr Gly Thr
            100                 105                 110
Asn Ala Val Glu Ala Ala Ile Arg Leu Ala Arg Val Ala Lys Gly Arg
        115                 120                 125
His Asn Ile Val Ser Phe Thr Asn Gly Phe His Gly Val Thr Met Gly
    130                 135                 140
Ala Leu Ala Thr Thr Gly Asn Arg Lys Phe Arg Glu Ala Thr Gly Gly
145                 150                 155                 160
Val Pro Thr Gln Ala Ala Ser Phe Met Pro Phe Asp Gly Tyr Leu Gly
                165                 170                 175
Ser Ser Thr Asp Thr Leu Asp Tyr Phe Glu Lys Leu Leu Gly Asp Lys
            180                 185                 190
Ser Gly Gly Leu Asp Val Pro Ala Ala Val Ile Val Glu Thr Val Gln
        195                 200                 205
Gly Glu Gly Gly Ile Asn Val Ala Gly Leu Glu Trp Leu Lys Arg Leu
    210                 215                 220
Glu Ser Ile Cys Arg Ala Asn Asp Ile Leu Leu Ile Ile Asp Asp Ile
225                 230                 235                 240
Gln Ala Gly Cys Gly Arg Thr Gly Lys Phe Phe Ser Phe Glu His Ala
                245                 250                 255
Gly Ile Thr Pro Asp Ile Val Thr Asn Ser Lys Ser Leu Ser Gly Tyr
            260                 265                 270
Gly Leu Pro Phe Ala His Val Leu Met Arg Pro Glu Leu Asp Lys Trp
        275                 280                 285
Lys Pro Gly Gln Tyr Asn Gly Thr Phe Arg Gly Phe Asn Leu Ala Phe
    290                 295                 300
Ala Thr Ala Ala Ala Ala Met Arg Lys Tyr Trp Ser Asp Asp Thr Phe
305                 310                 315                 320
Glu Arg Asp Val Gln Arg Lys Ala Arg Ile Val Glu Glu Arg Phe Gly
                325                 330                 335
Lys Ile Ala Ala Trp Leu Ser Glu Asn Gly Ile Glu Ala Ser Glu Arg
            340                 345                 350
Gly Arg Gly Leu Met Arg Gly Ile Asp Val Gly Ser Gly Asp Ile Ala
        355                 360                 365
Asp Lys Ile Thr His Gln Ala Phe Glu Asn Gly Leu Ile Ile Glu Thr
    370                 375                 380
Ser Gly Gln Asp Gly Glu Val Val Lys Cys Leu Cys Pro Leu Thr Ile
385                 390                 395                 400
Pro Asp Glu Asp Leu Val Glu Gly Leu Asp Ile Leu Glu Thr Ser Thr
                405                 410                 415
Lys Gln Ala Phe Ser Met Ala Ser Met Thr Gly Gly Gln Gln Met Gly
            420                 425                 430

<210> 3
<211> 146
<212> PRT
<213> Hansenula polymorpha (syn. Pichia angusta)


<220>
<221> CHAIN
<222> 1..137
<223> EctC

<220>
<221> CHAIN
<222> 138..146
<223> HA tag

<400> 3
Met Ile Val Arg Asn Leu Glu Glu Ala Arg Gln Thr Asp Arg Leu Val
1               5                   10                  15
Thr Ala Glu Asn Gly Asn Trp Asp Ser Thr Arg Leu Ser Leu Ala Glu
            20                  25                  30
Asp Gly Gly Asn Cys Ser Phe His Ile Thr Arg Ile Phe Glu Gly Thr
        35                  40                  45
Glu Thr His Ile His Tyr Lys His His Phe Glu Ala Val Tyr Cys Ile
    50                  55                  60
Glu Gly Glu Gly Glu Val Glu Thr Leu Ala Asp Gly Lys Ile Trp Pro
65                  70                  75                  80
Ile Lys Pro Gly Asp Ile Tyr Ile Leu Asp Gln His Asp Glu His Leu
                85                  90                  95
Leu Arg Ala Ser Lys Thr Met His Leu Ala Cys Val Phe Thr Pro Gly
            100                 105                 110
Leu Thr Gly Asn Glu Val His Arg Glu Asp Gly Ser Tyr Ala Pro Ala
        115                 120                 125
Asp Glu Ala Asp Asp Gln Lys Pro Leu Tyr Pro Tyr Asp Val Pro Asp
    130                 135                 140
Tyr Ala
145

<210> 4
<211> 340
<212> PRT
<213> Hansenula polymorpha (syn. Pichia angusta)


<220>
<221> CHAIN
<222> 1..1
<223> start methionine

<220>
<221> CHAIN
<222> 2..9
<223> His tag and flanking residues

<220>
<221> CHAIN
<222> 10..340
<223> EctD(2-332)

<400> 4
Met Lys His His His His His His Gln Ser Val Gln Thr Ser Ser Asn
1               5                   10                  15
Arg Pro Leu Pro Gln Ala Asn Leu His Ile Ala Thr Glu Thr Pro Glu
            20                  25                  30

Ala Asp Ser Arg Ile Arg Ser Ala Pro Arg Pro Gly Gln Asp Pro Tyr
            35                  40                  45
Pro Thr Arg Leu Ser Glu Pro Leu Asp Leu Pro Trp Leu Asn Arg Arg
    50                  55                  60
Glu Pro Val Val Lys Gly Glu Glu Ala Asp Gly Pro Leu Ser Ala Ala
65                  70                  75                  80
Gln Leu Asp Thr Phe Glu Arg Gln Gly Phe Ile Phe Glu Pro Asp Phe
                85                  90                  95
Leu Lys Gly Glu Glu Leu Glu Ala Leu Arg His Glu Leu Asn Ala Leu
            100                 105                 110
Leu Ala Arg Asp Asp Phe Arg Gly Arg Asp Phe Ala Ile Thr Glu Pro
        115                 120                 125
Gln Gly Asn Glu Ile Arg Ser Leu Phe Ala Val His Tyr Leu Ser Arg
    130                 135                 140
Val Phe Ser Arg Leu Ala Asn Asp Glu Arg Leu Met Gly Arg Ala Arg
145                 150                 155                 160
Gln Ile Leu Gly Gly Glu Pro Tyr Val His Gln Ser Arg Ile Asn Tyr
                165                 170                 175
Lys Pro Gly Phe Glu Gly Lys Gly Phe Asn Trp His Ser Asp Phe Glu
            180                 185                 190
Thr Trp His Ala Glu Asp Gly Met Pro Ala Met His Ala Val Ser Ala
        195                 200                 205
Ser Ile Val Leu Thr Asp Asn His Thr Phe Asn Gly Pro Leu Met Leu
    210                 215                 220
Val Pro Gly Ser His Arg Val Phe Val Pro Cys Leu Gly Glu Thr Pro
225                 230                 235                 240
Glu Asp His His Arg Gln Ser Leu Lys Thr Gln Glu Phe Gly Val Pro
                245                 250                 255
Ser Arg Gln Ala Leu Arg Glu Leu Ile Asp Arg His Gly Ile Glu Ala
            260                 265                 270
Pro Thr Gly Ala Ala Gly Gly Leu Leu Leu Phe Asp Cys Asn Thr Leu
        275                 280                 285
His Gly Ser Asn Ala Asn Met Ser Pro Asp Pro Arg Ser Asn Ala Phe
    290                 295                 300
Phe Val Tyr Asn Arg Arg Asp Asn Arg Cys Val Glu Pro Tyr Ala Ala
305                 310                 315                 320
Ser Lys Arg Arg Pro Arg Phe Leu Ala His Glu Pro Asp Glu Ala Trp
                325                 330                 335
Ser Pro Asp Gly
            340

<210> 5
<211> 8724
<212> DNA
<213> Hansenula polymorpha (syn. Pichia angusta)

<220>
<221> source
<222> 1..8724
<223> /mol_type="unassigned DNA"
      /organism="Hansenula polymorpha (syn. Pichia angusta)"

<220>
<221> promoter
<222> 7..1072
<223> /note="FMD promoter"

<220>
<221> misc_feature
<222> 1079..1112
<223> /note="loxP site"

<220>
<221> gene
<222> 1304..2215

<223> /note="Synthetic ADE1 gene; codons optimized for Hansenula
      polymorpha"

<220>
<221> misc_feature
<222> 2479..3510
<223> /note="HIS4 upstream region for homologous recombination of the
      integration cassette"

<220>
<221> gene
<222> 4603..5475
<223> /note="AmpR; Ampicillin resistence gene"

<220>
<221> misc_feature
<222> 6673..7770
<223> /note="HIS4 downstream region for homologous recombination of the
      integration cassette"

<220>
<221> terminator
<222> 7778..8105
<223> /note="MOX-T; teminator of the MOX gene"

<220>
<221> gene
<222> 8115..8723
<223> /note="EctA-myc fusion ORF (EctA coding region/myc tag coding
      region)"

<400> 5
aattcccgat gaaggcagag agcgcaagga ggcggtattt atagtgccat tcccctctct      60

gagagacccg gatggtagtc gagtgtatcg gagacagctt gatgtagact ccgtgcctgc     120

cggctcctct tattggcgga caccagtgag acaccccgga acttgctgtt tttctgcaaa     180

atccggggtg accagtggga gcctatttgc acacacgagc gggacacccc actctggtga     240

agagtgccaa agtcattctt tttcccgttg cggggcagcc gattgcatgt tttaggaaaa     300

tattaccttt gctacaccct gtcagattta ccctccacac atatatattc cgtcacctcc     360

agggactatt attcgtcgtt gcgccgccag cggaagatat ccagaagctg ttttccgaga     420

gactcggttg gcgcctggta tatttgatgg atgtcgcgct gcctcacgtc ccggtaccca     480

ggaacgcggt gggatctcgg gcccatcgaa gactgtgctc cagactgctc gcccagcagg     540

tgtttcttga tcgccgcctc taaattgtcc gcgcatcgcc ggtaacattt ttccagctcg     600

gagtttgcgt ttagatacag tttctgcgat gccaaaggag cctgcagatt ataacctcgg     660

atgctgtcat tcagcgcttt taatttgacc tccagatagt tgctgtattt ctgttcccat     720

tggctgctgc gcagcttcgt ataactcgag ttattgttgc gctctgcctc ggcgtactgg     780

ctcatgatct ggatcttgtc cgtgtcgctt ttcttcgagt gtttctcgca aacgatgtgc     840

acggcctgca gtgtccaatc ggagtcgagc tggcgccgaa actggcggat ctgagcctcc     900

acactgccct gtttctctat ccacggcgga accgcctcct gccgtttcag aatgttgttc     960

aagtggtact ctgtgcggtc aatgaaggcg ttattgccgg tgaaatcttt gggaagcggt    1020

tttcctcggg gaagattacg aaattccccg cgtcgttgcg cttcctggat ctcgatcgat 1080

aacttcgtat agcatacatt atacgaagtt atatgcgcac tctagattcc ttgcgtacaa 1140

acacctttcc gtcagcgaca cgccttactt tgcgcacaca tccgaacgag cctcgtccga 1200

tgagctccaa cacttggaaa tcgtccatcg aatagtatat ttacaaactt ccggatacag 1260

aaaataatta aattatttgg gacagcttgg tttatttcaa cacttaagcc caggttttac 1320

cggtcaaagc ctcgtaagcc tctttgtact tagcggcggt cttagcagca atctcctcgg 1380

tcatagacac accctcttta ccgttcagct ggtgagaagt caaccagtct ctcaggaact 1440

gcttgtcgaa agactcctga gatcttccca actcgtactt cttggcgtcc caaaatctcg 1500

aagagtctgg ggtcaaaacc tcgtcaacca gaaccagctc gttgttctcg ttcagtccga 1560

actcaaactt ggtgtcggcc aggataatac ctctcgacag agcgtagtct ctagccttag 1620

agtacagggc aatagccttc tgggcaacct cagcacacaa ctcctctcca accaactgag 1680

cagcctgctc tggagaaatg ttctcgtcgt gctgtccctg ctctgctttg gtagatgggg 1740

tgaagattgg cttctcgaaa gcctcagact cctgcaatcc agacttaact ggcagtccgt 1800

gaacggtaga cgacttcttg tactctttcc aggcagaacc ggtgatgtaa cctctcacaa 1860

tggcctccaa tggaaccagc ttgtgcttct tcaccagcag agatctacca gccaactgag 1920

ccttgtactt tggctcagac aactgctctg gcaacttggc aaagatgtcc ttgtcagagg 1980

aagcaaccag gtggtttgga ataacgtcag acagcaggtt gaaccagaac acagacagct 2040

gggtcagaat ctttcccttg tccttgatgc cgttctccat aatcacgtcg taggcagaga 2100

ttctgtcggt ggcaacaaac agcagcatgt tgtcgtccac ctggtagatg tctctcactt 2160

tacctctggc aaccaatggc agaataccgt ccaggttagt ctgggtcacg gtcatatgtg 2220

taataataat taagtaaaat tagtttagta aattaattga ctcgtgaggg tcgatcgacg 2280

cgatattggc tctccttttt atacgagttt ttgtcaactt tcctctgcta tgctcaatcc 2340

agtcaccgtc cacaaagact tctttgggtt tcctggggcc ttggttctga ccatcagtct 2400

acctttggtg gttgttttcc tgtatgcggc gtcgacggcg cgccataact tcgtatagca 2460

tacattatac gaagttatcc tgcaggttat gaatttacgg tgagatgtga gtcaagctga 2520

aaaatatctt gatttttttt tttttgacat gccacgactt tggtagcatg ggacgatata 2580

tcactatata cactgtgagt cagttattga acaaaaactg catatcggcc gccgatagcc 2640

tgctagccgc gttctggtcc tgatccagcg tggccttgac catgtcagcc ttcttctgct 2700

gcagctcaat gatccgcgac tcgatgctgt cctcgatgac caacttcaca attctgatag 2760

gccggtgctg tccaatccgg tgaattctgt ctgcggcctg acccgtgtcc aaagcagggt 2820

tccaccaact atccaggata aatacttgat tggcctcgac aagattcaga gccacacctc 2880

cggccttcaa agacaccaaa aatacctcca ccgacggatt ggtcataaag tgctggatca 2940

cagagtcacg ctggatcggg gtcatggatc cctgcaattt cgcaatggaa aaacccgccc 3000

ttttgagtcg ccactccacc aggtccagca tcgacgtgaa ctgcgaaaag acaatgcttt 3060

taacagtttg tctgtccgaa cgggacttgt acaactcttc cattagggcc tcaattttag 3120

tggaagactt ccattcgcct cccatgtcta ttcggttcac aatagagccc ttctcaacga 3180

cctcctggtt catctccagc gctggtgcct ccagatcaat actcagccca atgtggcaca 3240

ctggacactc gagacgctta tcgtcgccgt tgaacgactc cacgtactcc gtgatacaga 3300

tccggcagaa cttgtggtga catttggact ctatcggctc ctctgcttcg tcatcacaca 3360

gctggcagac aatggccccg ctgttgagga gatccctgga tgctttctta aatcgcttga 3420

gaaccagatc cggatgatcc gcaagctgtc tcatacgcgt gattagggta aagatgttgg 3480

catagttgtt cagaaccaca ccttgtgcag cggccgcgca tgcggtctct agcttggcac 3540

tggccgtcgt tttacaacgt cgtgactggg aaaaccctgg cgttacccaa cttaatcgcc 3600

ttgcagcaca tccccctttc gccagctggc gtaatagcga agaggcccgc accgatcgcc 3660

cttcccaaca gttgcgcagc ctgaatggcg aatggcgcct gatgcggtat tttctcctta 3720

cgcatctgtg cggtatttca caccgcatat gctcgtcaaa gcaaccatag tacgcgccct 3780

gtagcggcgc attaagcgcg gcgggtgtgg tggttacgcg cagcgtgacc gctacacttg 3840

ccagcgccct agcgcccgct cctttcgctt tcttcccttc ctttctcgcc acgttcgccg 3900

gctttccccg tcaagctcta aatcgggggc tccctttagg gttccgattt agtgctttac 3960

ggcacctcga ccccaaaaaa cttgatttgg gtgatggttc acgtagtggg ccatcgccct 4020

gatagacggt ttttcgccct ttgacgttgg agtccacgtt ctttaatagt ggactcttgt 4080

tccaaactgg aacaacactc aaccctatct cgggctattc ttttgattta taagggattt 4140

tgccgatttc ggcctattgg ttaaaaaatg agctgattta acaaaaattt aacgcgaatt 4200

ttaacaaaat attaacgttt acaattttat ggtgcactct cagtacaatc tgctctgatg 4260

ccgcatagtt aagccagccc cgacacccgc caacacccgc tgacgcgccc tgacgggctt 4320

gtctgctccc ggcatccgct tacagacaag ctgtgaccgt ctccgggagc tgcatgtgtc 4380

agaggttttc accgtcatca ccgaaacgcg cgaggacgaa agggcctcgt gatacgccta 4440

tttttatagg ttaatgtcat gataataatg gtttcttaga cgtcaggtgg cacttttcgg 4500

ggaaatgtgc gcggaacccc tatttgttta tttttctaaa tacattcaaa tatgtatccg 4560

ctcatgagac aataaccctg ataaatgctt caataatatt gaaaaaggaa gagtatgagt 4620

attcaacatt tccgtgtcgc ccttattccc ttttttgcgg cattttgcct tcctgttttt 4680

gctcacccag aaacgctggt gaaagtaaaa gatgctgaag atcagttggg tgcacgagtg 4740

ggttacatcg aactggatct caacagcggt aagatccttg agagttttcg ccccgaagaa 4800

cgttttccaa tgatgagcac ttttaaagtt ctgctatgtg gcgcggtatt atcccgtatt 4860

gacgccgggc aagagcaact cggtcgccgc atacactatt ctcagaatga cttggttgag 4920

tactcaccag tcacagaaaa gcatcttacg gatggcatga cagtaagaga attatgcagt 4980
gctgccataa ccatgagtga taacactgcg gccaacttac ttctgacaac gatcggagga 5040
ccgaaggagc taaccgcttt tttgcacaac atgggggatc atgtaactcg ccttgatcgt 5100
tgggaaccgg agctgaatga agccatacca aacgacgagc gtgacaccac gatgcctgta 5160
gcaatggcaa caacgttgcg caaactatta actggcgaac tacttactct agcttcccgg 5220
caacaattaa tagactggat ggaggcggat aaagttgcag gaccacttct gcgctcggcc 5280
cttccggctg gctggtttat tgctgataaa tctggagccg gtgagcgtgg gtctcgcggt 5340
atcattgcag cactggggcc agatggtaag ccctcccgta tcgtagttat ctacacgacg 5400
gggagtcagg caactatgga tgaacgaaat agacagatcg ctgagatagg tgcctcactg 5460
attaagcatt ggtaactgtc agaccaagtt tactcatata tactttagat tgatttaaaa 5520
cttcattttt aatttaaaag gatctaggtg aagatccttt ttgataatct catgaccaaa 5580
atcccttaac gtgagttttc gttccactga gcgtcagacc ccgtagaaaa gatcaaagga 5640
tcttcttgag atcctttttt tctgcgcgta atctgctgct tgcaaacaaa aaaaccaccg 5700
ctaccagcgg tggtttgttt gccggatcaa gagctaccaa ctctttttcc gaaggtaact 5760
ggcttcagca gagcgcagat accaaatact gtccttctag tgtagccgta gttaggccac 5820
cacttcaaga actctgtagc accgcctaca tacctcgctc tgctaatcct gttaccagtg 5880
gctgctgcca gtggcgataa gtcgtgtctt accgggttgg actcaagacg atagttaccg 5940
gataaggcgc agcggtcggg ctgaacgggg ggttcgtgca cacagcccag cttggagcga 6000
acgacctaca ccgaactgag atacctacag cgtgagcatt gagaaagcgc cacgcttccc 6060
gaagggagaa aggcggacag gtatccggta agcggcaggg tcggaacagg agagcgcacg 6120
agggagcttc cagggggaaa cgcctggtat ctttatagtc ctgtcgggtt tcgccacctc 6180
tgacttgagc gtcgattttt gtgatgctcg tcaggggggc ggagcctatg gaaaaacgcc 6240
agcaacgcgg cctttttacg gttcctggcc ttttgctggc cttttgctca catgttcttt 6300
cctgcgttat cccctgattc tgtggataac cgtattaccg cctttgagtg agctgatacc 6360
gctcgccgca gccgaacgac cgagcgcagc gagtcagtga gcgaggaagc ggaagagcgc 6420
ccaatacgca aaccgcctct ccccgcgcgt tggccgattc attaatgcag ctggcacgac 6480
aggtttcccg actggaaagc gggcagtgag cgcaacgcaa ttaatgtgag ttagctcact 6540
cattaggcac cccaggcttt acactttatg cttccggctc gtatgttgtg tggaattgtg 6600
agcggataac aatttcacac aggaaacagc tatgaccatg attacgaatt gagctcggta 6660
cccggcctcg aggtttaaac gaggattctg gccctacaac tgcaaagaag ctgtgcgagg 6720
gcacgctcgt cgtttgggcc agccaatgtg ggcaacaatc tcgccaacct ggacgttttg 6780
ggctcttctc cagtgcctga gaacaatatc gagatgatct ctaaggactc gattatcttc 6840

tccaacatga aggttgtgcg atcgcccaac aggcaaggga ctcttgttgc tgctctgttg 6900

ttggataacc agatatttga gctcaacaca aagtccatca gcaaaaaagg cccattggat 6960

ttgacaacta ctgagctctt tcccttgtta gaggcgctct acccaaaacc cgagctgttt 7020

gtgctgggtc ttgggttcag caaccggatg ttacacccag acacgaaaag acgcttttcc 7080

gagttgggga tcatgctcga agtaggcgac accaagagcg cagtgctcag ctacgatctt 7140

ctggctacgg aacgttctcc aaaacaggta ggggcactca tatttccatc ggtgttctga 7200

aatatgcgcg aaacaactat ttaagggctc tgaccaggaa ttgcgtgctg aacacatagg 7260

agtaggcagc aagggtaagg acgctcagac tgttgaacac gaggctgatt ccatggtact 7320

tgccgaactt cttggtcaaa gctgcgattt tttcgttggt ctcgggagtt ctctcgctct 7380

ctggcagttc ctcgagcgca aagcgttcag tcttcagccg gttgcacact ggcatcagcc 7440

acaagtaatt ggtgagacct gagagcgaag aggccgccag caatgcaatt ccgacggttg 7500

ggatcgtgta tggtgcagag agaccgacca ccacggggcc gatcgactgc gtcaggaagt 7560

ggatcgggaa cagcttggat tgcaggaggc caaactcctt gcggggcagg accttgaacg 7620

ctctgacaga ggacacaaat gagtggaacg aagtggctcc gaaaacatac gagtaagcaa 7680

gcaggtgcac agcagtcagc atggtaaaaa ttaattgcac ttattattta tttggtcgtg 7740

gctttattta atcagtgata attgtcacgt taattaacaa tctccggaat ggtgatctga 7800

tcgttcctga aaacctcgac attggctccc tcctgacaca ggtactcgta caggttccag 7860

gtaaacgagt cgtagttgtc gatcatgaca acgttcttag aagcggccgg cattttgaag 7920

gtgactaata gcctaagaaa atatttaatt taattttcat taaattttcc tatactcgct 7980

atttcagctt ttcatctcat cacttcataa acgatataaa ccagaaaaag aactattttc 8040

aaacacgctt ctcaaaagcg gtatgtcctt ccacgtctcc ttagaatctg gcaagtccgc 8100

gagggggatc cttactacag gtcttcctca gaaatcagct tctgctcaat ctggtcggtc 8160

tggaatggac caattctaac caagttctct gggtcgtgct caccacccaa ctggtcggta 8220

gaaaagtact ctctagagtt caatggggcc tgccatctgt cagccaatct tctaaacagg 8280

ccccaagaag cctggttgtc tggagtaatg gtagtctcca agtggtgaac ctcagccatc 8340

tctggtctgg tcataacggc ctcaaccaat cttctagcca gaccagtacc tctagccttc 8400

tcaccaacag caacctgcca caaaaagtag gtgtccggag cgttagactt cacgtaacca 8460

gacacgaaac caacgatctc accttcctcg ttggtagcaa cagcacaaga gtctctgaac 8520

tgggtagcca gcaacaggta agcgtaagca gagttaacgt ccagcggtgg acaagactta 8580

accaactcgt agatacccca gccgtcgtct ggagatggtt ttctgatgaa cagtggagaa 8640

gcctcgtgac caacaacagc gtcagcaaca gatggcttag ccaagtcagc agatggagtg 8700

aatggctcgg tggtagcgtt catg 8724

<210> 6
<211> 9360
<212> DNA
<213> Hansenula polymorpha (syn. Pichia angusta)

<220>
<221> source
<222> 1..9360
<223> /mol_type="unassigned DNA"
      /organism="Hansenula polymorpha (syn. Pichia angusta)"

<220>
<221> gene
<222> 190..1062
<223> /note="AmpR; Ampicillin resistence gene"

<220>
<221> misc_feature
<222> 2260..3360
<223> /note="LEU2 downstream region for homologous recombination of the
      integration cassette"

<220>
<221> terminator
<222> 3368..3695
<223> /note="MOX-T; teminator of the MOX gene"

<220>
<221> gene
<222> 3705..5003
<223> /note="EctB-T7 fusion ORF (EctB coding region/T7 tag coding
      region)"

<220>
<221> promoter
<222> 5011..6076
<223> /note="FMD promoter"

<220>
<221> misc_feature
<222> 6083..6116
<223> /note="loxP site"

<220>
<221> gene
<222> 6308..7219
<223> /note="Synthetic ADE1 gene; codons optimized for Hansenula
      polymorpha"

<220>
<221> misc_feature
<222> 7449..7482
<223> /note="loxP site"

<220>
<221> misc_feature
<222> 7483..8457
<223> /note="LEU2 upstream region for homologous recombination of the
      integration cassette"

<400> 6
ggacgaaagg gcctcgtgat acgcctattt ttataggtta atgtcatgat aataatggtt      60

tcttagacgt caggtggcac ttttcgggga aatgtgcgcg gaacccctat ttgtttattt     120

ttctaaatac attcaaatat gtatccgctc atgagacaat aaccctgata aatgcttcaa 180
taatattgaa aaaggaagag tatgagtatt caacatttcc gtgtcgccct tattcccttt 240
tttgcggcat tttgccttcc tgtttttgct cacccagaaa cgctggtgaa agtaaaagat 300
gctgaagatc agttgggtgc acgagtgggt tacatcgaac tggatctcaa cagcggtaag 360
atccttgaga gttttcgccc cgaagaacgt tttccaatga tgagcacttt taaagttctg 420
ctatgtggcg cggtattatc ccgtattgac gccgggcaag agcaactcgg tcgccgcata 480
cactattctc agaatgactt ggttgagtac tcaccagtca cagaaaagca tcttacggat 540
ggcatgacag taagagaatt atgcagtgct gccataacca tgagtgataa cactgcggcc 600
aacttacttc tgacaacgat cggaggaccg aaggagctaa ccgctttttt gcacaacatg 660
ggggatcatg taactcgcct tgatcgttgg gaaccggagc tgaatgaagc cataccaaac 720
gacgagcgtg acaccacgat gcctgtagca atggcaacaa cgttgcgcaa actattaact 780
ggcgaactac ttactctagc ttcccggcaa caattaatag actggatgga ggcggataaa 840
gttgcaggac cacttctgcg ctcggccctt ccggctggct ggtttattgc tgataaatct 900
ggagccggtg agcgtgggtc tcgcggtatc attgcagcac tggggccaga tggtaagccc 960
tcccgtatcg tagttatcta cacgacgggg agtcaggcaa ctatggatga acgaaataga 1020
cagatcgctg agataggtgc ctcactgatt aagcattggt aactgtcaga ccaagtttac 1080
tcatatatac tttagattga tttaaaactt catttttaat ttaaaaggat ctaggtgaag 1140
atcctttttg ataatctcat gaccaaaatc ccttaacgtg agttttcgtt ccactgagcg 1200
tcagaccccg tagaaaagat caaaggatct tcttgagatc ctttttttct gcgcgtaatc 1260
tgctgcttgc aaacaaaaaa accaccgcta ccagcggtgg tttgtttgcc ggatcaagag 1320
ctaccaactc tttttccgaa ggtaactggc ttcagcagag cgcagatacc aaatactgtc 1380
cttctagtgt agccgtagtt aggccaccac ttcaagaact ctgtagcacc gcctacatac 1440
ctcgctctgc taatcctgtt accagtggct gctgccagtg gcgataagtc gtgtcttacc 1500
gggttggact caagacgata gttaccggat aaggcgcagc ggtcgggctg aacggggggt 1560
tcgtgcacac agcccagctt ggagcgaacg acctacaccg aactgagata cctacagcgt 1620
gagcattgag aaagcgccac gcttcccgaa gggagaaagg cggacaggta tccggtaagc 1680
ggcagggtcg gaacaggaga gcgcacgagg gagcttccag ggggaaacgc ctggtatctt 1740
tatagtcctg tcgggtttcg ccacctctga cttgagcgtc gatttttgtg atgctcgtca 1800
ggggggcgga gcctatggaa aaacgccagc aacgcggcct ttttacggtt cctggccttt 1860
tgctggcctt ttgctcacat gttctttcct gcgttatccc ctgattctgt ggataaccgt 1920
attaccgcct ttgagtgagc tgataccgct cgccgcagcc gaacgaccga gcgcagcgag 1980
tcagtgagcg aggaagcgga agagcgccca atacgcaaac cgcctctccc cgcgcgttgg 2040
ccgattcatt aatgcagctg gcacgacagg tttcccgact ggaaagcggg cagtgagcgc 2100

aacgcaatta atgtgagtta gctcactcat taggcacccc aggctttaca ctttatgctt 2160

ccggctcgta tgttgtgtgg aattgtgagc ggataacaat ttcacacagg aaacagctat 2220

gaccatgatt acgaattgag ctcggtaccc ggcctcgagg tttaaaccga ttcctgctgc 2280

cgcacctcgc gagccagctg gaggctgacg gggagacgac gccaaaatgc tggatgcgcg 2340

cttttcgctg aggttgtctc tgtcgtcgcg ctggcgtctg cgccaaaaca ggaacaggag 2400

caggagtccc aagagcagca aagctccgag gatcgacccg acagcgattc cggccttcgc 2460

gcccttgctg agccccgtgg acctgttgtt ggcgctcgag gcacttgagg acgacgcggc 2520

agccgacgcc gaggtggctg tagcgacgga ctgcgtgatg tagatgatcg aggtctgcat 2580

gtttccaggt gaaacggacg tagttatccg cactgattcg gatgagggtg tggacgaaga 2640

ggtggttgaa gacgacgacg agctctggga gaaagaaaag gacgaaatcg agcttgttgt 2700

agtcgagcta gcggacagac ttgaggacgt tggagacgag tctgtggaag acatggtgga 2760

gctctgtggg gaggaacttt gcgtggagga agacgatgtt gaggagctgg acagactgga 2820

actggaggag gaggtggtgg aggtggagga ggtggaagtg gcatcgtctg tggacgagtg 2880

cgacgactgc gccgacgaac tgtcgtcgtc gttttcctca gactcgtcaa catagacgga 2940

ataagagtcg ctaccaccac acttctccat gggataacca gtgcaggacg ttgtgcacga 3000

gccgtcgggg ttgtccgaag gtacgtcgtc tccgcaataa cagtcctggc cgttgatcaa 3060

tgcagcaact gcgtgtcccg aacactgctc cgtgcaatgg ctggagctct gccatgtgta 3120

cgagtccttc ttggtcaagc cggaaatggc atccgagctg tagcaaccta aatacgtaaa 3180

gtctgctagg acttgggtga tcacgaatac gacaccgaat ctcatgttga catggaaaag 3240

ctaaaaaata aacaatagag cgtcgcgtcg aaaaaaataa aatctgtgag gcgtgcgtgt 3300

ttaggatttt tgggattatt taggaattat tcgagatcct actttttttt ctcccttatt 3360

taattaacaa tctccggaat ggtgatctga tcgttcctga aaacctcgac attggctccc 3420

tcctgacaca ggtactcgta caggttccag gtaaacgagt cgtagttgtc gatcatgaca 3480

acgttcttag aagcggccgg cattttgaag gtgactaata gcctaagaaa atatttaatt 3540

taattttcat taaattttcc tatactcgct atttcagctt ttcatctcat cacttcataa 3600

acgatataaa ccagaaaaag aactattttc aaacacgctt ctcaaaagcg gtatgtcctt 3660

ccacgtctcc ttagaatctg gcaagtccgc gagggggatc cttattagcc catctgctgg 3720

ccaccggtca tcgaggccat cgagaaggcc tgcttggtcg aggtttccag gatgtccagg 3780

ccttccacca ggtcctcgtc tgggatggtc agtgggcaca ggcacttcac cacctcgccg 3840

tcctggcccg aggtctcgat gatcaggccg ttctcgaagg cctggtgggt gatcttgtcg 3900

gcgatgtcgc ccgagcccac gtcgatgcct ctcatcaggc ctctgcctct ttccgaagcc 3960

tcgatgccgt tctccgacag ccaagcggcg atcttgccga atctctcttc cacgattctg 4020

gcctttctct gcacgtctct ctcgaaggtg tcgtccgacc agtactttct catggctgcg 4080

gcagcggtgg cgaaggccag gttgaagcct ctgaaggtgc cgttgtactg gcctggcttc 4140

cacttgtcca gctctggtct catcagcacg tgggcgaatg gcaggccgta gcccgacagc 4200

gacttcgagt tggtcacgat gtctggggtg atgccggcgt gctcgaacga gaagaacttg 4260

ccggttctgc cgcagccggc ctggatatcg tcgatgatca gcaggatgtc gttggctctg 4320

cagatcgatt ccagtctctt cagccattcc agaccggcca cgttgatgcc gccctcgccc 4380

tgcacggttt ccacgatcac ggcggctggc acgtccaggc cacccgactt gtcgcccagc 4440

agcttctcga agtagtccag ggtgtcggtc gacgagccca ggtagccgtc gaatggcatg 4500

aacgaggcag cctgggttgg gacgcctccg gtggcctctc tgaactttct gttgccggtg 4560

gtagccagag cgcccatggt cacaccgtgg aagccgttgg tgaacgacac gatgttgtgt 4620

ctgcccttgg ccactctggc cagtctgatg gcagcttcca cggcgttggt gccggttggg 4680

cctggcaggt gcaccttgta gtccaggcct cttggcttca ggatcacctc ttccagggtt 4740

tccaggtagt ctctcttggc ggcggtccaa aagtccaggc cgtgcacgat gccgtccgag 4800

tcgatgtagt ccagcagggc ctgcttcagg tgtgggttgt tgtggccgta gttcagggtg 4860

cctgcgccag ccagaaagtc gatgtactct ctgccttcct cgtcggtcag tctggcgttt 4920

ctggccttgg tgaacaccac tgggaacgat ctcgagtagg ttctcacgtc cgattccatt 4980

ctctccagga tctgggtctg catgaattcc cgatgaaggc agagagcgca aggaggcggt 5040

atttatagtg ccattcccct ctctgagaga cccggatggt agtcgagtgt atcggagaca 5100

gcttgatgta gactccgtgc ctgccggctc ctcttattgg cggacaccag tgagacaccc 5160

cggaacttgc tgtttttctg caaaatccgg ggtgaccagt gggagcctat ttgcacacac 5220

gagcgggaca ccccactctg gtgaagagtg ccaaagtcat tctttttccc gttgcggggc 5280

agccgattgc atgttttagg aaaatattac ctttgctaca ccctgtcaga tttaccctcc 5340

acacatatat attccgtcac ctccagggac tattattcgt cgttgcgccg ccagcggaag 5400

atatccagaa gctgttttcc gagagactcg gttggcgcct ggtatatttg atggatgtcg 5460

cgctgcctca cgtcccggta cccaggaacg cggtgggatc tcgggcccat cgaagactgt 5520

gctccagact gctcgcccag caggtgtttc ttgatcgccg cctctaaatt gtccgcgcat 5580

cgccggtaac atttttccag ctcggagttt gcgtttagat acagtttctg cgatgccaaa 5640

ggagcctgca gattataacc tcggatgctg tcattcagcg cttttaattt gacctccaga 5700

tagttgctgt atttctgttc ccattggctg ctgcgcagct tcgtataact cgagttattg 5760

ttgcgctctg cctcggcgta ctggctcatg atctggatct tgtccgtgtc gcttttcttc 5820

gagtgtttct cgcaaacgat gtgcacggcc tgcagtgtcc aatcggagtc gagctggcgc 5880

cgaaactggc ggatctgagc ctccacactg ccctgtttct ctatccacgg cggaaccgcc 5940

tcctgccgtt tcagaatgtt gttcaagtgg tactctgtgc ggtcaatgaa ggcgttattg 6000

ccggtgaaat ctttgggaag cggttttcct cggggaagat tacgaaattc cccgcgtcgt 6060

tgcgcttcct ggatctcgat cgataacttc gtatagcata cattatacga agttatatgc 6120

gcactctaga ttccttgcgt acaaacacct ttccgtcagc gacacgcctt actttgcgca 6180

cacatccgaa cgagcctcgt ccgatgagct ccaacacttg gaaatcgtcc atcgaatagt 6240

atatttacaa acttccggat acagaaaata attaaattat ttgggacagc ttggtttatt 6300

tcaacactta agcccaggtt ttaccggtca aagcctcgta agcctctttg tacttagcgg 6360

cggtcttagc agcaatctcc tcggtcatag acacaccctc tttaccgttc agctggtgag 6420

aagtcaacca gtctctcagg aactgcttgt cgaaagactc ctgagatctt cccaactcgt 6480

acttcttggc gtcccaaaat ctcgaagagt ctggggtcaa aacctcgtca accagaacca 6540

gctcgttgtt ctcgttcagt ccgaactcaa acttggtgtc ggccaggata atacctctcg 6600

acagagcgta gtctctagcc ttagagtaca gggcaatagc cttctgggca acctcagcac 6660

acaactcctc tccaaccaac tgagcagcct gctctggaga aatgttctcg tcgtgctgtc 6720

cctgctctgc tttggtagat ggggtgaaga ttggcttctc gaaagcctca gactcctgca 6780

atccagactt aactggcagt ccgtgaacgg tagacgactt cttgtactct ttccaggcag 6840

aaccggtgat gtaacctctc acaatggcct ccaatggaac cagcttgtgc ttcttcacca 6900

gcagagatct accagccaac tgagccttgt actttggctc agacaactgc tctggcaact 6960

tggcaaagat gtccttgtca gaggaagcaa ccaggtggtt tggaataacg tcagacagca 7020

ggttgaacca gaacacagac agctgggtca gaatctttcc cttgtccttg atgccgttct 7080

ccataatcac gtcgtaggca gagattctgt cggtggcaac aaacagcagc atgttgtcgt 7140

ccacctggta gatgtctctc actttacctc tggcaaccaa tggcagaata ccgtccaggt 7200

tagtctgggt cacggtcata tgtgtaataa taattaagta aaattagttt agtaaattaa 7260

ttgactcgtg agggtcgatc gacgcgatat tggctctcct ttttatacga gtttttgtca 7320

actttcctct gctatgctca atccagtcac cgtccacaaa gacttctttg ggtttcctgg 7380

ggccttggtt ctgaccatca gtctaccttt ggtggttgtt ttcctgtatg cggcgtcgac 7440

ggcgcgccat aacttcgtat agcatacatt atacgaagtt atcctgcagg gattgcaaaa 7500

tgatgcaact attttgcgcc ggtaccggga aaaattgaaa aaccatccac ttactcattc 7560

ctgtcttttt atttcgtatt accaaaccgc ttacgtactc acccactcag atctgctcag 7620

acacaaactg gtcgatgtcg acctcctcga gtggagcagt ggccgcattg ttcacctttg 7680

gcttctcctc tctgagctca agacgtttca gcttactaag agggccccag ccgtgcacag 7740

gtgggctaag tttgaatgga tccaaaaact tgacacactg tttgaacgag tcgcccaatg 7800

tggcaatctc gtggatgatg tcttccacac agatgatgcc gtagtctcca agctggtctt 7860

caatcaaatt gttgtcatcc aagttcacct tcttggttgt tccgtcctcg acgatactag 7920

ccgtggcacg tttctggatc aagtttcgga cagtagcaag cgatggagtg ccgatggcga 7980

tgtacggact ggtcagtctg aggagaggtt tgacggcagc gttcaacttg atgaaagttc 8040

ctgtgtacac gtgctctaga cgcagcagtt caagcacttt tctggctttg gagggcactt 8100

tggcaccatg cgggccgggg atacggacca caaacaaaag acgctcatcg gactcttccg 8160

gcacagcaga gctccgttca tgctgagaca cgcgcttcac acggtactgt tctctttctg 8220

atgcgcgatg tttagccact agggtttctg ctctgataaa ccttgccttt ctttgctgtt 8280

gtttgcgttt cttctgttcc tcgagcttct tacgtgcctg ctcctgtttc tctagacgga 8340

gtctgtcggc gttcttccgc ttcttgagca acacctctgg gttggtgttc agtttagtcg 8400

attcagccat gataaaaaaa aaaaaaattg tgcagaaaaa tttatttggc agtgttgcgg 8460

ccgcgcatgc ggtctctagc ttggcactgg ccgtcgtttt acaacgtcgt gactgggaaa 8520

accctggcgt tacccaactt aatcgccttg cagcacatcc ccctttcgcc agctggcgta 8580

atagcgaaga ggcccgcacc gatcgccctt cccaacagtt gcgcagcctg aatggcgaat 8640

ggcgcctgat gcggtatttt ctccttacgc atctgtgcgg tatttcacac cgcatatgct 8700

cgtcaaagca accatagtac gcgccctgta gcggcgcatt aagcgcggcg ggtgtggtgg 8760

ttacgcgcag cgtgaccgct acacttgcca gcgccctagc gcccgctcct ttcgctttct 8820

tcccttcctt tctcgccacg ttcgccggct ttccccgtca agctctaaat cgggggctcc 8880

ctttagggtt ccgatttagt gctttacggc acctcgaccc caaaaaactt gatttgggtg 8940

atggttcacg tagtgggcca tcgccctgat agacggtttt tcgccctttg acgttggagt 9000

ccacgttctt taatagtgga ctcttgttcc aaactggaac aacactcaac cctatctcgg 9060

gctattcttt tgatttataa gggattttgc cgatttcggc ctattggtta aaaaatgagc 9120

tgatttaaca aaaatttaac gcgaatttta acaaaatatt aacgtttaca attttatggt 9180

gcactctcag tacaatctgc tctgatgccg catagttaag ccagccccga cacccgccaa 9240

cacccgctga cgcgccctga cgggcttgtc tgctcccggc atccgcttac agacaagctg 9300

tgaccgtctc cgggagctgc atgtgtcaga ggttttcacc gtcatcaccg aaacgcgcga 9360

<210> 7
<211> 10130
<212> DNA
<213> Hansenula polymorpha (syn. Pichia angusta)

<220>
<221> source
<222> 1..10130
<223> /mol_type="unassigned DNA"
      /organism="Hansenula polymorpha (syn. Pichia angusta)"

<220>
<221> gene
<222> 190..1062
<223> /note="AmpR; Ampicillin resistence gene"

<220>
<221> misc_feature
<222> 2260..3270
<223> /note="ODC1 downstream region for homologous recombination of the
      integration cassette"

<220>
<221> terminator
<222> 3278..3605
<223> /note="MOX-T; teminator of the MOX gene"

<220>
<221> gene
<222> 3615..4055
<223> /note="EctC-HA fusion ORF (EctC coding region/HA tag coding
      region)"

<220>
<221> promoter
<222> 4063..5128
<223> /note="FMD promoter"

<220>
<221> misc_feature
<222> 5127..8160
<223> /note="loxP site"

<220>
<221> misc_feature
<222> 5135..5168
<223> /note="loxP site"

<220>
<221> gene
<222> 5168..7972
<223> /note="HIS4 of H. polymorpha"

<220>
<221> misc_feature
<222> 8161..9227
<223> /note="ODC1 upstream region for homologous recombination of the
      integration cassette"

<400> 7
ggacgaaagg gcctcgtgat acgcctattt ttataggtta atgtcatgat aataatggtt      60

tcttagacgt caggtggcac ttttcgggga aatgtgcgcg gaacccctat ttgtttattt     120

ttctaaatac attcaaatat gtatccgctc atgagacaat aaccctgata aatgcttcaa     180

taatattgaa aaaggaagag tatgagtatt caacatttcc gtgtcgccct tattcccttt     240

tttgcggcat tttgccttcc tgtttttgct cacccagaaa cgctggtgaa agtaaaagat     300

gctgaagatc agttgggtgc acgagtgggt tacatcgaac tggatctcaa cagcggtaag     360

atccttgaga gttttcgccc cgaagaacgt tttccaatga tgagcacttt taaagttctg     420

ctatgtggcg cggtattatc ccgtattgac gccgggcaag agcaactcgg tcgccgcata     480

cactattctc agaatgactt ggttgagtac tcaccagtca cagaaaagca tcttacggat     540

ggcatgacag taagagaatt atgcagtgct gccataacca tgagtgataa cactgcggcc     600

aacttacttc tgacaacgat cggaggaccg aaggagctaa ccgctttttt gcacaacatg     660

ggggatcatg taactcgcct tgatcgttgg gaaccggagc tgaatgaagc cataccaaac 720

gacgagcgtg acaccacgat gcctgtagca atggcaacaa cgttgcgcaa actattaact 780

ggcgaactac ttactctagc ttcccggcaa caattaatag actggatgga ggcggataaa 840

gttgcaggac cacttctgcg ctcggccctt ccggctggct ggtttattgc tgataaatct 900

ggagccggtg agcgtgggtc tcgcggtatc attgcagcac tggggccaga tggtaagccc 960

tcccgtatcg tagttatcta cacgacgggg agtcaggcaa ctatggatga acgaaataga 1020

cagatcgctg agataggtgc ctcactgatt aagcattggt aactgtcaga ccaagtttac 1080

tcatatatac tttagattga tttaaaactt catttttaat ttaaaaggat ctaggtgaag 1140

atcctttttg ataatctcat gaccaaaatc ccttaacgtg agttttcgtt ccactgagcg 1200

tcagaccccg tagaaaagat caaaggatct tcttgagatc ctttttttct gcgcgtaatc 1260

tgctgcttgc aaacaaaaaa accaccgcta ccagcggtgg tttgtttgcc ggatcaagag 1320

ctaccaactc tttttccgaa ggtaactggc ttcagcagag cgcagatacc aaatactgtc 1380

cttctagtgt agccgtagtt aggccaccac ttcaagaact ctgtagcacc gcctacatac 1440

ctcgctctgc taatcctgtt accagtggct gctgccagtg gcgataagtc gtgtcttacc 1500

gggttggact caagacgata gttaccggat aaggcgcagc ggtcgggctg aacggggggt 1560

tcgtgcacac agcccagctt ggagcgaacg acctacaccg aactgagata cctacagcgt 1620

gagcattgag aaagcgccac gcttcccgaa gggagaaagg cggacaggta tccggtaagc 1680

ggcagggtcg gaacaggaga gcgcacgagg gagcttccag ggggaaacgc ctggtatctt 1740

tatagtcctg tcgggtttcg ccacctctga cttgagcgtc gatttttgtg atgctcgtca 1800

ggggggcgga gcctatggaa aaacgccagc aacgcggcct ttttacggtt cctggccttt 1860

tgctggcctt ttgctcacat gttctttcct gcgttatccc ctgattctgt ggataaccgt 1920

attaccgcct ttgagtgagc tgataccgct cgccgcagcc gaacgaccga gcgcagcgag 1980

tcagtgagcg aggaagcgga agagcgccca atacgcaaac cgcctctccc cgcgcgttgg 2040

ccgattcatt aatgcagctg gcacgacagg tttcccgact ggaaagcggg cagtgagcgc 2100

aacgcaatta atgtgagtta gctcactcat taggcacccc aggctttaca ctttatgctt 2160

ccggctcgta tgttgtgtgg aattgtgagc ggataacaat ttcacacagg aaacagctat 2220

gaccatgatt acgaattgag ctcggtaccc ggcctcgagg tttaaacagg ccgtctcgat 2280

ttgactacct cacaacagca agagctttgt tgcatggaga gcttgcctcc aaaccgtgga 2340

gcttacgaac acattcagga atagctcttc tataccaatc aatcaaaaaa gaagggtgga 2400

attttattcc acgacaagta cttcctccaa tagtggcaaa cagcgcaatt ggagtgattc 2460

tttattctac ctatctcaca ctattacaac gttttaataa gagaaatatg aactcaattg 2520

atgatcctag tcctgtcgac actttcaaag ctggttttgt tgcgggagct gctgcgagct 2580

tggctgcatc accgctagat gcactttacg cccgatcgaa ttactcagag cttgtcagtg 2640

gaaaacagaa gagcatgtgg gagtatgcct tatacaaact aaaacaaatt ggtttagttg 2700

gaatatttgc cggcttcagt ttaaatttca taaaggaaag ccttggattt gcattctact 2760

tttcagtatt tgaactagtg aaaaaccagg ggtaccacaa gacaaaagca gctatacagt 2820

ggtatcaata ttggcgctcg agactttttc atgatgaaga aaaccaggag aagaattcaa 2880

gatttttcaa agctttgaat ttgacgttta tccttcttgc tggagcttcc gccgcaacta 2940

cactgcttgc aatccaatat ccactcaata aaattcaaaa ggtccacctg actagactcg 3000

aatctctaga tctctacaac gaagcacatc aactggatcg gcgtcgcttc ttcaaactct 3060

actataattc ttacattgag acatttgata ttattctaca acgatggcga aaatctcacc 3120

tctccctctt tggcttctgc tatagaggct ttttcagatc gtccttcact agtattcccg 3180

cgacttctat tgggcttttc gtattcgaaa tatcccgtca gcattttagc gaaaatcaat 3240

tttgacgatg tatatagaac tgaaagccgt taattaacaa tctccggaat ggtgatctga 3300

tcgttcctga aaacctcgac attggctccc tcctgacaca ggtactcgta caggttccag 3360

gtaaacgagt cgtagttgtc gatcatgaca acgttcttag aagcggccgg cattttgaag 3420

gtgactaata gcctaagaaa atatttaatt taattttcat taaattttcc tatactcgct 3480

atttcagctt ttcatctcat cacttcataa acgatataaa ccagaaaaag aactattttc 3540

aaacacgctt ctcaaaagcg gtatgtcctt ccacgtctcc ttagaatctg gcaagtccgc 3600

gagggggatc cctattaggc gtagtctggc acgtcgtatg ggtacaatgg tttctggtcg 3660

tcagcctcgt cagctggagc gtaagaaccg tcctctctgt gaacctcgtt accagtcaaa 3720

cctggggtaa aaacacaggc caggtgcata gtcttagaag ctctcaacag gtgctcgtcg 3780

tgctggtcca aaatgtagat gtcgcctggc ttaataggcc agatcttacc gtcagccaaa 3840

gtctcaacct cgccctcacc ctcaatacag tacacggcct cgaagtggtg tttgtagtgg 3900

atgtgagtct cggttccctc gaagattctg gtgatgtgga aagagcagtt accaccgtcc 3960

tcagccaaag acaatctggt agagtcccag ttaccgttct cagcagtaac caatctgtcg 4020

gtctgtctag cttcttccaa gtttctcacg atcatgaatt cccgatgaag gcagagagcg 4080

caaggaggcg gtatttatag tgccattccc ctctctgaga gacccggatg gtagtcgagt 4140

gtatcggaga cagcttgatg tagactccgt gcctgccggc tcctcttatt ggcggacacc 4200

agtgagacac cccggaactt gctgtttttc tgcaaaatcc ggggtgacca gtgggagcct 4260

atttgcacac acgagcggga caccccactc tggtgaagag tgccaaagtc attctttttc 4320

ccgttgcggg gcagccgatt gcatgtttta ggaaaatatt acctttgcta caccctgtca 4380

gatttaccct ccacacatat atattccgtc acctccaggg actattattc gtcgttgcgc 4440

cgccagcgga agatatccag aagctgtttt ccgagagact cggttggcgc ctggtatatt 4500

tgatggatgt cgcgctgcct cacgtcccgg tacccaggaa cgcggtggga tctcgggccc 4560

atcgaagact gtgctccaga ctgctcgccc agcaggtgtt tcttgatcgc cgcctctaaa 4620

ttgtccgcgc atcgccggta acatttttcc agctcggagt ttgcgtttag atacagtttc 4680

tgcgatgcca aaggagcctg cagattataa cctcggatgc tgtcattcag cgcttttaat 4740

ttgacctcca gatagttgct gtatttctgt tcccattggc tgctgcgcag cttcgtataa 4800

ctcgagttat tgttgcgctc tgcctcggcg tactggctca tgatctggat cttgtccgtg 4860

tcgcttttct tcgagtgttt ctcgcaaacg atgtgcacgg cctgcagtgt ccaatcggag 4920

tcgagctggc gccgaaactg gcggatctga gcctccacac tgccctgttt ctctatccac 4980

ggcggaaccg cctcctgccg tttcagaatg ttgttcaagt ggtactctgt gcggtcaatg 5040

aaggcgttat tgccggtgaa atctttggga agcggttttc ctcggggaag attacgaaat 5100

tccccgcgtc gttgcgcttc ctggatctcg atcgataact tcgtatagca tacattatac 5160

gaagttatat gcgcactcta gagcaattcc gacggttggg atcgtgtatg gtgcagagag 5220

accgaccacc acggggccga tcgactgcgt caggaagtgg atcgggaaca gcttggattg 5280

caggaggcca aactccttgc ggggcaggac cttgaacgct ctgacagagg acacaaatga 5340

gtggaacgaa gtggctccga aaacatacga gtaagcaagc aggtgcacag cagtcagcat 5400

ggtaaaaatt aattgcactt attatttatt tggtcgtggc tttatttaat cagtgataat 5460

tgtcacgcta tatgtagcca agtttttcca ttctaatctt gacggcgttg cggtgcgcgt 5520

cgagaccctc gacggcagcc aggtccatca cggccttgcc aatgctcttg agaccctcct 5580

tggtgaccac ctgcgatgta atgaatttct ggaacgtggt ggtgttaaca cccgagtaca 5640

ttctggcgta gccgtacgta ggcagcgtgt ggttggttcc cgaggagtag tcaccgcagc 5700

tttccggcga ataggcaccc acaaacacag agccagcatt gtcgaccttg tcgacccatt 5760

tctcggcgtc ctggatctgc aggataaggt gctccggagc gtatctgttg ctcatctcga 5820

acgcctcatc gtagctcttc acaatgatcg tggtggagtg agcaatgcac tggcgcacga 5880

tctccacacg tggcagctgc agcgcctggc gatggacggc ctcgtcgatc ttgtcgatct 5940

cggcagacgt catgtccacg gcgatcaata tcacttgcga gtcaacgccg tgttctgcct 6000

gcgacagcag gtccgaggcc acaaagtctg ggtcggcgta cttgtccgcg acaaccagaa 6060

cctcggatgg cccggcaggc atatcaatgg cacacatggc gttggaatca ttggaaacga 6120

gcatcttggc tgccgtcaca aactggtttc cggggcccat gatcttgtca catttaggca 6180

cagactcggt accgtacgcc attgcggcca ccgcctgggc ccctccggca agcacaatgc 6240

acttggcacc caccttgtgc gccacataga ccacctctgg cgtcagacgg ccgtccttgc 6300

cgggaggaga cgcaaacaca atatctttgc aaccggcaac gagagcaggc acagagagca 6360

taagggacgt ggacggcagc acggcagttc cgccggggat gtaacagcca acacgctcga 6420

tcggacgtgc aaatcgagag cacacaacgc cgggacacgt ttccacgcgt agagtctctt 6480

tctggttctg cgcttcgtgg aaggtcttga tattggcaaa cgagatgtca atggccttgc 6540

gcacgtcttc gctgatgttc atcatttctg ctgggaatgg agcgttcaat acaggagagc 6600

taagctgcac cttgtcgaac ttggctgtga gctccaccaa cgcagcgtct ccttttgttc 6660

tcactttctc gacgatcggc gtaaccaggt tcatgatatc cgcagacttt tggattggtc 6720

tttgaagaca tttttcgata tcttctttgg aggcagtggt ggcatccaca cggttcatca 6780

cgatgcggcc atctccgttc acaagccgct ttggcaattt tggcaccgtt ggcaccgttg 6840

gttcctctag ctttttttcc gtagcgtcct gctcaacata cttcgccttt gcgtcgccct 6900

tacgtctcgt caccttcagg gccttgatgt ccaaattccg ctcaacgtct gcaagcgaaa 6960

cgccgtactt cacacatctg gcaagcgcaa agtagaccaa atcggcagct tcccacgcaa 7020

tctcgtcctt cgaggtggct tccaccagct cgtcggcttc ttccctaatc ttggcaccga 7080

gcagcttctc atcctcaaac agtctcttcg tgtaggagcc ttcaggagca ttcgccttgc 7140

ggtcccaaag agtggcttcc agagctgcca gacccttgga cgagccaaaa caggaggcag 7200

tgtcgaggtg gcagaaacca gcaccggcct gttccacaac aaatttcaaa caatcgctat 7260

cgcagtctag ctccagttgg agcaattttt gcgtggctcc cgatgtctgg cctttgtacc 7320

acaagccgtg ctttctggac tggtagacac ccgtgccggt ccttattgcc tcagcaatgg 7380

acttcttgga cgagtacacc aaaccaagag cctggttcct gctgtcagtg acaagagttg 7440

tgtataagcc gtctggtctg tcggtgttca tggcagcgag atagcactcc acaaacggcg 7500

ttttggtgcc cgcgttttcg tactccttag tcagctgatc gagctcaata acagcagtga 7560

agcccttggc gatctgccgg agtggttgtt caaaataacc aaagactctt ctttttccaa 7620

ttttcacttc agtctcccca aaaacacaga tattctcggc agcatctttt ccatccttga 7680

tgaccagtct ggcgttggga atacggcttc taatctgctc agcaacagca gaggtgacaa 7740

aaagaccgta cacaccgttg ttcaaaagag ccagaacaat gtctatttgg ctatcatttt 7800

ccacagaccc cagagcccag tcgacatata cctggcctag atactcttta gccaacgcga 7860

taatttcctg cacagcggca gccgcatcaa gcgacacaag aacctcgctc acaaacggca 7920

cgctcaaaac gtcccttggt tgagcaacgc tcacaaggat aggagaaacc atttatgaat 7980

ttacggtgag atgtgagtca agctgaaaaa tatcttgatt tttttttttt tgacatgcca 8040

cgactttggt agcatgggac gatatatcac tatatacact gtgagtcagt tattgaacaa 8100

aaactgcata tcgtcgacgg cgcgccataa cttcgtatag catacattat acgaagttat 8160

cctgcagggt tgattattat tcagggaaat gttgaaaatt tttcgataat tcattagttt 8220

atttttgata caccatggat caactcaact ataaagaaca gcaagaattc cagaaaattg 8280

tggagcagaa gcaaatgaat gacttcatga gagtaagtga acgccttctg aaaactaaca 8340

ttgcagctct acaccagttt ggtggacaga tgttttaatg attgcgtcaa cgacttcacc 8400

tccaacaaac ttagctctag agaagaaaca tgtttgaaca agtgcgctga aaagttcctc 8460

aagcactccg aaagagttgg tttaagattc caggaacaga accagatgtt gatgaaccag 8520

atgagaggcg gcaattaatg agctttaatt gctggatgat tcccggaata ggctcaacaa 8580

gttttgcgtt tcttccatca tttcaatcgt ccatttgtaa gtttctgtct gattcatatc 8640

gttgtacata attgcgttgc tgaacatcag ttgaagctca aattccaact cgtcgaatga 8700

ctttatttca cccgtcctaa tagctttacc caaactctta atgtccacgg gatgccgtat 8760

tagcttgtag tagttgggct cttcgttttc attcactggt tgcatgaaca tagacgcaaa 8820

ccggttggat gatatctgtg tgagaagttg tgcagacaga gcattgaagc gcttagtaca 8880

accagggggg acgctttcgg atccgcgtcg cttgctccca cgttcttcat ctccacttgc 8940

tggcgtagat ggtgtagcag gttctggagt tggcgatggt atttctttag aagccgactt 9000

ggattccaat ttcgtttggg gttgggattc tacattcgac tctgaggttg aagctgggcc 9060

ttcttgaggt tcggcatgag tttccaataa ctctgttttg cttttttcca aagcagtgac 9120

cggctgagat tgagccgctg attctgcctc gggctccgca acttcatcga tctctttgtc 9180

tgcgtcatta tgcgtcacgc cctcttcagg ttcttgtgcg tcacctgcgg ccgcgcatgc 9240

ggtctctagc ttggcactgg ccgtcgtttt acaacgtcgt gactgggaaa accctggcgt 9300

tacccaactt aatcgccttg cagcacatcc ccctttcgcc agctggcgta atagcgaaga 9360

ggcccgcacc gatcgccctt cccaacagtt gcgcagcctg aatggcgaat ggcgcctgat 9420

gcggtatttt ctccttacgc atctgtgcgg tatttcacac cgcatatgct cgtcaaagca 9480

accatagtac gcgccctgta gcggcgcatt aagcgcggcg ggtgtggtgg ttacgcgcag 9540

cgtgaccgct acacttgcca gcgccctagc gcccgctcct ttcgctttct tcccttcctt 9600

tctcgccacg ttcgccggct ttccccgtca agctctaaat cgggggctcc ctttagggtt 9660

ccgatttagt gctttacggc acctcgaccc caaaaaactt gatttgggtg atggttcacg 9720

tagtgggcca tcgccctgat agacggtttt tcgccctttg acgttggagt ccacgttctt 9780

taatagtgga ctcttgttcc aaactggaac aacactcaac cctatctcgg gctattcttt 9840

tgatttataa gggattttgc cgatttcggc ctattggtta aaaaatgagc tgatttaaca 9900

aaaatttaac gcgaatttta acaaaatatt aacgtttaca attttatggt gcactctcag 9960

tacaatctgc tctgatgccg catagttaag ccagccccga cacccgccaa cacccgctga 10020

cgcgccctga cgggcttgtc tgctcccggc atccgcttac agacaagctg tgaccgtctc 10080

cgggagctgc atgtgtcaga ggttttcacc gtcatcaccg aaacgcgcga 10130

<210> 8
<211> 9374
<212> DNA
<213> Hansenula polymorpha (syn. Pichia angusta)

<220>
<221> source

<222> 1..9374
<223> /mol_type="unassigned DNA"
      /organism="Hansenula polymorpha (syn. Pichia angusta)"

<220>
<221> gene
<222> 6..1028
<223> /note="H6-EctD fusion ORF (start methionine/His tag and flanking
      residues coding region/EctD coding region)"

<220>
<221> terminator
<222> 1038..1365
<223> /note="MOX-T; terminator of MOX gene"

<220>
<221> misc_feature
<222> 1371..2436
<223> /note="ADE1 downstream region for homologous integration of the
      integration cassette"

<220>
<221> gene
<222> 1371..2436
<223> /note="AmpR; Ampicillin resistence gene"

<220>
<221> misc_feature
<222> 5602..6658
<223> /note="ADE1 upstream region for homologous integration of the
      integration cassette"

<220>
<221> misc_feature
<222> 6665..6698
<223> /note="loxP site"

<220>
<221> gene
<222> 6962..8050
<223> /note="Synthetic H. polymorpha LEU2 gene; codons optimized for
      Hansenula polymorpha"

<220>
<221> misc_feature
<222> 8267..8300
<223> /note="loxP site"

<220>
<221> promoter
<222> 8307..9372
<223> /note="FMD promoter"

<400> 8
aattcatgaa acaccatcat caccaccacc agtctgttca gacctcgtct aacagaccat      60

tgccacaggc taacctgcac attgctaccg agactccaga ggctgactcg agaattagat     120

cggctccaag accaggacag gacccatacc caaccagatt gtctgagcca ttggacttgc     180

cttggctgaa cagaagagag ccagttgtta aaggcgagga ggctgacggt ccattgtctg     240

ctgctcagtt ggacaccttt gagagacagg gcttcatttt cgagccagac tttttgaagg     300

gcgaggagtt ggaggctttg agacacgagc tgaacgcttt gttggctaga gatgacttca     360

gaggtagaga cttcgctatt accgagccac agggcaacga gattagatcg ctgttcgccg 420
ttcactacct gtcgagagtg ttttcgagac tggccaacga cgagagattg atgggtagag 480
ccagacagat tttgggtgga gagccatacg ttcaccagtc gagaatcaac tacaagccag 540
gctttgaggg aaagggtttc aactggcact ctgactttga gacttggcac gctgaggacg 600
gaatgccagc tatgcacgcc gtttctgcct ctattgtgct gaccgacaac cacactttta 660
acggcccact gatgttggtt ccaggctctc acagagtgtt cgttccatgt ctgggagaga 720
ctccagagga ccaccacaga cagtctctga aaacccagga gttcggagtt ccatcgagac 780
aggctctgag agagctgatt gacagacacg gtattgaggc tccaactggt gctgccggtg 840
gtttgttgct gttcgactgc aacactctgc acggttctaa cgccaacatg tcgccagacc 900
caagatcgaa cgccttcttc gtgtacaata gaagagacaa cagatgcgtt gagccatacg 960
ccgcttctaa gagaagacca agattcttgg ctcacgagcc agacgaggct tggtcgccag 1020
acggttaata aggatccccc tcgcggactt gccagattct aaggagacgt ggaaggacat 1080
accgcttttg agaagcgtgt ttgaaaatag ttctttttct ggtttatatc gtttatgaag 1140
tgatgagatg aaaagctgaa atagcgagta taggaaaatt taatgaaaat taaattaaat 1200
attttcttag gctattagtc accttcaaaa tgccggccgc ttctaagaac gttgtcatga 1260
tcgacaacta cgactcgttt acctggaacc tgtacgagta cctgtgtcag gagggagcca 1320
atgtcgaggt tttcaggaac gatcagatca ccattccgga gattgttaat taagtgttga 1380
aataaaccaa gctgtcccaa ataatttaat tattttctgt atccggaagt ttgtaaatat 1440
actattcgat ggacgatttc caagtgttgg agctcatcgg acgaggctcg ttcggatgtg 1500
tgcgcaaagt aaggcgtgtc gctgacggaa aggtgtttgt acgcaaggaa atatcatacg 1560
gggccatgaa tagcaaggag aagcaccagc ttagcaccga gttcaggatc ctacgagaac 1620
tgagacatcc caacattgtg cagtatctcc accatgacca tgtccaggat gagcaccagg 1680
ttcatttgta tatggagtac tgcgacggag gcgatctatc ggtgctgata aagaagtaca 1740
aggagagaaa cgagtttata ccggaaacac tgatctggca gatatttacc caggtgttga 1800
atgcgcttta caaatgtcac tatggaacgt cgatagagcc ggtcagcaat cttttccagt 1860
cgtctcccga actagaaccg aaacctgtgg accatcaggt gattattcat cgcgacatca 1920
aaccagacaa cattttcctt ttgtcggacg ggtattcgat caaactgggg gatttcggtc 1980
ttgcaaagtg gctccaacac gaaaacgagt ttgccaagac ctatgtgggc acgccatact 2040
acatgtctcc cgaggtgctg atggatagac cgtacgaccc cgtctgcgac gtgtggtcgc 2100
tcggatgcgt catgtatgaa ttgtgtgctc tacgtcctcc tttccaggcc aagactcact 2160
tggcattgca ggaaaaaatc aaggctggca cgttccagcc gatccctgac cattactcgc 2220
atagactaca gatggtcatc aacgcatgtc ttattgtcga ccccatggaa cgcgctaccg 2280

tcaaccaact gctacaggac gcctctttca aattgttcag aaaagagtgg gaaatgacgc 2340

aatgggagaa ccggctgcgc gagcacgaaa cagagctgct tcttaaggaa cgcaagctgg 2400

tcaagtttga ggacgctctc aacgaagaat gcgtttaaac ctcgaggccg ggtaccgagc 2460

tcaattcgta atcatggtca tagctgtttc ctgtgtgaaa ttgttatccg ctcacaattc 2520

cacacaacat acgagccgga agcataaagt gtaaagcctg gggtgcctaa tgagtgagct 2580

aactcacatt aattgcgttg cgctcactgc ccgctttcca gtcgggaaac ctgtcgtgcc 2640

agctgcatta atgaatcggc caacgcgcgg ggagaggcgg tttgcgtatt gggcgctctt 2700

ccgcttcctc gctcactgac tcgctgcgct cggtcgttcg gctgcggcga gcggtatcag 2760

ctcactcaaa ggcggtaata cggttatcca cagaatcagg ggataacgca ggaaagaaca 2820

tgtgagcaaa aggccagcaa aaggccagga accgtaaaaa ggccgcgttg ctggcgtttt 2880

tccataggct ccgcccccct gacgagcatc acaaaaatcg acgctcaagt cagaggtggc 2940

gaaacccgac aggactataa agataccagg cgtttccccc tggaagctcc ctcgtgcgct 3000

ctcctgttcc gaccctgccg cttaccggat acctgtccgc ctttctccct tcgggaagcg 3060

tggcgctttc tcaatgctca cgctgtaggt atctcagttc ggtgtaggtc gttcgctcca 3120

agctgggctg tgtgcacgaa ccccccgttc agcccgaccg ctgcgcctta tccggtaact 3180

atcgtcttga gtccaacccg gtaagacacg acttatcgcc actggcagca gccactggta 3240

acaggattag cagagcgagg tatgtaggcg gtgctacaga gttcttgaag tggtggccta 3300

actacggcta cactagaagg acagtatttg gtatctgcgc tctgctgaag ccagttacct 3360

tcggaaaaag agttggtagc tcttgatccg gcaaacaaac caccgctggt agcggtggtt 3420

tttttgtttg caagcagcag attacgcgca gaaaaaaagg atctcaagaa gatcctttga 3480

tcttttctac ggggtctgac gctcagtgga acgaaaactc acgttaaggg attttggtca 3540

tgagattatc aaaaaggatc ttcacctaga tccttttaaa ttaaaaatga agttttaaat 3600

caatctaaag tatatatgag taaacttggt ctgacagtta ccaatgctta atcagtgagg 3660

cacctatctc agcgatctgt ctatttcgtt catccatagt tgcctgactc cccgtcgtgt 3720

agataactac gatacgggag ggcttaccat ctggccccag tgctgcaatg ataccgcgag 3780

acccacgctc accggctcca gatttatcag caataaacca gccagccgga agggccgagc 3840

gcagaagtgg tcctgcaact ttatccgcct ccatccagtc tattaattgt tgccgggaag 3900

ctagagtaag tagttcgcca gttaatagtt tgcgcaacgt tgttgccatt gctacaggca 3960

tcgtggtgtc acgctcgtcg tttggtatgg cttcattcag ctccggttcc caacgatcaa 4020

ggcgagttac atgatccccc atgttgtgca aaaaagcggt tagctccttc ggtcctccga 4080

tcgttgtcag aagtaagttg gccgcagtgt tatcactcat ggttatggca gcactgcata 4140

attctcttac tgtcatgcca tccgtaagat gcttttctgt gactggtgag tactcaacca 4200

agtcattctg agaatagtgt atgcggcgac cgagttgctc ttgcccggcg tcaatacggg 4260

ataataccgc gccacatagc agaactttaa aagtgctcat cattggaaaa cgttcttcgg 4320
ggcgaaaact ctcaaggatc ttaccgctgt tgagatccag ttcgatgtaa cccactcgtg 4380
cacccaactg atcttcagca tcttttactt tcaccagcgt ttctgggtga gcaaaaacag 4440
gaaggcaaaa tgccgcaaaa aagggaataa gggcgacacg gaaatgttga atactcatac 4500
tcttcctttt tcaatattat tgaagcattt atcagggtta ttgtctcatg agcggataca 4560
tatttgaatg tatttagaaa aataaacaaa taggggttcc gcgcacattt ccccgaaaag 4620
tgccacctga cgtctaagaa accattatta tcatgacatt aacctataaa aataggcgta 4680
tcacgaggcc ctttcgtcct cgcgcgtttc ggtgatgacg gtgaaaacct ctgacacatg 4740
cagctcccgg agacggtcac agcttgtctg taagcggatg ccgggagcag acaagcccgt 4800
cagggcgcgt cagcgggtgt tggcgggtgt cggggctggc ttaactatgc ggcatcagag 4860
cagattgtac tgagagtgca ccataaaatt gtaaacgtta atattttgtt aaaattcgcg 4920
ttaaattttt gttaaatcag ctcatttttt aaccaatagg ccgaaatcgg caaaatccct 4980
tataaatcaa aagaatagcc cgagataggg ttgagtgttg ttccagtttg gaacaagagt 5040
ccactattaa agaacgtgga ctccaacgtc aaagggcgaa aaaccgtcta tcagggcgat 5100
ggcccactac gtgaaccatc acccaaatca agttttttgg ggtcgaggtg ccgtaaagca 5160
ctaaatcgga accctaaagg gagcccccga tttagagctt gacggggaaa gccggcgaac 5220
gtggcgagaa aggaagggaa gaaagcgaaa ggagcgggcg ctagggcgct ggcaagtgta 5280
gcggtcacgc tgcgcgtaac caccacaccc gccgcgctta atgcgccgct acagggcgcg 5340
tactatggtt gctttgacga gcatatgcgg tgtgaaatac cgcacagatg cgtaaggaga 5400
aaataccgca tcaggcgcca ttcgccattc aggctgcgca actgttggga agggcgatcg 5460
gtgcgggcct cttcgctatt acgccagctg gcgaaagggg gatgtgctgc aaggcgatta 5520
agttgggtaa cgccagggtt ttcccagtca cgacgttgta aaacgacggc cagtgccaag 5580
ctagagaccg catgcgcggc cgccacacca ctgaaggctc ccgaagacag atgcacaggt 5640
ctgagcgaga gatatcttgc ttgcagggag taagtgaatg gaacccaaca cagatcgcca 5700
aatgcgagca tgaaaccgaa tccgtcggtg gtaatgtcca tcatggaaag cacgccttcc 5760
tcgttgagca ctccgtcgaa aatgtagaag gattgcatcg caacgacgaa caagagcgag 5820
ttagccaccg ttccagtttc cagatactgt ttttggatgc atgagaggtc tatcagcagc 5880
caaagcagca ttcctggccg tagctcgcaa aagagcttga tgtcccaggg tcctattctg 5940
gggttgagct ctctgccgat aaaccagtca aaaaggacgt ttttggagtt tccaccctcc 6000
gcaagaatgc gctcgtacgt tcctgcacca ttgggccgtc tcagcgggaa aaaggagcag 6060
acgtagacaa acgtgctgag aataagagaa aattcccacg aaacgaccat caggggcaaa 6120
aaattgtcat aaataaactg aagttcgggc acattgcccc ttgtgtacaa cgctcttgct 6180

gccaaagctg cacagaccaa aacactcagc tccttaccgt tgatcagata cgttaaatgt 6240

gttccgtcac gcagcgttgt gcctttttta tagtagccag gaacaataag atccagcaat 6300

gcaagaccaa aaaaccagac caggtatgct atccagcatt tggcactcag aaacgtttcc 6360

ttggtgagtt gttctgcaag caagtttttg atcgtgttga tctgcaaatt gcacccctcg 6420

acaacatacc gatcattgca cgccgcatac aggaaaacaa ccaccaaagg tagactgatg 6480

gtcagaacca aggccccagg aaacccaaag aagtctttgt ggacggtgac tggattgagc 6540

atagcagagg aaagttgaca aaaactcgta taaaaaggag agccaatatc gcgtcgatcg 6600

accctcacga gtcaattaat ttactaaact aattttactt aattattatt acacatcctg 6660

caggataact tcgtataatg tatgctatac gaagttatgg cgcgccgtcg actgtgcacg 6720

gctggggccc tcttagtaag ctgaaacgtc ttgagctcag agaggagaag ccaaaggtga 6780

acaatgcggc cactgctcca ctcgaggagg tagacatcga ccagtttgtg tctgagcaga 6840

tctgagtggg tgagtacgta agcggtttgg taatacgaaa taaaaagaca ggaatgagta 6900

agtggatggt ttttcaattt ttcccggtac cggcgcaaaa tagttgcatc attttgcaat 6960

catgtcgaag aacatcgttc tgttgccagg tgaccacgtt ggaccagagg ttgtggctga 7020

ggctgttaag gttttggagg ccgtttcttc tgccatcggc gtgaagttca acttctcgaa 7080

gcacctgatt ggtggtgctt ctattgacgc ctacggcgtt ccattgtctg acgaggcttt 7140

ggaggctgct aaaaaggctg acgctgttct gttgggtgct gttggtggtc caaaatgggg 7200

aaccggctct gttagaccag agcagggact gctgaagatc agaaaagagc tgaacctgta 7260

cgctaacctt agaccatgtt cttttgcctc tgacgctctg ttgaagctgt ctccactgaa 7320

gtctgagatc gtgaagggaa ccgacttcgt ggttgtgaga gagctggttg gcggtatcta 7380

cttcggcgac agaaaagagg acgctggcga cggcgttgct tctgacactg agagctactc 7440

tgttccagag gtgcagagaa tcaccagaat ggccgctttt ctggctttgc agtctgaccc 7500

accattgcca ttgtggtctt tggacaaggc taacgtgttg gcttcttcga gactgtggag 7560

aaagaccgtg gaggaaacta tcaagaacga gttcccacag ttgactgttc agcaccagct 7620

gattgactct gccgccatga tcttggttaa gtcgccaacc aagctgaacg gtgttatcgt 7680

gaccaacaac atgttcggcg acatcatttc ggacgaggcc tctgttattc caggttctct 7740

gggattgctg ccatctgcat ctttggcttc cctgccagac accaacaagg ctttcggtct 7800

gtacgagcca tgtcacggtt ctgctccaga cttgggtcca ggaaaggtta acccactggc 7860

caccattttg tctgctgcca tgatgctgaa gctgtctttg gacttggttg acgccggtag 7920

agctattgag caggccgtga agaacgtttt ggacgccggt attatgactg ctgacctggg 7980

cggttcttct tctactcagg aagttggcga cgctgttgca caggaagttg ccaagctgct 8040

gaagaactaa ataagggaga aaaaaaagta ggatctcgaa taattcctaa ataatcccaa 8100

aaatcctaaa cacgcacgcc tcacagattt tatttttttc gacgcgacgc tctattgttt 8160

attttttagc ttttccatgt caacatgaga ttcggtgtcg tattcgtgat cacccaagtc 8220

ctagcagact ttacgtattt aggttgctac actctagagt gcgcatataa cttcgtataa 8280

tgtatgctat acgaagttat cgatcgagat ccaggaagcg caacgacgcg gggaatttcg 8340

taatcttccc cgaggaaaac cgcttcccaa agatttcacc ggcaataacg ccttcattga 8400

ccgcacagag taccacttga acaacattct gaaacggcag gaggcggttc cgccgtggat 8460

agagaaacag ggcagtgtgg aggctcagat ccgccagttt cggcgccagc tcgactccga 8520

ttggacactg caggccgtgc acatcgtttg cgagaaacac tcgaagaaaa gcgacacgga 8580

caagatccag atcatgagcc agtacgccga ggcagagcgc aacaataact cgagttatac 8640

gaagctgcgc agcagccaat gggaacagaa atacagcaac tatctggagg tcaaattaaa 8700

agcgctgaat gacagcatcc gaggttataa tctgcaggct cctttggcat cgcagaaact 8760

gtatctaaac gcaaactccg agctggaaaa atgttaccgg cgatgcgcgg acaatttaga 8820

ggcggcgatc aagaaacacc tgctgggcga gcagtctgga gcacagtctt cgatgggccc 8880

gagatcccac cgcgttcctg ggtaccggga cgtgaggcag cgcgacatcc atcaaatata 8940

ccaggcgcca accgagtctc tcggaaaaca gcttctggat atcttccgct ggcggcgcaa 9000

cgacgaataa tagtccctgg aggtgacgga atatatatgt gtggagggta aatctgacag 9060

ggtgtagcaa aggtaatatt ttcctaaaac atgcaatcgg ctgccccgca acgggaaaaa 9120

gaatgacttt ggcactcttc accagagtgg ggtgtcccgc tcgtgtgtgc aaataggctc 9180

ccactggtca ccccggattt tgcagaaaaa cagcaagttc cggggtgtct cactggtgtc 9240

cgccaataag aggagccggc aggcacggag tctacatcaa gctgtctccg atacactcga 9300

ctaccatccg ggtctctcag agaggggaat ggcactataa ataccgcctc cttgcgctct 9360

ctgccttcat cggg 9374

<210> 9
<211> 5559
<212> DNA
<213> Hansenula polymorpha (syn. Pichia angusta)

<220>
<221> source
<222> 1..5559
<223> /mol_type="unassigned DNA"
      /organism="Hansenula polymorpha (syn. Pichia angusta)"

<220>
<221> misc_feature
<222> 1..1094
<223> /note="HIS4 upstream region for homologous recombination of the
      integration cassette"

<220>
<221> terminator
<222> 1102..1429
<223> /note="MOX-T; terminator of MOX gene"

<220>
<221> gene
<222> 1439..2047
<223> /note="EctA-myc fusion ORF (EctA coding region/myc tag coding
      region)"

<220>
<221> promoter
<222> 2055..3120
<223> /note="FMD promoter"

<220>
<221> misc_feature
<222> 3127..3160
<223> /note="loxP site"

<220>
<221> gene
<222> 3352..4263
<223> /note="Synthetic ADE1 gene; codons optimized for Hansenula
      polymorpha"

<220>
<221> misc_feature
<222> 4493..4526
<223> /note="loxP site"

<220>
<221> misc_feature
<222> 4527..5558
<223> /note="HIS4 downstream region for homologous recombination of the
      integration cassette"

<400> 9
aaacgaggat tctggcccta caactgcaaa gaagctgtgc gagggcacgc tcgtcgtttg      60

ggccagccaa tgtgggcaac aatctcgcca acctggacgt tttgggctct tctccagtgc     120

ctgagaacaa tatcgagatg atctctaagg actcgattat cttctccaac atgaaggttg     180

tgcgatcgcc caacaggcaa gggactcttg ttgctgctct gttgttggat aaccagatat     240

ttgagctcaa cacaaagtcc atcagcaaaa aaggcccatt ggatttgaca actactgagc     300

tctttccctt gttagaggcg ctctacccaa aacccgagct gtttgtgctg ggtcttgggt     360

tcagcaaccg gatgttacac ccagacacga aaagacgctt ttccgagttg gggatcatgc      420

tcgaagtagg cgacaccaag agcgcagtgc tcagctacga tcttctggct acggaacgtt     480

ctccaaaaca ggtaggggca ctcatatttc catcggtgtt ctgaaatatg cgcgaaacaa     540

ctatttaagg gctctgacca ggaattgcgt gctgaacaca taggagtagg cagcaagggt     600

aaggacgctc agactgttga acacgaggct gattccatgg tacttgccga acttcttggt     660

caaagctgcg attttttcgt tggtctcggg agttctctcg ctctctggca gttcctcgag     720

cgcaaagcgt tcagtcttca gccggttgca cactggcatc agccacaagt aattggtgag     780

acctgagagc gaagaggccg ccagcaatgc aattccgacg gttgggatcg tgtatggtgc     840

agagagaccg accaccacgg ggccgatcga ctgcgtcagg aagtggatcg ggaacagctt     900

ggattgcagg aggccaaact ccttgcgggg caggaccttg aacgctctga cagaggacac 960
aaatgagtgg aacgaagtgg ctccgaaaac atacgagtaa gcaagcaggt gcacagcagt 1020
cagcatggta aaaattaatt gcacttatta tttatttggt cgtggcttta tttaatcagt 1080
gataattgtc acgttaatta acaatctccg gaatggtgat ctgatcgttc ctgaaaacct 1140
cgacattggc tccctcctga cacaggtact cgtacaggtt ccaggtaaac gagtcgtagt 1200
tgtcgatcat gacaacgttc ttagaagcgg ccggcatttt gaaggtgact aatagcctaa 1260
gaaaatattt aatttaattt tcattaaatt ttcctatact cgctatttca gcttttcatc 1320
tcatcacttc ataaacgata taaaccagaa aaagaactat tttcaaacac gcttctcaaa 1380
agcggtatgt ccttccacgt ctccttagaa tctggcaagt ccgcgagggg gatccttact 1440
acaggtcttc ctcagaaatc agcttctgct caatctggtc ggtctggaat ggaccaattc 1500
taaccaagtt ctctgggtcg tgctcaccac ccaactggtc ggtagaaaag tactctctag 1560
agttcaatgg ggcctgccat ctgtcagcca atcttctaaa caggccccaa gaagcctggt 1620
tgtctggagt aatggtagtc tccaagtggt gaacctcagc catctctggt ctggtcataa 1680
cggcctcaac caatcttcta gccagaccag tacctctagc cttctcacca acagcaacct 1740
gccacaaaaa gtaggtgtcc ggagcgttag acttcacgta accagacacg aaaccaacga 1800
tctcaccttc ctcgttggta gcaacagcac aagagtctct gaactgggta gccagcaaca 1860
ggtaagcgta agcagagtta acgtccagcg gtggacaaga cttaaccaac tcgtagatac 1920
cccagccgtc gtctggagat ggttttctga tgaacagtgg agaagcctcg tgaccaacaa 1980
cagcgtcagc aacagatggc ttagccaagt cagcagatgg agtgaatggc tcggtggtag 2040
cgttcatgaa ttcccgatga aggcagagag cgcaaggagg cggtatttat agtgccattc 2100
ccctctctga gagacccgga tggtagtcga gtgtatcgga gacagcttga tgtagactcc 2160
gtgcctgccg gctcctctta ttggcggaca ccagtgagac accccggaac ttgctgtttt 2220
tctgcaaaat ccggggtgac cagtgggagc ctatttgcac acacgagcgg gacaccccac 2280
tctggtgaag agtgccaaag tcattctttt tcccgttgcg gggcagccga ttgcatgttt 2340
taggaaaata ttacctttgc tacaccctgt cagatttacc ctccacacat atatattccg 2400
tcacctccag ggactattat tcgtcgttgc gccgccagcg gaagatatcc agaagctgtt 2460
ttccgagaga ctcggttggc gcctggtata tttgatggat gtcgcgctgc ctcacgtccc 2520
ggtacccagg aacgcggtgg gatctcgggc ccatcgaaga ctgtgctcca gactgctcgc 2580
ccagcaggtg tttcttgatc gccgcctcta aattgtccgc gcatcgccgg taacattttt 2640
ccagctcgga gtttgcgttt agatacagtt tctgcgatgc caaaggagcc tgcagattat 2700
aacctcggat gctgtcattc agcgctttta atttgacctc cagatagttg ctgtatttct 2760
gttcccattg gctgctgcgc agcttcgtat aactcgagtt attgttgcgc tctgcctcgg 2820
cgtactggct catgatctgg atcttgtccg tgtcgctttt cttcgagtgt ttctcgcaaa 2880

cgatgtgcac ggcctgcagt gtccaatcgg agtcgagctg gcgccgaaac tggcggatct 2940

gagcctccac actgccctgt ttctctatcc acggcggaac cgcctcctgc cgtttcagaa 3000

tgttgttcaa gtggtactct gtgcggtcaa tgaaggcgtt attgccggtg aaatctttgg 3060

gaagcggttt tcctcgggga agattacgaa attccccgcg tcgttgcgct tcctggatct 3120

cgatcgataa cttcgtatag catacattat acgaagttat atgcgcactc tagattcctt 3180

gcgtacaaac acctttccgt cagcgacacg ccttactttg cgcacacatc cgaacgagcc 3240

tcgtccgatg agctccaaca cttggaaatc gtccatcgaa tagtatattt acaaacttcc 3300

ggatacagaa aataattaaa ttatttggga cagcttggtt tatttcaaca cttaagccca 3360

ggttttaccg gtcaaagcct cgtaagcctc tttgtactta gcggcggtct tagcagcaat 3420

ctcctcggtc atagacacac cctctttacc gttcagctgg tgagaagtca accagtctct 3480

caggaactgc ttgtcgaaag actcctgaga tcttcccaac tcgtacttct tggcgtccca 3540

aaatctcgaa gagtctgggg tcaaaacctc gtcaaccaga accagctcgt tgttctcgtt 3600

cagtccgaac tcaaacttgg tgtcggccag gataatacct ctcgacagag cgtagtctct 3660

agccttagag tacagggcaa tagccttctg ggcaacctca gcacacaact cctctccaac 3720

caactgagca gcctgctctg gagaaatgtt ctcgtcgtgc tgtccctgct ctgctttggt 3780

agatggggtg aagattggct tctcgaaagc ctcagactcc tgcaatccag acttaactgg 3840

cagtccgtga acggtagacg acttcttgta ctctttccag gcagaaccgg tgatgtaacc 3900

tctcacaatg gcctccaatg gaaccagctt gtgcttcttc accagcagag atctaccagc 3960

caactgagcc ttgtactttg gctcagacaa ctgctctggc aacttggcaa agatgtcctt 4020

gtcagaggaa gcaaccaggt ggtttggaat aacgtcagac agcaggttga accagaacac 4080

agacagctgg gtcagaatct ttcccttgtc cttgatgccg ttctccataa tcacgtcgta 4140

ggcagagatt ctgtcggtgg caacaaacag cagcatgttg tcgtccacct ggtagatgtc 4200

tctcacttta cctctggcaa ccaatggcag aataccgtcc aggttagtct gggtcacggt 4260

catatgtgta ataataatta agtaaaatta gtttagtaaa ttaattgact cgtgagggtc 4320

gatcgacgcg atattggctc tcctttttat acgagttttt gtcaactttc ctctgctatg 4380

ctcaatccag tcaccgtcca caaagacttc tttgggtttc ctggggcctt ggttctgacc 4440

atcagtctac ctttggtggt tgttttcctg tatgcggcgt cgacggcgcg ccataacttc 4500

gtatagcata cattatacga agttatcctg caggttatga atttacggtg agatgtgagt 4560

caagctgaaa aatatcttga tttttttttt tttgacatgc cacgactttg gtagcatggg 4620

acgatatatc actatataca ctgtgagtca gttattgaac aaaaactgca tatcggccgc 4680

cgatagcctg ctagccgcgt tctggtcctg atccagcgtg gccttgacca tgtcagcctt 4740

cttctgctgc agctcaatga tccgcgactc gatgctgtcc tcgatgacca acttcacaat 4800

tctgataggc cggtgctgtc caatccggtg aattctgtct gcggcctgac ccgtgtccaa 4860

agcagggttc caccaactat ccaggataaa tacttgattg gcctcgacaa gattcagagc 4920

cacacctccg gccttcaaag acaccaaaaa tacctccacc gacggattgg tcataaagtg 4980

ctggatcaca gagtcacgct ggatcggggt catggatccc tgcaatttcg caatggaaaa 5040

acccgccctt ttgagtcgcc actccaccag gtccagcatc gacgtgaact gcgaaaagac 5100

aatgctttta acagtttgtc tgtccgaacg ggacttgtac aactcttcca ttagggcctc 5160

aattttagtg gaagacttcc attcgcctcc catgtctatt cggttcacaa tagagccctt 5220

ctcaacgacc tcctggttca tctccagcgc tggtgcctcc agatcaatac tcagcccaat 5280

gtggcacact ggacactcga gacgcttatc gtcgccgttg aacgactcca cgtactccgt 5340

gatacagatc cggcagaact tgtggtgaca tttggactct atcggctcct ctgcttcgtc 5400

atcacacagc tggcagacaa tggccccgct gttgaggaga tccctggatg ctttcttaaa 5460

tcgcttgaga accagatccg gatgatccgc aagctgtctc atacgcgtga ttagggtaaa 5520

gatgttggca tagttgttca gaaccacacc ttgtgcagc 5559

<210> 10
<211> 6195
<212> DNA
<213> Hansenula polymorpha (syn. Pichia angusta)

<220>
<221> source
<222> 1..6195
<223> /mol_type="unassigned DNA"
      /organism="Hansenula polymorpha (syn. Pichia angusta)"

<220>
<221> misc_feature
<222> 1..1097
<223> /note="LEU2 downstream region for homologous recombination of the
      integration cassette"

<220>
<221> terminator
<222> 1105..1432
<223> /note="MOX-T; terminator of MOX gene"

<220>
<221> gene
<222> 1442..2740
<223> /note="EctB-T7 fusion ORF (EctB coding region/T7 tag coding
      region)"

<220>
<221> promoter
<222> 2748..3813
<223> /note="FMD promoter"

<220>
<221> misc_feature
<222> 3820..3853
<223> /note="loxP site"

<220>
<221> gene
<222> 4045..4956
<223> /note="Synthetic ADE1 gene; codons optimized for Hansenula
      polymorpha"

<220>
<221> misc_feature
<222> 5186..5219
<223> /note="loxP site"

<220>
<221> misc_feature
<222> 5220..6194
<223> /note="LEU2 upstream region for homologous recombination of the
      integration cassette"

<400> 10
   aaaccgattc ctgctgccgc acctcgcgag ccagctggag gctgacgggg agacgacgcc      60
   aaaatgctgg atgcgcgctt ttcgctgagg ttgtctctgt cgtcgcgctg gcgtctgcgc     120
   caaaacagga acaggagcag gagtcccaag agcagcaaag ctccgaggat cgacccgaca     180
   gcgattccgg ccttcgcgcc cttgctgagc cccgtggacc tgttgttggc gctcgaggca     240
   cttgaggacg acgcggcagc cgacgccgag gtggctgtag cgacggactg cgtgatgtag     300
   atgatcgagg tctgcatgtt tccaggtgaa acggacgtag ttatccgcac tgattcggat     360
   gagggtgtgg acgaagaggt ggttgaagac gacgacgagc tctgggagaa agaaaaggac     420
   gaaatcgagc ttgttgtagt cgagctagcg gacagacttg aggacgttgg agacgagtct     480
   gtggaagaca tggtggagct ctgtggggag gaactttgcg tggaggaaga cgatgttgag     540
   gagctggaca gactggaact ggaggaggag gtggtggagg tggaggaggt ggaagtggca     600
   tcgtctgtgg acgagtgcga cgactgcgcc gacgaactgt cgtcgtcgtt ttcctcagac     660
   tcgtcaacat agacggaata agagtcgcta ccaccacact tctccatggg ataaccagtg     720
   caggacgttg tgcacgagcc gtcggggttg tccgaaggta cgtcgtctcc gcaataacag     780
   tcctggccgt tgatcaatgc agcaactgcg tgtcccgaac actgctccgt gcaatggctg     840
   gagctctgcc atgtgtacga gtccttcttg gtcaagccgg aaatggcatc cgagctgtag     900
   caacctaaat acgtaaagtc tgctaggact tgggtgatca cgaatacgac accgaatctc     960
   atgttgacat ggaaaagcta aaaaataaac aatagagcgt cgcgtcgaaa aaaataaaat    1020
   ctgtgaggcg tgcgtgttta ggatttttgg gattatttag gaattattcg agatcctact    1080
   tttttttctc ccttatttaa ttaacaatct ccggaatggt gatctgatcg ttcctgaaaa    1140
   cctcgacatt ggctccctcc tgacacaggt actcgtacag gttccaggta aacgagtcgt    1200
   agttgtcgat catgacaacg ttcttagaag cggccggcat tttgaaggtg actaatagcc    1260
   taagaaaata tttaatttaa ttttcattaa attttcctat actcgctatt tcagcttttc    1320
   atctcatcac ttcataaacg atataaacca gaaaaagaac tattttcaaa cacgcttctc    1380
   aaaagcggta tgtccttcca cgtctcctta gaatctggca agtccgcgag gggatccttt    1440
   attagcccat ctgctggcca ccggtcatcg aggccatcga gaaggcctgc ttggtcgagg 1500
   tttccaggat gtccaggcct tccaccaggt cctcgtctgg gatggtcagt gggcacaggc 1560
   acttcaccac ctcgccgtcc tggcccgagg tctcgatgat caggccgttc tcgaaggcct 1620
   ggtgggtgat cttgtcggcg atgtcgcccg agcccacgtc gatgcctctc atcaggcctc 1680
   tgcctctttc cgaagcctcg atgccgttct ccgacagcca agcggcgatc ttgccgaatc 1740
   tctcttccac gattctggcc tttctctgca cgtctctctc gaaggtgtcg tccgaccagt 1800
   actttctcat ggctgcggca gcggtggcga aggccaggtt gaagcctctg aaggtgccgt 1860
   tgtactggcc tggcttccac ttgtccagct ctggtctcat cagcacgtgg gcgaatggca 1920
   ggccgtagcc cgacagcgac ttcgagttgg tcacgatgtc tggggtgatg ccggcgtgct 1980
   cgaacgagaa gaacttgccg gttctgccgc agccggcctg gatatcgtcg atgatcagca 2040
   ggatgtcgtt ggctctgcag atcgattcca gtctcttcag ccattccaga ccggccacgt 2100
   tgatgccgcc ctcgccctgc acggtttcca cgatcacggc ggctggcacg tccaggccac 2160
   ccgacttgtc gcccagcagc ttctcgaagt agtccagggt gtcggtcgac gagcccaggt 2220
   agccgtcgaa tggcatgaac gaggcagcct gggttgggac gcctccggtg gcctctctga 2280
   actttctgtt gccggtggta gccagagcgc ccatggtcac accgtggaag ccgttggtga 2340
   acgacacgat gttgtgtctg cccttggcca ctctggccag tctgatggca gcttccacgg 2400
   cgttggtgcc ggttgggcct ggcaggtgca ccttgtagtc caggcctctt ggcttcagga 2460
   tcacctcttc cagggtttcc aggtagtctc tcttggcggc ggtccaaaag tccaggccgt 2520
   gcacgatgcc gtccgagtcg atgtagtcca gcagggcctg cttcaggtgt gggttgttgt 2580
   ggccgtagtt cagggtgcct gcgccagcca gaaagtcgat gtactctctg ccttcctcgt 2640
   cggtcagtct ggcgtttctg gccttggtga acaccactgg gaacgatctc gagtaggttc 2700
   tcacgtccga ttccattctc tccaggatct gggtctgcat gaattcccga tgaaggcaga 2760
   gagcgcaagg aggcggtatt tatagtgcca ttcccctctc tgagagaccc ggatggtagt 2820
   cgagtgtatc ggagacagct tgatgtagac tccgtgcctg ccggctcctc ttattggcgg 2880
   acaccagtga gacaccccgg aacttgctgt ttttctgcaa aatccggggt gaccagtggg 2940
   agcctatttg cacacacgag cgggacaccc cactctggtg aagagtgcca aagtcattct 3000
   ttttcccgtt gcggggcagc cgattgcatg ttttaggaaa atattacctt tgctacaccc 3060
   tgtcagattt accctccaca catatatatt ccgtcacctc cagggactat tattcgtcgt 3120
   tgcgccgcca gcggaagata tccagaagct gttttccgag agactcggtt ggcgcctggt 3180
   atatttgatg gatgtcgcgc tgcctcacgt cccggtaccc aggaacgcgg tgggatctcg 3240
   ggcccatcga agactgtgct ccagactgct cgcccagcag gtgtttcttg atcgccgcct 3300
   ctaaattgtc cgcgcatcgc cggtaacatt tttccagctc ggagtttgcg tttagataca 3360
   gtttctgcga tgccaaagga gcctgcagat tataacctcg gatgctgtca ttcagcgctt 3420
   ttaatttgac ctccagatag ttgctgtatt tctgttccca ttggctgctg cgcagcttcg 3480
   tataactcga gttattgttg cgctctgcct cggcgtactg gctcatgatc tggatcttgt 3540
   ccgtgtcgct tttcttcgag tgtttctcgc aaacgatgtg cacggcctgc agtgtccaat 3600
   cggagtcgag ctggcgccga aactggcgga tctgagcctc cacactgccc tgtttctcta 3660
   tccacggcgg aaccgcctcc tgccgtttca gaatgttgtt caagtggtac tctgtgcggt 3720
   caatgaaggc gttattgccg gtgaaatctt tgggaagcgg ttttcctcgg ggaagattac 3780
   gaaattcccc gcgtcgttgc gcttcctgga tctcgatcga taacttcgta tagcatacat 3840
   tatacgaagt tatatgcgca ctctagattc cttgcgtaca aacacctttc cgtcagcgac 3900
   acgccttact ttgcgcacac atccgaacga gcctcgtccg atgagctcca acacttggaa 3960
   atcgtccatc gaatagtata tttacaaact tccggataca gaaaataatt aaattatttg 4020
   ggacagcttg gtttatttca acacttaagc ccaggtttta ccggtcaaag cctcgtaagc 4080
   ctctttgtac ttagcggcgg tcttagcagc aatctcctcg gtcatagaca caccctcttt 4140
   accgttcagc tggtgagaag tcaaccagtc tctcaggaac tgcttgtcga aagactcctg 4200
   agatcttccc aactcgtact tcttggcgtc ccaaaatctc gaagagtctg gggtcaaaac 4260
   ctcgtcaacc agaaccagct cgttgttctc gttcagtccg aactcaaact tggtgtcggc 4320
   caggataata cctctcgaca gagcgtagtc tctagcctta gagtacaggg caatagcctt 4380
   ctgggcaacc tcagcacaca actcctctcc aaccaactga gcagcctgct ctggagaaat 4440
   gttctcgtcg tgctgtccct gctctgcttt ggtagatggg gtgaagattg gcttctcgaa 4500
   agcctcagac tcctgcaatc cagacttaac tggcagtccg tgaacggtag acgacttctt 4560
   gtactctttc caggcagaac cggtgatgta acctctcaca atggcctcca atggaaccag 4620
   cttgtgcttc ttcaccagca gagatctacc agccaactga gccttgtact ttggctcaga 4680
   caactgctct ggcaacttgg caaagatgtc cttgtcagag gaagcaacca ggtggtttgg 4740
   aataacgtca gacagcaggt tgaaccagaa cacagacagc tgggtcagaa tctttccctt 4800
   gtccttgatg ccgttctcca taatcacgtc gtaggcagag attctgtcgg tggcaacaaa 4860
   cagcagcatg ttgtcgtcca cctggtagat gtctctcact ttacctctgg caaccaatgg 4920
   cagaataccg tccaggttag tctgggtcac ggtcatatgt gtaataataa ttaagtaaaa 4980
   ttagtttagt aaattaattg actcgtgagg gtcgatcgac gcgatattgg ctctcctttt 5040
   tatacgagtt tttgtcaact ttcctctgct atgctcaatc cagtcaccgt ccacaaagac 5100
   ttctttgggt ttcctggggc cttggttctg accatcagtc tacctttggt ggttgttttc 5160
   ctgtatgcgg cgtcgacggc gcgccataac ttcgtatagc atacattata cgaagttatc 5220
   ctgcagggat tgcaaaatga tgcaactatt ttgcgccggt accgggaaaa attgaaaaac 5280
   catccactta ctcattcctg tctttttatt tcgtattacc aaaccgctta cgtactcacc 5340
   cactcagatc tgctcagaca caaactggtc gatgtcgacc tcctcgagtg gagcagtggc 5400
   cgcattgttc acctttggct tctcctctct gagctcaaga cgtttcagct tactaagagg 5460
   gccccagccg tgcacaggtg ggctaagttt gaatggatcc aaaaacttga cacactgttt 5520
   gaacgagtcg cccaatgtgg caatctcgtg gatgatgtct tccacacaga tgatgccgta 5580
   gtctccaagc tggtcttcaa tcaaattgtt gtcatccaag ttcaccttct tggttgttcc 5640
   gtcctcgacg atactagccg tggcacgttt ctggatcaag tttcggacag tagcaagcga 5700
   tggagtgccg atggcgatgt acggactggt cagtctgagg agaggtttga cggcagcgtt 5760
   caacttgatg aaagttcctg tgtacacgtg ctctagacgc agcagttcaa gcacttttct 5820
   ggctttggag ggcactttgg caccatgcgg gccggggata cggaccacaa acaaaagacg 5880
   ctcatcggac tcttccggca cagcagagct ccgttcatgc tgagacacgc gcttcacacg 5940
   gtactgttct ctttctgatg cgcgatgttt agccactagg gtttctgctc tgataaacct 6000
   tgcctttctt tgctgttgtt tgcgtttctt ctgttcctcg agcttcttac gtgcctgctc 6060
   ctgtttctct agacggagtc tgtcggcgtt cttccgcttc ttgagcaaca cctctgggtt 6120
   ggtgttcagt ttagtcgatt cagccatgat aaaaaaaaaa aaaattgtgc agaaaaattt 6180
   atttggcagt gttgc 6195

<210> 11
<211> 6965
<212> DNA
<213> Hansenula polymorpha (syn. Pichia angusta)

<220>
<221> source
<222> 1..6965
<223> /mol_type="unassigned DNA"
/organism="Hansenula polymorpha (syn. Pichia angusta)"

<220>
<221> misc_feature
<222> 1..1007
<223> /note="ODC1 downstream region for homologous recombination of the
integration cassette"

<220>
<221> terminator
<222> 1015..1342
<223> /note="MOX-T; terminator of MOX gene"

<220>
<221> gene
<222> 1352..1792
<223> /note="EctC-HA fusion ORF (EctC coding region/HA tag coding
region)"

<220>
<221> promoter
<222> 1800..2865
<223> /note="FMD promoter"

<220>
<221> misc_feature
<222> 2872..2905
<223> /note="loxP site"

<220>
<221> gene
<222> 3205..5709
<223> /note="H. polymorpha HIS4"

<220>
<221> misc_feature
<222> 5864..5897
<223> /note="loxP site"

<220>
<221> misc_feature
<222> 5898..6964
<223> /note="ODC1 upstream region for homologous recombination of the
      integration cassette"

<400> 11
aaacaggccg tctcgatttg actacctcac aacagcaaga gctttgttgc atggagagct      60

tgcctccaaa ccgtggagct tacgaacaca ttcaggaata gctcttctat accaatcaat     120

caaaaaagaa gggtggaatt ttattccacg acaagtactt cctccaatag tggcaaacag     180

cgcaattgga gtgattcttt attctaccta tctcacacta ttacaacgtt ttaataagag     240

aaatatgaac tcaattgatg atcctagtcc tgtcgacact ttcaaagctg gttttgttgc     300

gggagctgct gcgagcttgg ctgcatcacc gctagatgca ctttacgccc gatcgaatta     360

ctcagagctt gtcagtggaa aacagaagag catgtgggag tatgccttat acaaactaaa     420

acaaattggt ttagttggaa tatttgccgg cttcagttta aatttcataa aggaaagcct     480

tggatttgca ttctactttt cagtatttga actagtgaaa aaccaggggt accacaagac     540

aaaagcagct atacagtggt atcaatattg gcgctcgaga ctttttcatg atgaagaaaa     600

ccaggagaag aattcaagat ttttcaaagc tttgaatttg acgtttatcc ttcttgctgg     660

agcttccgcc gcaactacac tgcttgcaat ccaatatcca ctcaataaaa ttcaaaaggt     720

ccacctgact agactcgaat ctctagatct ctacaacgaa gcacatcaac tggatcggcg     780

tcgcttcttc aaactctact ataattctta cattgagaca tttgatatta ttctacaacg     840

atggcgaaaa tctcacctct ccctctttgg cttctgctat agaggctttt tcagatcgtc     900

cttcactagt attcccgcga cttctattgg gcttttcgta ttcgaaatat cccgtcagca     960

ttttagcgaa aatcaatttt gacgatgtat atagaactga aagccgttaa ttaacaatct    1020

ccggaatggt gatctgatcg ttcctgaaaa cctcgacatt ggctccctcc tgacacaggt    1080

actcgtacag gttccaggta aacgagtcgt agttgtcgat catgacaacg ttcttagaag    1140

cggccggcat tttgaaggtg actaatagcc taagaaaata tttaatttaa ttttcattaa    1200

attttcctat actcgctatt tcagcttttc atctcatcac ttcataaacg atataaacca    1260

gaaaaagaac tattttcaaa cacgcttctc aaaagcggta tgtccttcca cgtctcctta    1320

gaatctggca agtccgcgag ggggatccct attaggcgta gtctggcacg tcgtatgggt 1380

acaatggttt ctggtcgtca gcctcgtcag ctggagcgta agaaccgtcc tctctgtgaa 1440

cctcgttacc agtcaaacct ggggtaaaaa cacaggccag gtgcatagtc ttagaagctc 1500

tcaacaggtg ctcgtcgtgc tggtccaaaa tgtagatgtc gcctggctta ataggccaga 1560

tcttaccgtc agccaaagtc tcaacctcgc cctcaccctc aatacagtac acggcctcga 1620

agtggtgttt gtagtggatg tgagtctcgg ttccctcgaa gattctggtg atgtggaaag 1680

agcagttacc accgtcctca gccaaagaca atctggtaga gtcccagtta ccgttctcag 1740

cagtaaccaa tctgtcggtc tgtctagctt cttccaagtt tctcacgatc atgaattccc 1800

gatgaaggca gagagcgcaa ggaggcggta tttatagtgc cattcccctc tctgagagac 1860

ccggatggta gtcgagtgta tcggagacag cttgatgtag actccgtgcc tgccggctcc 1920

tcttattggc ggacaccagt gagacacccc ggaacttgct gtttttctgc aaaatccggg 1980

gtgaccagtg ggagcctatt tgcacacacg agcgggacac cccactctgg tgaagagtgc 2040

caaagtcatt ctttttcccg ttgcggggca gccgattgca tgttttagga aaatattacc 2100

tttgctacac cctgtcagat ttaccctcca cacatatata ttccgtcacc tccagggact 2160

attattcgtc gttgcgccgc cagcggaaga tatccagaag ctgttttccg agagactcgg 2220

ttggcgcctg gtatatttga tggatgtcgc gctgcctcac gtcccggtac ccaggaacgc 2280

ggtgggatct cgggcccatc gaagactgtg ctccagactg ctcgcccagc aggtgtttct 2340

tgatcgccgc ctctaaattg tccgcgcatc gccggtaaca tttttccagc tcggagtttg 2400

cgtttagata cagtttctgc gatgccaaag gagcctgcag attataacct cggatgctgt 2460

cattcagcgc ttttaatttg acctccagat agttgctgta tttctgttcc cattggctgc 2520

tgcgcagctt cgtataactc gagttattgt tgcgctctgc ctcggcgtac tggctcatga 2580

tctggatctt gtccgtgtcg cttttcttcg agtgtttctc gcaaacgatg tgcacggcct 2640

gcagtgtcca atcggagtcg agctggcgcc gaaactggcg gatctgagcc tccacactgc 2700

cctgtttctc tatccacggc ggaaccgcct cctgccgttt cagaatgttg ttcaagtggt 2760

actctgtgcg gtcaatgaag gcgttattgc cggtgaaatc tttgggaagc ggttttcctc 2820

ggggaagatt acgaaattcc ccgcgtcgtt gcgcttcctg gatctcgatc gataacttcg 2880

tatagcatac attatacgaa gttatatgcg cactctagag caattccgac ggttgggatc 2940

gtgtatggtg cagagagacc gaccaccacg gggccgatcg actgcgtcag gaagtggatc 3000

gggaacagct tggattgcag gaggccaaac tccttgcggg gcaggacctt gaacgctctg 3060

acagaggaca caaatgagtg gaacgaagtg gctccgaaaa catacgagta agcaagcagg 3120

tgcacagcag tcagcatggt aaaaattaat tgcacttatt atttatttgg tcgtggcttt 3180

atttaatcag tgataattgt cacgctatat gtagccaagt ttttccattc taatcttgac 3240

ggcgttgcgg tgcgcgtcga gaccctcgac ggcagccagg tccatcacgg ccttgccaat 3300

gctcttgaga ccctccttgg tgaccacctg cgatgtaatg aatttctgga acgtggtggt 3360

gttaacaccc gagtacattc tggcgtagcc gtacgtaggc agcgtgtggt tggttcccga 3420

ggagtagtca ccgcagcttt ccggcgaata ggcacccaca aacacagagc cagcattgtc 3480

gaccttgtcg acccatttct cggcgtcctg gatctgcagg ataaggtgct ccggagcgta 3540

tctgttgctc atctcgaacg cctcatcgta gctcttcaca atgatcgtgg tggagtgagc 3600

aatgcactgg cgcacgatct ccacacgtgg cagctgcagc gcctggcgat ggacggcctc 3660

gtcgatcttg tcgatctcgg cagacgtcat gtccacggcg atcaatatca cttgcgagtc 3720

aacgccgtgt tctgcctgcg acagcaggtc cgaggccaca aagtctgggt cggcgtactt 3780

gtccgcgaca accagaacct cggatggccc ggcaggcata tcaatggcac acatggcgtt 3840

ggaatcattg gaaacgagca tcttggctgc cgtcacaaac tggtttccgg ggcccatgat 3900

cttgtcacat ttaggcacag actcggtacc gtacgccatt gcggccaccg cctgggcccc 3960

tccggcaagc acaatgcact tggcacccac cttgtgcgcc acatagacca cctctggcgt 4020

cagacggccg tccttgccgg gaggagacgc aaacacaata tctttgcaac cggcaacgag 4080

agcaggcaca gagagcataa gggacgtgga cggcagcacg gcagttccgc cggggatgta 4140

acagccaaca cgctcgatcg gacgtgcaaa tcgagagcac acaacgccgg gacacgtttc 4200

cacgcgtaga gtctctttct ggttctgcgc ttcgtggaag gtcttgatat tggcaaacga 4260

gatgtcaatg gccttgcgca cgtcttcgct gatgttcatc atttctgctg ggaatggagc 4320

gttcaataca ggagagctaa gctgcacctt gtcgaacttg gctgtgagct ccaccaacgc 4380

agcgtctcct tttgttctca ctttctcgac gatcggcgta accaggttca tgatatccgc 4440

agacttttgg attggtcttt gaagacattt ttcgatatct tctttggagg cagtggtggc 4500

atccacacgg ttcatcacga tgcggccatc tccgttcaca agccgctttg gcaattttgg 4560

caccgttggc accgttggtt cctctagctt tttttccgta gcgtcctgct caacatactt 4620

cgcctttgcg tcgcccttac gtctcgtcac cttcagggcc ttgatgtcca aattccgctc 4680

aacgtctgca agcgaaacgc cgtacttcac acatctggca agcgcaaagt agaccaaatc 4740

ggcagcttcc cacgcaatct cgtccttcga ggtggcttcc accagctcgt cggcttcttc 4800

cctaatcttg gcaccgagca gcttctcatc ctcaaacagt ctcttcgtgt aggagccttc 4860

aggagcattc gccttgcggt cccaaagagt ggcttccaga gctgccagac ccttggacga 4920

gccaaaacag gaggcagtgt cgaggtggca gaaaccagca ccggcctgtt ccacaacaaa 4980

tttcaaacaa tcgctatcgc agtctagctc cagttggagc aatttttgcg tggctcccga 5040

tgtctggcct ttgtaccaca agccgtgctt tctggactgg tagacacccg tgccggtcct 5100

tattgcctca gcaatggact tcttggacga gtacaccaaa ccaagagcct ggttcctgct 5160

gtcagtgaca agagttgtgt ataagccgtc tggtctgtcg gtgttcatgg cagcgagata 5220

gcactccaca aacggcgttt tggtgcccgc gttttcgtac tccttagtca gctgatcgag 5280

ctcaataaca gcagtgaagc ccttggcgat ctgccggagt ggttgttcaa aataaccaaa 5340

gactcttctt tttccaattt tcacttcagt ctccccaaaa acacagatat tctcggcagc 5400

atcttttcca tccttgatga ccagtctggc gttgggaata cggcttctaa tctgctcagc 5460

aacagcagag gtgacaaaaa gaccgtacac accgttgttc aaaagagcca gaacaatgtc 5520

tatttggcta tcattttcca cagaccccag agcccagtcg acatatacct ggcctagata 5580

ctctttagcc aacgcgataa tttcctgcac agcggcagcc gcatcaagcg acacaagaac 5640

ctcgctcaca aacggcacgc tcaaaacgtc ccttggttga gcaacgctca caaggatagg 5700

agaaaccatt tatgaattta cggtgagatg tgagtcaagc tgaaaaatat cttgattttt 5760

ttttttttga catgccacga ctttggtagc atgggacgat atatcactat atacactgtg 5820

agtcagttat tgaacaaaaa ctgcatatcg tcgacggcgc gccataactt cgtatagcat 5880

acattatacg aagttatcct gcagggttga ttattattca gggaaatgtt gaaaattttt 5940

cgataattca ttagtttatt tttgatacac catggatcaa ctcaactata aagaacagca 6000

agaattccag aaaattgtgg agcagaagca aatgaatgac ttcatgagag taagtgaacg 6060

ccttctgaaa actaacattg cagctctaca ccagtttggt ggacagatgt tttaatgatt 6120

gcgtcaacga cttcacctcc aacaaactta gctctagaga agaaacatgt ttgaacaagt 6180

gcgctgaaaa gttcctcaag cactccgaaa gagttggttt aagattccag gaacagaacc 6240

agatgttgat gaaccagatg agaggcggca attaatgagc tttaattgct ggatgattcc 6300

cggaataggc tcaacaagtt ttgcgtttct tccatcattt caatcgtcca tttgtaagtt 6360

tctgtctgat tcatatcgtt gtacataatt gcgttgctga acatcagttg aagctcaaat 6420

tccaactcgt cgaatgactt tatttcaccc gtcctaatag ctttacccaa actcttaatg 6480

tccacgggat gccgtattag cttgtagtag ttgggctctt cgttttcatt cactggttgc 6540

atgaacatag acgcaaaccg gttggatgat atctgtgtga gaagttgtgc agacagagca 6600

ttgaagcgct tagtacaacc aggggggacg ctttcggatc cgcgtcgctt gctcccacgt 6660

tcttcatctc cacttgctgg cgtagatggt gtagcaggtt ctggagttgg cgatggtatt 6720

tctttagaag ccgacttgga ttccaatttc gtttggggtt gggattctac attcgactct 6780

gaggttgaag ctgggccttc ttgaggttcg gcatgagttt ccaataactc tgttttgctt 6840

ttttccaaag cagtgaccgg ctgagattga gccgctgatt ctgcctcggg ctccgcaact 6900

tcatcgatct ctttgtctgc gtcattatgc gtcacgccct cttcaggttc ttgtgcgtca 6960

cctgc 6965

<210> 12
<211> 6213
<212> DNA

<213> Hansenula polymorpha (syn. Pichia angusta)

<220>
<221> source
<222> 1..6213
<223> /mol_type="unassigned DNA"
      /organism="Hansenula polymorpha (syn. Pichia angusta)"

<220>
<221> misc_feature
<222> 5..1061
<223> /note="ADE1 upstream region for homologous recombination of the
      integration cassette"

<220>
<221> misc_feature
<222> 1068..1101
<223> /note="loxP site"

<220>
<221> gene
<222> 1365..2453
<223> /note="Synthetic H. polymorpha LEU2 gene; codons optimized for
      Hansenula polymorpha"

<220>
<221> misc_feature
<222> 2670..2703
<223> /note="loxP site"

<220>
<221> promoter
<222> 2710..3775
<223> /note="FMD promoter"

<220>
<221> gene
<222> 3783..4805
<223> /note="H6-EctD fusion ORF (start methionine/His tag and flanking
      residues coding region/EctD coding region)"

<220>
<221> terminator
<222> 4815..5142
<223> /note="MOX-T; terminator of MOX gene"

<220>
<221> misc_feature
<222> 5148..6213
<223> /note="ADE1 downstream region for homologous recombination of the
      integration cassette"

<400> 12
ggccgccaca ccactgaagg ctcccgaaga cagatgcaca ggtctgagcg agagatatct       60

tgcttgcagg gagtaagtga atggaaccca acacagatcg ccaaatgcga gcatgaaacc      120

gaatccgtcg gtggtaatgt ccatcatgga aagcacgcct tcctcgttga gcactccgtc      180

gaaaatgtag aaggattgca tcgcaacgac gaacaagagc gagttagcca ccgttccagt      240

ttccagatac tgtttttgga tgcatgagag gtctatcagc agccaaagca gcattcctgg      300

ccgtagctcg caaaagagct tgatgtccca gggtcctatt ctggggttga gctctctgcc      360

gataaaccag tcaaaaagga cgtttttgga gtttccaccc tccgcaagaa tgcgctcgta 420

cgttcctgca ccattgggcc gtctcagcgg gaaaaaggag cagacgtaga caaacgtgct 480

gagaataaga gaaaattccc acgaaacgac catcaggggc aaaaaattgt cataaataaa 540

ctgaagttcg ggcacattgc cccttgtgta caacgctctt gctgccaaag ctgcacagac 600

caaaacactc agctccttac cgttgatcag atacgttaaa tgtgttccgt cacgcagcgt 660

tgtgcctttt ttatagtagc caggaacaat aagatccagc aatgcaagac caaaaaacca 720

gaccaggtat gctatccagc atttggcact cagaaacgtt tccttggtga gttgttctgc 780

aagcaagttt ttgatcgtgt tgatctgcaa attgcacccc tcgacaacat accgatcatt 840

gcacgccgca tacaggaaaa caaccaccaa aggtagactg atggtcagaa ccaaggcccc 900

aggaaaccca aagaagtctt tgtggacggt gactggattg agcatagcag aggaaagttg 960

acaaaaactc gtataaaaag gagagccaat atcgcgtcga tcgaccctca cgagtcaatt 1020

aatttactaa actaatttta cttaattatt attacacatc ctgcaggata acttcgtata 1080

atgtatgcta tacgaagtta tggcgcgccg tcgactgtgc acggctgggg ccctcttagt 1140

aagctgaaac gtcttgagct cagagaggag aagccaaagg tgaacaatgc ggccactgct 1200

ccactcgagg aggtagacat cgaccagttt gtgtctgagc agatctgagt gggtgagtac 1260

gtaagcggtt tggtaatacg aaataaaaag acaggaatga gtaagtggat ggtttttcaa 1320

tttttcccgg taccggcgca aaatagttgc atcattttgc aatcatgtcg aagaacatcg 1380

ttctgttgcc aggtgaccac gttggaccag aggttgtggc tgaggctgtt aaggttttgg 1440

aggccgtttc ttctgccatc ggcgtgaagt tcaacttctc gaagcacctg attggtggtg 1500

cttctattga cgcctacggc gttccattgt ctgacgaggc tttggaggct gctaaaaagg 1560

ctgacgctgt tctgttgggt gctgttggtg gtccaaaatg gggaaccggc tctgttagac 1620

cagagcaggg actgctgaag atcagaaaag agctgaacct gtacgctaac cttagaccat 1680

gttcttttgc ctctgacgct ctgttgaagc tgtctccact gaagtctgag atcgtgaagg 1740

gaaccgactt cgtggttgtg agagagctgg ttggcggtat ctacttcggc gacagaaaag 1800

aggacgctgg cgacggcgtt gcttctgaca ctgagagcta ctctgttcca gaggtgcaga 1860

gaatcaccag aatggccgct tttctggctt tgcagtctga cccaccattg ccattgtggt 1920

ctttggacaa ggctaacgtg ttggcttctt cgagactgtg gagaaagacc gtggaggaaa 1980

ctatcaagaa cgagttccca cagttgactg ttcagcacca gctgattgac tctgccgcca 2040

tgatcttggt taagtcgcca accaagctga acggtgttat cgtgaccaac aacatgttcg 2100

gcgacatcat ttcggacgag gcctctgtta ttccaggttc tctgggattg ctgccatctg 2160

catctttggc ttccctgcca gacaccaaca aggctttcgg tctgtacgag ccatgtcacg 2220

gttctgctcc agacttgggt ccaggaaagg ttaacccact ggccaccatt ttgtctgctg 2280

ccatgatgct gaagctgtct ttggacttgg ttgacgccgg tagagctatt gagcaggccg 2340

tgaagaacgt tttggacgcc ggtattatga ctgctgacct gggcggttct tcttctactc 2400

aggaagttgg cgacgctgtt gcacaggaag ttgccaagct gctgaagaac taaataaggg 2460

agaaaaaaaa gtaggatctc gaataattcc taaataatcc caaaaatcct aaacacgcac 2520

gcctcacaga ttttattttt ttcgacgcga cgctctattg tttatttttt agcttttcca 2580

tgtcaacatg agattcggtg tcgtattcgt gatcacccaa gtcctagcag actttacgta 2640

tttaggttgc tacactctag agtgcgcata taacttcgta taatgtatgc tatacgaagt 2700

tatcgatcga gatccaggaa gcgcaacgac gcggggaatt tcgtaatctt ccccgaggaa 2760

aaccgcttcc caaagatttc accggcaata acgccttcat tgaccgcaca gagtaccact 2820

tgaacaacat tctgaaacgg caggaggcgg ttccgccgtg gatagagaaa cagggcagtg 2880

tggaggctca gatccgccag tttcggcgcc agctcgactc cgattggaca ctgcaggccg 2940

tgcacatcgt ttgcgagaaa cactcgaaga aaagcgacac ggacaagatc cagatcatga 3000

gccagtacgc cgaggcagag cgcaacaata actcgagtta tacgaagctg cgcagcagcc 3060

aatgggaaca gaaatacagc aactatctgg aggtcaaatt aaaagcgctg aatgacagca 3120

tccgaggtta taatctgcag gctcctttgg catcgcagaa actgtatcta aacgcaaact 3180

ccgagctgga aaaatgttac cggcgatgcg cggacaattt agaggcggcg atcaagaaac 3240

acctgctggg cgagcagtct ggagcacagt cttcgatggg cccgagatcc caccgcgttc 3300

ctgggtaccg ggacgtgagg cagcgcgaca tccatcaaat ataccaggcg ccaaccgagt 3360

ctctcggaaa acagcttctg gatatcttcc gctggcggcg caacgacgaa taatagtccc 3420

tggaggtgac ggaatatata tgtgtggagg gtaaatctga cagggtgtag caaaggtaat 3480

attttcctaa aacatgcaat cggctgcccc gcaacgggaa aaagaatgac tttggcactc 3540

ttcaccagag tggggtgtcc cgctcgtgtg tgcaaatagg ctcccactgg tcaccccgga 3600

ttttgcagaa aaacagcaag ttccggggtg tctcactggt gtccgccaat aagaggagcc 3660

ggcaggcacg gagtctacat caagctgtct ccgatacact cgactaccat ccgggtctct 3720

cagagagggg aatggcacta taaataccgc ctccttgcgc tctctgcctt catcgggaat 3780

tcatgaaaca ccatcatcac caccaccagt ctgttcagac ctcgtctaac agaccattgc 3840

cacaggctaa cctgcacatt gctaccgaga ctccagaggc tgactcgaga attagatcgg 3900

ctccaagacc aggacaggac ccatacccaa ccagattgtc tgagccattg gacttgcctt 3960

ggctgaacag aagagagcca gttgttaaag gcgaggaggc tgacggtcca ttgtctgctg 4020

ctcagttgga cacctttgag agacagggct tcattttcga gccagacttt ttgaagggcg 4080

aggagttgga ggctttgaga cacgagctga acgctttgtt ggctagagat gacttcagag 4140

gtagagactt cgctattacc gagccacagg gcaacgagat tagatcgctg ttcgccgttc 4200

actacctgtc gagagtgttt tcgagactgg ccaacgacga gagattgatg ggtagagcca 4260

gacagatttt gggtggagag ccatacgttc accagtcgag aatcaactac aagccaggct 4320

ttgagggaaa gggtttcaac tggcactctg actttgagac ttggcacgct gaggacggaa 4380

tgccagctat gcacgccgtt tctgcctcta ttgtgctgac cgacaaccac acttttaacg 4440

gcccactgat gttggttcca ggctctcaca gagtgttcgt tccatgtctg ggagagactc 4500

cagaggacca ccacagacag tctctgaaaa cccaggagtt cggagttcca tcgagacagg 4560

ctctgagaga gctgattgac agacacggta ttgaggctcc aactggtgct gccggtggtt 4620

tgttgctgtt cgactgcaac actctgcacg gttctaacgc caacatgtcg ccagacccaa 4680

gatcgaacgc cttcttcgtg tacaatagaa gagacaacag atgcgttgag ccatacgccg 4740

cttctaagag aagaccaaga ttcttggctc acgagccaga cgaggcttgg tcgccagacg 4800

gttaataagg atccccctcg cggacttgcc agattctaag gagacgtgga aggacatacc 4860

gcttttgaga agcgtgtttg aaaatagttc tttttctggt ttatatcgtt tatgaagtga 4920

tgagatgaaa agctgaaata gcgagtatag gaaaatttaa tgaaaattaa attaaatatt 4980

ttcttaggct attagtcacc ttcaaaatgc cggccgcttc taagaacgtt gtcatgatcg 5040

acaactacga ctcgtttacc tggaacctgt acgagtacct gtgtcaggag ggagccaatg 5100

tcgaggtttt caggaacgat cagatcacca ttccggagat tgttaattaa gtgttgaaat 5160

aaaccaagct gtcccaaata atttaattat tttctgtatc cggaagtttg taaatatact 5220

attcgatgga cgatttccaa gtgttggagc tcatcggacg aggctcgttc ggatgtgtgc 5280

gcaaagtaag gcgtgtcgct gacggaaagg tgtttgtacg caaggaaata tcatacgggg 5340

ccatgaatag caaggagaag caccagctta gcaccgagtt caggatccta cgagaactga 5400

gacatcccaa cattgtgcag tatctccacc atgaccatgt ccaggatgag caccaggttc 5460

atttgtatat ggagtactgc gacggaggcg atctatcggt gctgataaag aagtacaagg 5520

agagaaacga gtttataccg gaaacactga tctggcagat atttacccag gtgttgaatg 5580

cgctttacaa atgtcactat ggaacgtcga tagagccggt cagcaatctt ttccagtcgt 5640

ctcccgaact agaaccgaaa cctgtggacc atcaggtgat tattcatcgc gacatcaaac 5700

cagacaacat tttccttttg tcggacgggt attcgatcaa actgggggat ttcggtcttg 5760

caaagtggct ccaacacgaa aacgagtttg ccaagaccta tgtgggcacg ccatactaca 5820

tgtctcccga ggtgctgatg gatagaccgt acgaccccgt ctgcgacgtg tggtcgctcg 5880

gatgcgtcat gtatgaattg tgtgctctac gtcctccttt ccaggccaag actcacttgg 5940

cattgcagga aaaaatcaag gctggcacgt tccagccgat ccctgaccat tactcgcata 6000

gactacagat ggtcatcaac gcatgtctta ttgtcgaccc catggaacgc gctaccgtca 6060

accaactgct acaggacgcc tctttcaaat tgttcagaaa agagtgggaa atgacgcaat 6120

gggagaaccg gctgcgcgag cacgaaacag agctgcttct taaggaacgc aagctggtca 6180

agtttgagga cgctctcaac gaagaatgcg ttt 6213